(19) Europäisches Patentamt
European Patent Office
Office européen des brevets

(11) Veröffentlichungsnummer: **0 470 543 A1**

# (12) EUROPÄISCHE PATENTANMELDUNG

(21) Anmeldenummer: **91113121.7**

(22) Anmeldetag: **05.08.91**

(51) Int. Cl.5: **C07D 471/04**, C07D 487/04, C07D 473/00, C07D 473/40, A61K 31/435, A61K 31/52, A61K 31/505, A61K 31/495

(30) Priorität: **10.08.90 DE 4025358**
**05.10.90 DE 4031601**
**25.02.91 DE 4105827**

(43) Veröffentlichungstag der Anmeldung:
**12.02.92 Patentblatt 92/07**

(84) Benannte Vertragsstaaten:
**AT BE CH DE DK ES FR GB GR IT LI LU NL SE**

(71) Anmelder: **Dr. Karl Thomae GmbH**
**Postfach 1755**
**W-7950 Biberach 1(DE)**

(72) Erfinder: **Ries, Uwe, Dr. Dipl.-Chem.**
**Dunantstrasse 10**
**W-7950 Biberach 1(DE)**
Erfinder: **Narr, Berthold, Dr. Dipl.-Chem.**
**Obere Au 5**
**W-7950 Biberach 1(DE)**
Erfinder: **Bomhard, Andreas, Dr. Dipl.-Chem.**
**Max-Born-Strasse 41**
**W-4000 Düsseldorf 13(DE)**
Erfinder: **Hauel, Norbert, Dr. Dipl.-Chem.**
**Stresemannstrasse 40**
**W-7950 Biberach 1(DE)**
Erfinder: **van Meel, Jacques, Dr.**
**Schubertweg 4**
**W-7951 Mittelbiberach(DE)**
Erfinder: **Wienen, Wolfgang, Dr. Dipl.-Biol.**
**Am Schiessberg 28**
**W-7951 Aepfingen(DE)**
Erfinder: **Entzeroth, Michael, Dr. Dipl.Chem.**
**Sebastian-Sailer-Strasse 20**
**W-7951 Warthausen(DE)**

## (54) Heterocyclische Imidazole, diese Verbindungen enthaltende Arzneimittel und Verfahren zur ihrer Herstellung.

(57) Die Erfindung betrifft heterocyclische Imidazole der allgemeinen Formel

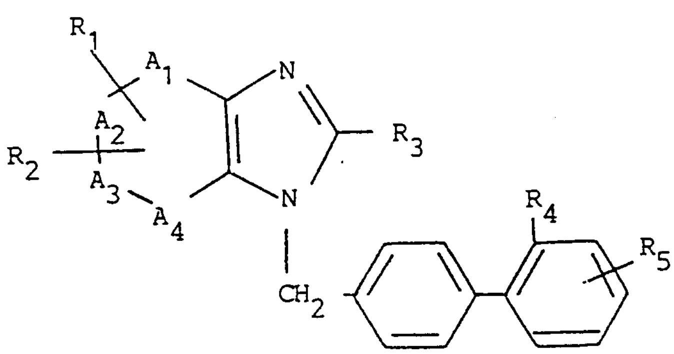

(I)

in der
$A_1$ bis $A_4$ und $R_1$ bis $R_5$ wie im Anspruch 1 definiert sind, deren 1-, 3-Isomerengemische, deren Tautomere, deren Enantiomere und deren Additionssalze, welche wertvolle pharmakologische Eigenschaften aufweisen.

Die neuen Verbindungen stellen Angiotensin-Antagonisten, insbesondere Angiotensin-II-Antagonisten, dar.

EP 0 470 543 A1

Gegenstand der vorliegenden Erfindung sind heterocyclische Imidazole der allgemeinen Formel

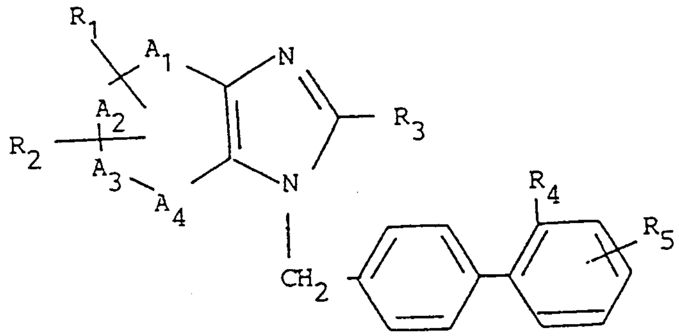

(I)

deren 1-, 3-Isomerengemische, deren Tautomere, deren Enantiomere sowie deren Additionssalze, insbesondere für die pharmazeutische Anwendung deren physiologisch verträglichen Additionssalze mit anorganischen oder organischen Säuren oder Basen.

In der obigen allgemeinen Formel bedeutet

einer oder zwei der Reste $A_1$, $A_2$, $A_3$ oder $A_4$ ein Stickstoffatom und die verbleibenden Reste $A_1$, $A_2$, $A_3$ oder $A_4$ jeweils eine Methingruppe,

$R_1$ ein Fluor-, Chlor- oder Bromatom, eine Hydroxy-, Alkyl- oder Alkoxygruppe, eine gegebenenfalls am Stickstoffatom durch eine Alkylgruppe mit 1 bis 6 Kohlenstoffatomen, durch eine Phenyl-, Cycloalkyl-, Phenylalkyl- oder Cycloalkylalkylgruppe mono- oder disubstituierte Aminogruppe, wobei die Substituenten gleich oder verschieden sein können, oder monosubstituierte N-Acylaminogruppe, in welcher der Acylrest eine Alkanoylgruppe mit 1 bis 7 Kohlenstoffatomen, welche in 3-, 4-, 5-, 6- oder 7-Stellung durch ein Halogenatom oder eine Hydroxygruppe substituiert sein kann, eine Alkoxycarbonylgruppe mit insgesamt 2 bis 4 Kohlenstoffatomen, eine Alkylsulfonylgruppe mit 1 bis 6 Kohlenstoffatomen, eine Benzoyl-, Benzolsulfonyl-, Phenylalkansulfonyl-, Naphthalensulfonyl-, 2-Carboxy-cyclohexylcarbonyl-, 2-Aminocarbonyl-cyclohexylcarbonyl-, Cycloalkylcarbonyl-, Phenylalkanoyl- oder Cycloalkylalkanoylgruppe darstellt, wobei die vorstehend erwähnten Phenylkerne durch ein Fluor-, Chlor- oder Bromatom, durch eine Methyl- oder Methoxygruppe mono- oder disubstituiert und die Substituenten gleich oder verschieden sein können, eine Alkylsultamgruppe mit 3 bis 5 Kohlenstoffatomen im Alkylteil, eine gegebenenfalls durch eine Alkyl- oder Hydroxycarbonylalkylgruppe substituierte Pyrrolidino-, Piperidino- oder Hexamethyleniminogruppe, eine Pyrrolidinon-, Piperidinon- oder Hexamethyleniminongruppe, eine gegebenenfalls ganz oder teilweise hydrierte Phthalimido- oder 2-Oxoisoindolin-1-yl-gruppe oder eine Gruppe der Formel

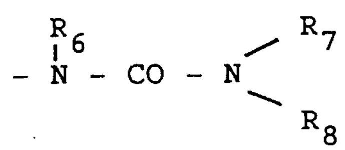

,

in welcher

$R_6$, $R_7$ und $R_8$, die gleich oder verschieden sein können, Wasserstoffatome, Alkylgruppen mit 1 bis 8 Kohlenstoffatomen, Cycloalkyl-, Phenyl-, Cycloalkylalkyl- oder Phenylalkylgruppen oder

$R_7$ auch eine 2-Chlor-ethyl-, 2-Brom-ethyl-, 3-Chlor-propyl- oder 3-Brom-propylgruppe oder auch, wenn $R_6$ und $R_7$ zusammen eine Ethylen- oder Propylengruppe darstellen, eine Dialkylaminocarbonylgruppe, oder

$R_6$ und $R_7$ zusammen eine Ethylen- oder Propylengruppe darstellen, wobei, der Phenylkern der vorstehend erwähnten Phenyl- und Phenylalkylgruppen jeweils durch Hydroxy- oder Alkoxygruppen mono- oder disubstituiert und die Substituenten gleich oder verschieden sein können, und, soweit nichts anderes erwähnt wurde, die vorstehend erwähnten Alkyl- und Alkoxyteile jeweils 1 bis 3 Kohlenstoffatome, die Alkanoylteile 1 bis 4 Kohlenstoffatome und die Cycloalkylteile jeweils 5 bis 7 Kohlenstoffatome enthalten können, oder

$R_1$ auch ein Wasserstoffatom, wenn nicht gleichzeitig $R_2$ ein Wasserstoffatom, $R_3$ eine n-Butylgruppe, $R_4$ eine Carboxygruppe und $R_5$ ein Wasserstoffatom sowie

(i) einer der Reste $A_1$ bis $A_4$ ein Stickstoffatom und die verbleibenden Reste $A_1$ bis $A_4$ jeweils eine Methingruppe oder

(ii) $A_2$ und $A_4$ oder $A_1$ und $A_3$ jeweils ein Stickstoffatom und die verbleibenden Reste $A_1$ und $A_3$ oder $A_2$ und $A_4$ jeweils eine Methingruppe oder

(iii) $A_4$ ein Stickstoffatom und $A_3$ eine durch eine Hydroxy- oder Methoxygruppe substituierte Methingruppe und die verbleibenden Reste $A_1$ und $A_2$ jeweils eine Methingruppe oder

(iv) $A_4$ ein Stickstoffatom, $A_1$ durch eine Methylgruppe substituierte Methingruppe und die verbleibenden Reste $A_2$ und $A_3$ jeweils eine Methingruppe darstellen,

$R_2$ ein Wasserstoffatom oder eine Alkylgruppe mit 1 bis 3 Kohlenstoffatomen,

$R_3$ eine Alkylgruppe mit 1 bis 6 Kohlenstoffatomen,

$R_4$ eine Carboxy-, Cyano-, 1H-Tetrazolyl- oder 1-Triphenylmethyl-tetrazolylgruppe oder eine Alkoxycarbonylgruppe mit insgesamt 2 bis 5 Kohlenstoffatomen und

$R_5$ ein Wasserstoff-, Fluor-, Chlor- oder Bromatom.

Die neuen Verbindungen der obigen Formel I weisen wertvolle Eigenschaften auf. So weisen die Verbindungen der Formel I, in der $R_4$ eine Alkoxycarbonylgruppe, eine Carboxy- oder 1H-Tetrazolylgruppe bedeutet, insbesondere wertvolle pharmakologische Eigenschaften auf, da diese Angiotensin-Antagonisten, insbesondere Angiotensin-II-Antagonisten, darstellen.

Gegenstand der vorliegenden Erfindung sind somit die neuen vorstehend erwähnten heterocyclischen Imidazole, wobei die entsprechenden Cyano-, Alkoxycarbonyl- und Triphenylmethylverbindungen auch wertvolle Zwischenprodukte darstellen.

Ein weiterer Gegenstand der vorliegenden Erfindung sind somit neue Arzneimittel, welche eine der oben erwähnten pharmakologisch wirksamen Verbindungen der allgemeinen Formel I oder ein entsprechendes physiologisch verträgliches Additionssalz enthalten und insbesondere zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen geeignet sind.

Für die bei der Definition durch die Reste $A_1$, $A_2$, $A_3$ und $A_4$ eingangs erwähnten heteroaromatischen Reste kommt die Pyrido-, Pyrimido-, Pyrazino- oder Pyridazinogruppe, welche im Kohlenstoffgerüst durch die Reste $R_1$ und $R_2$ substituiert sein können,

für $R_1$ die des Wasserstoff-, Fluor-, Chlor- oder Bromatoms, der Methyl-, Ethyl-, n-Propyl-, Isopropyl-, Hydroxy-, Methoxy-, Ethoxy-, n-Propoxy-, Isopropoxy-, Formylamino-, Acetylamino-, Propionylamino-, n-Butanoylamino-, Isobutanoylamino-, n-Pentanoylamino-, 2-Methyl-n-butanoylamino-, 3-Methyl-n-buta noylamino-, n-Hexanoylamino-, n-Heptanoylamino-, Cyclopentylcarbonylamino-, Cyclohexylcarbonylamino-, Cycloheptylcarbonylamino-, Cyclopentylmethylcarbonylamino-, Cyclohexylmethylcarbonylamino-, Cycloheptylmethylcarbonylamino-, (2-Cyclopentylethyl)-carbonylamino-, (2-Cyclohexylethyl)-carbonylamino-, (2-Cycloheptylethyl)-carbonylamino-, Benzoylamino-, Phenylacetylamino-, 2-Phenylpropionylamino-, Naphthyl-(1)-carbonylamino-,Naphthyl-(2)-carbonylamino-, Methoxycarbonylamino-, Ethoxycarbonylamino-, n-Propoxycarbonylamino-, Methansulfonylamino-, Ethansulfonylamino-, n-Propansulfonylamino-, Isopropansulfonylamino-, n-Butansulfonylamino-, n-Pentansulfonylamino-, n-Hexansulfonylamino-, Benzolsulfonylamino-, 2-Methylbenzolsulfonylamino-, 4-Methylbenzolsulfonylamino-, 2-Methoxybenzolsulfonylamino-, 4-Methoxybenzolsulfonylamino-, 2-Fluorbenzolsulfonylamino-, 4-Fluorbenzolsulfonylamino-, 2-Chlorbenzolsulfonylamino-, 4-Chlorbenzolsulfonylamino-, 2-Brombenzolsulfonylamino-, 4-Brombenzolsulfonylamino-, 2,4-Dimethoxybenzolsulfonylamino-, Benzylsulfonylamino-, Naphthalen-(1)-sulfonylamino-, Naphthalen-(2)-sulfonylamino-, Propansultam-(1)-yl-, n-Butansultam-(1)-yl-, n-Pentansultam-(1)-yl-, Amino-, Methylamino-, Ethylamino-, n-Propylamino-, Isopropylamino-, n-Butylamino-, Isobutylamino-, n-Pentylamino-, n-Hexylamino-, Dimethylamino-, Diethylamino-, Di-n-propylamino-, Methyl-ethylamino-, Methyl-isopropylamino-, Methyl-n-butylamino-, Ethyl-n-propylamino-, Methyl-n-pentylamino-, Ethyl-n-hexylamino-, Cyclopentylamino-, Cyclohexylamino-, Cycloheptylamino-, Cyclopentylmethylamino-, Cyclohexylmethylamino-, Cycloheptylmethylamino-, (2-Cyclopentylethyl)-amino-, (2-Cyclohexylethyl)-amino-, (2-Cycloheptylethyl)-amino-, (3-Cyclopentylpropyl)-amino-, (3-Cyclohexylpropyl)-amino-, (3-Cycloheptylpropyl)-amino-, Benzylamino-, 2-Phenylethylamino-, 3-Phenylpropylamino-, Phenylamino-, Dicyclohexylamino-, Dicyclohexylmethylamino-, Dibenzylamino-, N-Methyl-cyclopropylamino-, N-Methyl-cyclopentylamino-,N-Ethyl-cyclohexylamino-, N-(n-Propyl)-cycloheptylamino-, N-Methyl-cyclopentylmethylamino-, N-Ethyl-cyclohexylmethylamino-, N-(n-Propyl)-cycloheptylmethylamino-, N-Methyl-(2-cyclopentylethyl)-amino-, N-Ethyl-(2-cyclohexylethyl)-amino-, N-(n-Propyl)-(2-cycloheptylethyl)-amino-, N-Methyl-(3-cyclopentylpropyl)-amino-, N-Ethyl-(3-cyclohexylpropyl)-amino-, N-Methyl-benzylamino-, N-Ethyl-benzylamino-, N-Isopropyl-benzylamino-, N-Methyl-(2-phenylethyl)-amino-, N-Methyl-phenylamino-, N-(n-Propyl)-phenylamino-, N-Acetyl-methylamino-, N-Acetyl-ethylamino-, N-Acetyl-isopropylamino-, N-Acetyl-n-butylamino-, N-Acetyl-n-hexylamino-, N-Propionyl-methylamino-, N-Propionyl-ethylamino-, N-Propionyl-n-butylamino-, N-n-Butanoyl-methylamino-, N-n-Butanoyl-ethylamino-, N-n-Butanoyl-n-pentylamino-, N-Isobutanoyl-methylamino-, N-Isobutanoyl-ethylamino-, N-Isobutanoylisopropylamino-, N-Isobutanoyl-n-butylamino-, N-Isobutanoyl-n-pentylamino-, N-n-Pentanoyl-methylamino-, N-n-Pentanoyl-ethylamino-, N-n-Pentanoyl-isopropylamino-, N-n-Pentanoyl-n-pentylamino-, N-n-Hexanoylmethylamino-, N-n-Hexanoyl-ethylamino-, N-n-Hexanoyl-isopropylamino-, N-n-Hexanoyl-n-pentylamino-, N-Cyclopentylcarbonyl-methylamino-, N-Cyclohexylcarbonyl-methylamino-, N-Cyclohexylcarbonyl-ethylamino-, N-Cycloheptylcarbonyl-methylamino-, N-Cyclopentylmethylcarbonyl-methylamino-, N-Cyclohexylmethylcarbonyl-methylamino-, N-Cycloheptylmethylcarbonyl-methylamino-, N-(2-Cyclopentylethylcarbonyl)-methylamino-, N-(2-Cyclohexylethylcarbonyl)-methylamino-, N-(2-Cycloheptylethylcarbonyl)-methylamino-, N-Benzoyl-methylamino-, N-Benzoyl-ethylamino-, N-Benzoyl-isopropylamino-, N-Benzoyl-n-butylamino-, N-Benzoyl-n-pentylamino-, N-Benzoyl-n-hexylamino-, N-Phenylacetyl-methylamino-, N-Naphthyl-(1)-carbonyl-methylamino-, N-Naphthyl-(2)-carbonyl-methylamino-, N-Methoxycarbonyl-methylamino-,N-Methoxycarbonyl-ethylamino-, N-Methoxycarbonyl-n-butylamino-, N-Methoxycarbonylisobutylamino-, N-Ethoxycarbonyl-methylamino-, N-Ethoxycarbonyl-ethylamino-, N-Ethoxycarbonylisopropylamino-, N-Ethoxycarbonyl-n-butylamino-, N-n-Propoxycarbonyl-methylamino-, N-Methansulfonylmethylamino-, N-Ethansulfonyl-isopropylamino-, N-(n-Propansulfonyl)-methylamino-, Isopropansulfonylamino-, N-(n-Butansulfonyl)-methylamino-, N-(n-Pentansulfonyl)-methylamino-, N-(n-Hexansulfonyl)-methylamino-, Benzolsulfonylamino-, N-(2-Methylbenzolsulfonyl)-methylamino-, N-(4-Methylbenzolsulfonyl)-methylamino-, N-(2-Methoxybenzolsulfonyl)-methylamino-, N-(4-Methoxybenzolsulfonyl)-methylamino-, N-(2-Fluorbenzolsulfonyl)-methylamino-, N-(4-Fluorbenzolsulfonyl)-methylamino-, N-(2-Chlorbenzolsulfonyl)-amino-, N-(4-Chlorbenzolsulfonyl)-methylamino-, N-(2-Brombenzolsulfonyl)-methylamino-, N-(4-Brombenzolsulfonyl)-methylamino-, N-(2,4-Dimethoxybenzolsulfonyl)-methylamino-, N-Benzylsulfonyl-methylamino-, N-(Naphthalen-(1)-sulfonyl)-methylamino-, N-(Naphthalen-(2)-sulfonyl)-methylamino-, N-Benzoyl-cyclopentylamino-,N-Benzoyl-cyclohexylamino-, N-Benzoyl-cycloheptylamino-, N-Phenylacetylcyclopentylamino-, N-Phenylacetyl-cyclohexylamino-, N-Phenylacetyl-cycloheptylamino-, N-Benzoylcyclopentylmethylamino-, N-Benzoyl-cyclohexylmethylamino-, N-Benzoyl-cycloheptylmethylamino-, N-Phenylacetyl-cyclopentylmethylamino-,N-Phenylacetyl-cyclohexylmethylamino-, N-Phenylacetylcycloheptylmethylamino-, N-(3-Phenylpropionyl)-methylamino-, N-(3-Phenylpropionyl)-ethylamino-, N-(3-Phenylpropionyl)-isopropylamino-, N-(3-Phenylpropionyl)-isobutylamino-, N-Benzoyl-(2-cyclopentylethyl)-amino-, N-Benzoyl-(2-cyclohexylethyl)-amino-, N-Benzoyl-(2-cycloheptylethyl)-amino-, N-Phenylacetyl-(2-cyclopentylethyl)-amino-, N-Phenylacetyl-(2-cyclohexylethyl)-amino-, N-Phenylacetyl-(2-cycloheptylethyl)-amino-, N-Benzoyl-(3-cyclopentylpropyl)-amino-, N-Benzoyl-(3-cyclohexylpropyl)-amino-, N-Benzoyl-(3-cycloheptylpropyl)-amino-, N-Phenylacetyl-(3-cyclopentylpropyl)-amino-, N-Phenylacetyl-(3-cyclohexylpropyl)-amino-, N-Phenylacetyl-(3-cycloheptylpropyl)-amino-, N-Acetyl-cyclopentylamino-,N-Acetyl-cyclohexylamino-, N-Acetyl-cycloheptylamino-, N-Acetyl-cyclopentylmethylamino-, N-Acetylcyclohexylmethylamino-, N-Acetyl-cycloheptylmethylamino-, N-Acetyl-(2-cyclopentylethyl)-amino-,N-Acetyl-(2-cyclohexylethyl)-amino-, N-Acetyl-(2-cycloheptylethyl)-amino-, N-Acetyl-(3-cyclopentylpropyl)-amino-, N-Acetyl-(3-cyclohexylpropyl)-amino-, N-Acetyl-(3-cycloheptylpropyl)-amino-, N-Acetyl-benzylamino-, N-Acetyl-(2-phenylethyl)-amino-, N-Acetyl-(3-phenylpropyl)-amino-,N-Benzoyl-benzylamino-, N-Benzoyl-(2-phenylethyl)-amino-, N-Benzoyl-(3-phenylpropyl)-amino-, 2-Carboxy-cyclohexylcarbonylamino-, 2-Aminocarbonyl-cyclohexylcarbonylamino-, Phthalimido-, Tetrahydrophthalimido-, Hexahydrophthalimido-, cis-Hexahydrophthalimido-, trans-Hexahydrophthalimido-,Pyrrolidino-, Methylpyrrolidino-, Ethylpyrrolidino-, Isopropylpyrrolidino-, Piperidino-, Methylpiperidino-, Ethylpiperidino-, Isopropylpiperidino-, Hexamethylenimino-, Methylhexamethylenimino-, Ethylhexamethylenimino-, Isopropylhexamethylenimino-, 2-Carboxymethyl-pyrrolidino-, 2-Oxo-pyrrolidino-, 2-Oxo-piperidino-, 2-Oxo-hexamethylenimino-, Aminocarbonylamino-, Methylaminocarbonylamino-, Dimethylaminocarbonylamino-, N-Methylaminocarbonylmethylamino-, N-(Dimethylaminocarbonyl)-methylamino-, N-Dimethylaminocarbonyl-ethylamino-,N-Dimethylaminocarbonyl-isopropylamino-, N-(Dimethylaminocarbonyl)-n-pentylamino-, N-Methylaminocarbonyl-ethylamino-, N-Methylaminocarbonyl-n-pentylamino-, N-Methylaminocarbonyl-n-hexylamino-, N-Methylaminocarbonyl-n-octylamino-, N-Methylaminocarbonyl-cyclohexylamino-, Ethylaminocarbonylamino-, N-Ethylaminocarbonyl-methylamino-, N-Ethylaminocarbonyl-ethylamino-, N-Ethylaminocarbonyl-n-hexylamino-, N-Ethylaminocarbonyl-n-heptylamino-, N-Ethylaminocarbonylcyclohexylamino-, Diethylaminocarbonylamino-, N-(Diethylaminocarbonyl)-methylamino-, N-(Diethylaminocarbonyl)-ethylamino-,N-(Diethylaminocarbonyl)-n-butylamino-, N-(Diethylaminocarbonyl)-n-hexylamino-, N-(Diethylaminocarbonyl)-n-octylamino-, Isopropylaminocarbonylamino-, N-Isopropylaminocarbonyl-methylamino-, n-Butylaminocarbonylamino-, N-(n-Butylaminocarbonyl)-methylamino-, N-(n-Butylaminocarbonyl)-ethylamino-, N-(n-Butylaminocarbonyl)-isopropylamino-, N-(n-Butylaminocarbonyl)-n-butylamino-, N-(n-Butylaminocarbonyl)-n-hexylamino-, N-(n-Butylaminocarbonyl)-cyclohexylamino-, N-(Di-(n-Butyl)-aminocarbonyl)-amino-, N-(Di-(n-Butyl)-aminocarbonyl)-methylamino-, N-(Di-(n-Butyl)-aminocarbonyl)-ethylamino-, N-(Di-(n-Butyl)-aminocarbonyl)-n-butylamino-, N-(Di-(n-Butyl)-aminocarbonyl)-n-hexylamino-, N-(n-Pentylaminocarbonyl)-ethylamino-, N-(n-Hexylaminocarbonyl)-ethylamino-, n-Hexylaminocarbonylamino-, n-Heptylaminocarbonylamino-, n-Octylaminocarbonylamino-, N-(n-Hexylaminocarbonyl)-n-butylamino-, N-(n-Hexylaminocarbonyl)-n-pentylamino-, N-(n-Hexylaminocarbonyl)-n-hexylamino-, N-(n-Hexylaminocarbonyl)-cyclohexylamino-, Di-(n-Hexyl)-aminocarbonylamino-, N-(Di-(n-Hexyl)-aminocarbonyl)-methylamino-, N-((n-Hexyl)-methylaminocarbonyl)-amino-, Cyclohexylaminocarbonylamino-, N-Cyclohexylaminocarbonyl-methylamino-, N-Cyclohexylaminocarbonylethylamino-, N-Cyclohexylaminocarbonyl-n-butylamino-, N-Cyclohexylaminocarbonyl-isobutylamino-, N-Cyclohexylaminocarbonyl-n-pentylamino-, N-Cyclohexylaminocarbonyl-n-hexylamino-, N-Cyclohexylaminocarbonyl-cyclohexylamino-, N-(Ethyl-cyclohexylaminocarbonyl)-methylamino-, N-(Propyl-cyclohexylaminocarbonyl)-methylamino-, N-(n-Butyl-cyclohexylaminocarbonyl)-methylamino-, N-Benzylaminocarbonyl-isobutylamino-, 2(1H)-Imidazolidinon-1-yl-, 3-Methyl-2(1H)-imidazolidinon-1-yl-,3-Ethyl-2(1H)-imidazolidinon-1-yl-, 3-n-Propyl-2(1H)-imidazolidinon-1-yl-, 3-Isopropyl-2(1H)-imidazolidinon-1-yl-, 3-n-Butyl-2-(1H)-imidazolidinon-1-yl-, 3-Isobutyl-2(1H)-imidazolidinon-1-yl-, 3-n-Pentyl-2(1H)-imidazolidinon-1-yl-, 3-n-Hexyl-2(1H)-imidazolidinon-1-yl-, 3-Cyclopentyl-2(1H)-imidazolidinon-1-yl-, 3-Cyclohexyl-2(1H)-imidazolidinon-1-yl-, 3-Cycloheptyl-2(1H)-imidazolidinon-1-yl-, 3-Benzyl-2(1H)-imidazolidinon-1-yl-, 3-(3-Hydroxybenzyl)-2(1H)-imidazolidinon-1-yl-, 3-(4-Hydroxybenzyl)-2(1H)-imidazolidinon-1-yl-, 3-(3-Methoxybenzyl)-2(1H)-imidazolidinon-1-yl-, 3-(4-Methoxybenzyl-)-2(1H)-imidazolidinon-1-yl-, 3-(3,4-Dihydroxybenzyl)-2-(1H)-imidazolidinon-1-yl-, 3-(3,4-Dimethoxybenzyl)-2(1H)-imidazolidinon-1-yl-, 3-Cyclopentylmethyl-2(1H)-imidazolidinon-1-yl-, 3-Cyclohexylmethyl-2(1H)-imidazolidinon-1-yl-, 3-Cycloheptylmethyl-2(1H)-imidazolidinon-1-yl-, 3-(2-Phenylethyl)-2(1H)-imidazolidinon-1-yl-, 3-(2-Cyclopentylethyl)-2(1H)-imidazolidinon-1-yl-, 3-(2-Cyclohexylethyl)-2(1H)-imidazolidinon-1-yl-, 3-(2-Cycloheptylethyl)-2(1H)-imidazolidinon-1-yl-, 3-(3-Phenylpropyl)-2(1H)-imidazolidinon-1-yl-, 3-(3-Cyclopentylpropyl)-2(1H)-imidazolidinon-1-yl-, 3-(3-Cyclohexylpropyl)-2(1H)-imidaxolidinon-1-yl-, 3-(3-Cycloheptylpropyl)-2(1H)-imidazolidinon-1-yl-, 3,4,5,6-Tetrahydro-2(1H)-pyrimidon-1-yl-, 3-Methyl-3,4,5,6-tetrahydro-2(1H)-pyrimidon-1-yl-, 3-Ethyl-3,4,5,6-tetrahydro-2(1H)-pyrimidon-1-yl-, 3-n-Propyl-3,4,5,6-tetrahydro-2(1H)-pyrimidon-1-yl-, 3-Isopropyl-3,4,5,6-tetrahydro-2(1H)-pyrimidon-1-yl-, 3-n-Butyl-3,4,5,6-tetrahydro-2(1H)-pyrimidon-1-yl-, 3-Isobutyl-3,4,5,6-tetrahydro-2(1H)-pyrimidon-1-yl-, 3-n-Pentyl-3,4,5,6-tetrahydro-2(1H)-pyrimidon-1-yl-, 3-n-Hexyl-3,4,5,6-tetrahydro-2(1H)-pyrimidon-1-yl-, 3-Cyclopen tyl-3,4,5,6-tetrahydro-2(1H)-pyrimidon-1-yl-, 3-Cyclohexyl-3,4,5,6-tetrahydro-2(1H)-pyrimidon-1-yl-, 3-Cycloheptyl-3,4,5,6-tetrahydro-2(1H)-pyrimidon-1-yl-, 3-Benzyl-3,4,5,6-tetrahydro-2(1H)-pyrimidon-1-yl-, 3-(3-Hydroxybenzyl)-3,4,5,6-tetrahydro-2(1H)-pyrimidon-1-yl-, 3-(4-Hydroxybenzyl)-3,4,5,6-tetrahydro-2(1H)-pyrimidon-1-yl, 3-(3-Methoxybenzyl)-3,4,5,6-tetrahydro-2-(1H)-pyrimidon-1-yl-, 3-(4-Methoxybenzyl)-3,4,5,6-tetrahydro-2(1H)-pyrimidon-1-yl-, 3-(3,4-Dihydroxybenzyl)-3,4,5,6-tetrahydro-2(1H)-pyrimidon-1-yl-,3-(3,4-Dimethoxybenzyl)-3,4,5,6-tetrahydro-2(1H)-pyrimidon-1-yl-,3-Cyclohexylmethyl-3,4,5,6-tetrahydro-2(1H)-pyrimidon-1-yl-, 3-Cycloheptylmethyl-3,4,5,6-tetrahydro-2(1H)-pyrimidon-1-yl-, 3-(2-Phenylethyl)-3,4,5,6-tetrahydro-2(1H)-pyrimidon-1-yl-, 3-(2-Cyclopentylethyl)-3,4,5,6-tetrahydro-2(1H)-pyrimidon-1-yl-, 3-(2-Cyclohexylethyl)-3,4,5,6-tetrahydro-2(1H)-pyrimidon-1-yl-, 3-(2-Cycloheptylethyl)-3,4,5,6-tetrahydro-2(1H)-pyrimidon-1-yl-, 3-(3-Phenylpropyl)-3,4,5,6-tetrahydro-2(1H)-pyrimidon-1-yl-, 3-(3-Cyclopentylpropyl)-3,4,5,6-tetrahydro-2(1H)-pyrimidon-1-yl-,3-(3-Cyclohexylpropyl)-3,4,5,6-tetrahydro-2(1H)-pyrimidon-1-yl-, 3-(3-Cycloheptylpropyl)-3,4,5,6-tetrahydro-2(1H)-pyrimidon-1-yl-,2-Chlorethylaminocarbonylamino-, 3-Chlorpropylaminocarbonylamino-, 2-Bromethylaminocarbonylamino-, 3-Brompropylaminocarbonylamino-, (N-2-Chlorethyl-methylaminocarbonyl)-amino-, (N-2-Bromethyl-methylaminocarbonyl)-amino-, (N-2-Chlorpropyl-methylaminocarbonyl)-amino-, tert.Butylcarbonylamino-, (N-2-Brompropyl-methylaminocarbonyl)-amino-, 2-Oxo-isoindolin-1-yl-, 3-Dimethylaminocarbonyl-2(1H)-imidazolidinon-1-yl-, 3-Diethylaminocarbonyl-2(1H)-imidazolidinon-1-yl-, 3-Di-n-propylaminocarbonyl-2(1H)-imidazolidinon-1-yl-, 3-Dimethylaminocarbonyl-3,4,5,6-tetrahydro-2(1H)-pyrimidon-1-yl-, 3-Diethylaminocarbonyl-3,4,5,6-tetrahydro-2(1H)-pyrimidon-1-yl-, 3-Diisopropylaminocarbonyl-3,4,5,6-tetrahydro-2(1H)-pyrimidon-1-yl-, 3-Chlor-propionylamino-, 4-Chlor-butanoylamino-, 5-Chlorpentanoylamino-, 6-Chlor-hexanoylamino-, 7-Chlor-heptanoylamino-, 3-Hydroxy-propionylamino-, 4-Hydroxy-butanoylamino-, 5-Hydroxy-pentanoylamino-, 6-Hydroxy-hexanoylamino-7-Hydroxy-heptanoylamino-, 2,2-Dimethylpropionylamino- oder tert.Butylaminogruppe,

für $R_2$ die des Wasserstoffatoms, der Methyl-, Ethyl-, n-Propyl- oder Isopropylgruppe,

für $R_3$ die der Methyl-, Ethyl-, n-Propyl-, Isopropyl-, n-Butyl-, Isobutyl-, tert.Butyl-, n-Pentyl-, n-Hexyl-, 1-Methylpropyl-, 1-Methylbutyl-, 2-Methylbutyl-, 3-Methylbutyl-, 1-Methylpentyl-, 2-Methylpentyl-, 3-Methylpentyl-, 4-Methylpentyl-, 1-Ethylpropyl- oder 1,1-Diethylethylgruppe,

für $R_4$ die der Carboxy-, Cyano-, 1H-Tetrazolyl-, 1-Triphenylmethyl-tetrazolyl-, Methoxycarbonyl-, Ethoxycarbonyl-, n-Propoxycarbonyl-, Isopropoxycarbonyl-, n-Butoxycarbonyl-, Isobutoxycarbonyl- oder tert.Butoxycarbonylgruppe und

für $R_5$ die des Wasserstoff-, Fluor-, Chlor- oder Bromatoms in Betracht.

Bevorzugte Verbindungen der obigen allgemeinen Formel I sind diejenigen, in denen

einer oder zwei der Reste $A_1$, $A_2$, $A_3$ oder $A_4$ ein Stickstoffatom und die verbleibenden Reste $A_1$, $A_2$, $A_3$ oder $A_4$ jeweils eine Methingruppe,

$R_1$ ein Fluoratom, eine am Stickstoffatom durch eine Phenyl-, Cycloalkyl-, Phenylalkyl- oder Cycloalkylalkylgruppe substituierte Amino- oder Alkylaminogruppe mit 1 bis 6 Kohlenstoffatomen, eine durch eine Phenyl-, Cycloalkyl-, Phenylalkyl- oder Cycloalkylalkylgruppe disubstituierte Aminogruppe, wobei die Substituenten gleich oder verschieden sein können, oder eine gegebenenfalls am Stickstoffatom durch eine Alkylgruppe mit 1 bis 6 Kohlenstoffatomen oder durch eine Phenyl-, Cycloalkyl-, Phenylalkyl- oder Cycloalkylalkylgruppe substituierte Acylaminogruppe, in welcher der Acylrest eine Alkanoylgruppe mit 2 bis 7 Kohlenstoffatomen, welche endständig durch ein Chloratom oder eine Hydroxygruppe substituiert sein kann, eine Alkoxycarbonylgruppe mit insgesamt 2 bis 4 Kohlenstoffatomen, eine Alkylsulfonylgruppe mit 1 bis 6 Kohlenstoffatomen, eine Formyl-, Benzoyl-, Benzolsulfonyl-, Phenylalkansulfonyl-, Naphthalensulfonyl-, 2-Carboxy-cyclohexylcarbonyl-, 2-Aminocarbonyl-cyclohexylcarbonyl-, Cycloalkylcarbonyl-, Phenylalkanoyl- oder Cycloalkylalkanoylgruppe darstellt, wobei die vorstehend erwähnten Phenylkerne durch ein Fluor-, Chlor- oder Bromatom, durch eine Methyl- oder Methoxygruppe mono- oder disubstituiert und die Substituenten gleich oder verschieden sein können, eine Alkylsultamgruppe mit 3 bis 5 Kohlenstoffatomen im Alkylteil, eine gegebenenfalls durch eine Alkyl- oder Hydroxycarbonylalkylgruppe substituierte Pyrrolidino-, Piperidino- oder Hexamethyleniminogruppe, eine Pyrrolidinon-, Piperidinon- oder Hexamethyleniminongruppe, eine gegebenenfalls ganz oder teilweise hydrierte Phthalimidogruppe oder eine Gruppe der Formel

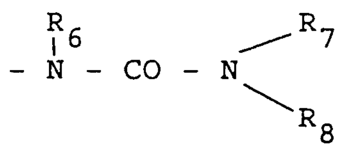
,

in welcher

$R_6$, $R_7$ und $R_8$, die gleich oder verschieden sein können, Wasserstoffatome, Alkylgruppen mit 1 bis 8 Kohlenstoffatomen, Cycloalkyl-, Phenyl-, Cycloalkylalkyl- oder Phenylalkylgruppen oder

$R_7$ auch eine 2-Chlor-ethyl-, 2-Brom-ethyl-, 3-Chlor-propyl- oder 3-Brom-propylgruppe oder auch, wenn $R_6$ und $R_7$ zusammen eine Ethylen- oder Propylengruppe darstellen, eine Dialkylaminocarbonylgruppe, oder

$R_6$ und $R_7$ zusammen eine Ethylen- oder Propylengruppe darstellen, wobei, der Phenylkern der vorstehend erwähnten Phenyl- und Phenylalkylgruppen jeweils durch Hydroxy- oder Alkoxygruppen mono- oder disubstituiert und die Substituenten gleich oder verschieden sein können, und, soweit nichts anderes erwähnt wurde, die vorstehend erwähnten Alkyl- und Alkoxyteile jeweils 1 bis 3 Kohlenstoffatome, die Alkanoylteile 1 bis 4 Kohlenstoffatome und die Cycloalkylteile jeweils 5 bis 7 Kohlenstoffatome enthalten können,

$R_2$ ein Wasserstoffatom oder eine Alkylgruppe mit 1 bis 3 Kohlenstoffatomen,

$R_3$ eine Alkylgruppe mit 1 bis 6 Kohlenstoffatomen,

$R_4$ eine Carboxy-, Cyano-, 1H-Tetrazolyl- oder 1-Triphenylmethyl-tetrazolylgruppe oder eine Alkoxycarbonylgruppe mit insgesamt 2 bis 5 Kohlenstoffatomen und

$R_5$ ein Wasserstoff-, Fluor-, Chlor- oder Bromatom bedeuten, insbesondere diejenigen Verbindungen der allgemeinen Formel I, in denen

einer oder zwei der Reste $A_1$, $A_2$, $A_3$ oder $A_4$ ein Stickstoffatom und die verbleibenden Reste $A_1$, $A_2$, $A_3$ oder $A_4$ jeweils eine Methingruppe,

$R_1$ eine durch eine Cyclohexyl-, Cyclohexylmethyl-, Benzyl- oder Dimethoxybenzylgruppe substituierte Amino-, Methylamino- oder Ethylaminogruppe, eine durch eine Alkanoylgruppe mit 2 bis 6 Kohlenstoffatomen, durch eine Alkansulfonylgruppe mit 1 bis 4 Kohlenstoffatomen, durch eine Alkoxycarbonylgruppe mit insgesamt 2 bis 4 Kohlenstoffatomen, durch eine Cyclohexylcarbonyl-, Benzylcarbonyl-, 2-Aminocarbonyl-cyclohexylcarbonyl-, Benzolsulfonyl- oder Chlorbenzolsulfonylgruppe substituierte Amino-, Cyclohexylamino-, Cyclohexylmethyl- oder Alkylaminogruppe mit 1 bis 4 Kohlenstoffatomen, eine Alkylsultamgruppe mit 3 oder 4 Kohlenstoffatomen im Alkylteil, eine gegebenenfalls durch eine Methyl- oder Hydroxycarbonylmethylgruppe substituierte Pyrrolidino- oder Piperidinogruppe, eine Pyrrolidin-2-on-1-yl-

oder Piperidin-2-on-1-yl-gruppe, eine gegebenenfalls ganz hydrierte Phthalimido- oder 2-Oxo-isoindolin-1-yl-gruppe oder eine Gruppe der Formel

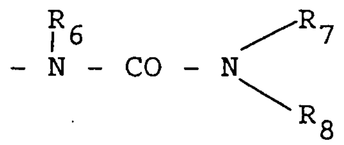
,

in welcher
$R_6$, $R_7$ und $R_8$, die gleich oder verschieden sein können, Wasserstoffatome, Alkylgruppen mit 1 bis 4 Kohlenstoffatomen, Cyclohexyl-, Phenyl-, Cyclohexylmethyl-, Benzyl-, Methoxybenzyl- oder Hydroxybenzylgruppen oder
$R_7$ auch eine 2-Chlor-ethyl-, 2-Brom-ethyl-, 3-Chlor-propyl- oder 3-Brom-propylgruppe oder auch, wenn $R_6$ und $R_7$ zusammen eine Ethylen- oder Propylengruppe darstellen, eine Dimethylaminocarbonylgruppe, oder
$R_6$ und $R_7$ zusammen eine Ethylen- oder Propylengruppe darstellen,
$R_2$ ein Wasserstoffatom, eine Methyl- oder Ethylgruppe,
$R_3$ eine Alkylgruppe mit 1 bis 5 Kohlenstoffatomen,
$R_4$ eine Carboxy- oder 1H-Tetrazolylgruppe und
$R_5$ ein Wasserstoffatom bedeuten,
deren 1-, 3-Isomerengemische, deren Tautomeren, deren Enantiomeren und deren Additionssalze mit organischen oder anorganischen Säuren oder Basen.

Besonders bevorzugte Verbindungen der allgemeinen Formel I sind diejenigen, in denen
einer oder zwei der Reste $A_1$, $A_2$, $A_3$ oder $A_4$ ein Stickstoffatom und die verbleibenden Reste $A_1$, $A_2$, $A_3$ oder $A_4$ jeweils eine Methingruppe,
$R_1$ eine durch eine Cyclohexyl- oder Cyclohexylmethylgruppe substituierte Amino-, Methylamino- oder Ethylaminogruppe, eine durch eine Alkanoylgruppe mit 2 bis 6 Kohlenstoffatomen, durch eine Alkansulfonylgruppe mit 1 bis 4 Kohlenstoffatomen, durch eine Alkoxycarbonylgruppe mit insgesamt 2 bis 4 Kohlenstoffatomen, durch eine Benzylcarbonyl-, 2-Aminocarbonyl-cyclohexylcarbonyl-, Benzolsulfonyl- oder Chlorbenzolsulfonylgruppe substituierte Cyclohexylamino- oder Cyclohexylmethylaminogruppe, eine Cyclohexylcarbonylaminogruppe, eine durch eine Alkansulfonylgruppe mit 1 bis 4 Kohlenstoffatomen, durch eine Cyclohexylcarbonyl-, 2-Aminocarbonyl-cyclohexylcarbonyl-, Benzolsulfonyl- oder Chlorbenzolsulfonylgruppe substituierte Cyclohexylamino-, Cyclohexylmethylamino- oder Alkylaminogruppe mit 1 bis 4 Kohlenstoffatomen, eine Alkylsultamgruppe mit 3 oder 4 Kohlenstoffatomen im Alkylteil, eine durch eine Methyl- oder Hydroxycarbonylmethylgruppe substituierte Pyrrolidino- oder Piperidinogruppe, eine Pyrrolidin-2-on-1-yl- oder Piperidin-2-on-1-yl-gruppe, eine gegebenenfalls ganz hydrierte Phthalimido- oder 2-Oxo-isoindolin-1-yl-gruppe oder eine Gruppe der Formel

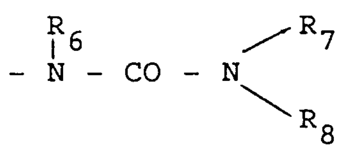
,

in welcher
einer der Reste $R_6$, $R_7$ oder $R_8$ eine Cyclopentyl-, Cyclohexyl- oder Cyclohexylmethylgruppe und
die verbleibenden Reste der Reste $R_6$, $R_7$ oder $R_8$, die gleich oder verschieden sein können, Wasserstoffatome, Alkylgruppen mit 1 bis 4 Kohlenstoffatomen, Cyclohexyl-, Phenyl-, Cyclohexylmethyl-, Benzyl-, Methoxybenzyl- oder Hydroxybenzylgruppen oder
$R_7$ eine 2-Chlor-ethyl-, 2-Brom-ethyl-, 3-Chlor-propyl- oder 3-Brom-propylgruppe und
$R_6$ und $R_8$, die gleich oder verschieden sein können, Wasserstoffatome, Alkylgruppen mit 1 bis 4 Kohlenstoffatomen, Cyclohexyl-, Phenyl-, Cyclohexylmethyl-, Benzyl-, Methoxybenzyl- oder Hydroxybenzylgruppen oder
$R_6$ und $R_7$ zusammen eine Ethylen- oder Propylengruppe und $R_8$ ein Wasserstoffatom, eine Alkylgruppen mit 1 bis 4 Kohlenstoffatomen, eine Cyclohexyl-, Phenyl-, Cyclohexylmethyl-, Benzyl-, Methoxybenzyl-, Hydroxybenzyl-, 2-Chlor-ethyl-, 2-Brom-ethyl-, 3-Chlor-propyl-, 3-Brom-propyl- oder Dimethylaminocarbo-

nylgruppe darstellen,
$R_2$ ein Wasserstoffatom, eine Methyl- oder Ethylgruppe,
$R_3$ eine Alkylgruppe mit 1 bis 5 Kohlenstoffatomen,
$R_4$ eine Carboxy- oder 1H-Tetrazolylgruppe und
$R_5$ ein Wasserstoffatom bedeuten,
deren 1-, 3-Isomerengemische, deren Tautomeren, deren Enantiomeren und deren Additionssalze mit organischen oder anorganischen Säuren oder Basen.

Erfindungsgemäß erhält man die Verbindungen nach folgenden Verfahren:

a) Cyclisierung einer Verbindung der allgemeinen Formel

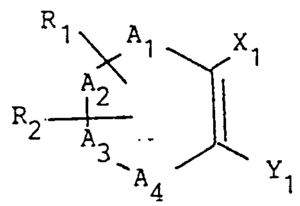

(II)

in der
$R_1$, $R_2$ und $A_1$ bis $A_4$ wie eingangs definiert sind,
einer der Reste $X_1$ oder $Y_1$ eine Gruppe der allgemeinen Formel

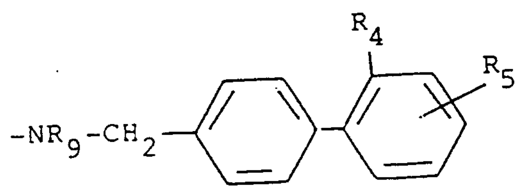

und der andere der Reste $X_1$ oder $Y_1$ eine Gruppe der allgemeinen Formel

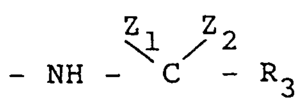

darstellen, wobei
$R_3$ und $R_5$ wie eingangs definiert sind,
$R_9$ ein Wasserstoffatom oder eine $R_3CO$-Gruppe, wobei $R_3$ wie vorstehend erwähnt definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder
$Z_1$ und $Z_2$ zusammen ein Sauerstoff- oder Schwefelatom,
eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, wobei jedoch einer der Reste $X_1$ oder $Y_1$ eine Gruppe der allgemeinen Formel

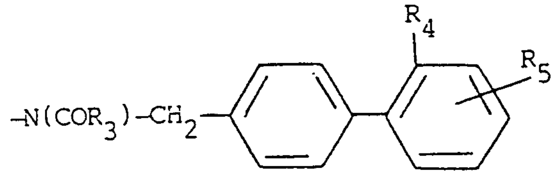

oder

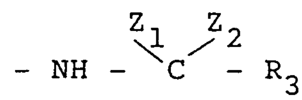

darstellen muß.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glyclomonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester oder Amid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kalium-tert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen Formel II im Reaktionsgemisch durch Reduktion einer entsprechenden o-Nitro-aminoverbindung gegebenenfalls in Gegenwart einer Carbonsäure der allgemeinen Formel $R_3COOH$ oder durch Acylierung einer entsprechenden o-Diaminoverbindung hergestellt wird. Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid wird anschließend mittels Reduktion in eine entsprechende Verbindung der allgemeinen Formel I übergeführt.

Die anschließende Reduktion des erhaltenen N-Oxids der Formel I wird vorzugsweise in einem Lösungsmittel wie Wasser, Wasser/Äthanol, Methanol, Eisessig, Essigsäureäthylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50°C, vorzugsweise jedoch bei Raumtemperatur durchgeführt.

b) Umsetzung einer Verbindung der allgemeinen Formel

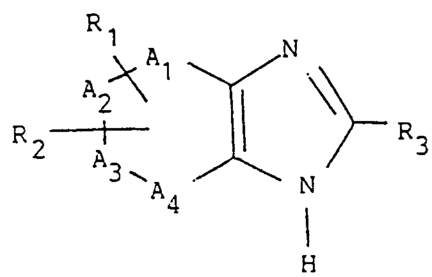

(III)

in der

$R_1$ bis $R_3$ und $A_1$ bis $A_4$ wie eingangs definiert sind,

mit einer Biphenylverbindung der allgemeinen Formel

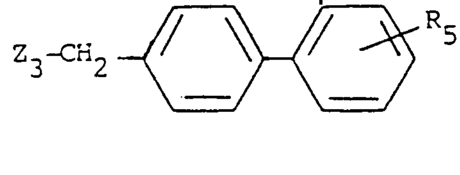

(IV)

in der

$R_4$ und $R_5$ wie eingangs definiert sind und

$Z_3$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt.

Die Umsetzung wird zweckmäßigerweise in einem Lösungsmittel oder Lösungsmittelgemisch wie Methylenchlorid, Diethylether, Aceton, Tetrahydrofuran, Dioxan, Dimethylsulfoxid oder Benzol gegebenenfalls in Gegenwart eines säurebindenden Mittels, wie Natriumcarbonat, Kaliumcarbonat, Natriumhydroxid, Kalium-tert.butylat, Natriumhydrid, Triethylamin oder Pyridin, wobei die beiden letzteren gleichzeitig auch als Lösungsmittel verwendet werden können, vorzugsweise bei Temperaturen zwischen 0 und 100 °C, z.B. bei Temperaturen zwischen Raumtemperatur und 50 °C, durchgeführt.

Bei der Umsetzung erhält man vorzugsweise ein Gemisch der 1- und 3-Isomeren, welches gewünschtenfalls anschließend, vorzugsweise chromatographisch unter Verwendung eines Trägers wie Kieselgel oder Aluminiumoxid, in das entsprechende 1- und 3-Isomere aufgetrennt wird.

c) Zur Herstellung einer Verbindung der allgemeinen Formel I, in der $R_4$ eine Carboxygruppe darstellt: Überführung einer Verbindung der allgemeinen Formel

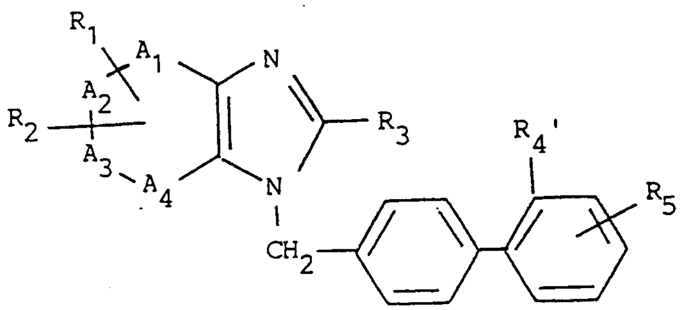

(V)

in der

$R_1$ bis $R_3$, $R_5$ und $A_1$ bis $A_4$ wie eingangs definiert sind und

$R_4$' eine mittels Hydrolyse, Thermolyse oder Hydrogenolyse in eine Carboxygruppe überführbare Gruppe darstellt.

Beispielsweise können funktionelle Derivate der Carboxygruppe wie deren unsubstituierte oder substituierte Amide, Ester, Thiolester, Orthoester, Iminoäther, Amidine oder Anhydride oder die Nitrilgruppe mittels Hydrolyse in eine Carboxygruppe,

Ester mit tertiären Alkoholen, z.B. der tert. Butylester, mittels Thermolyse in eine Carboxygruppe und

Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_4$' in einer Verbindung der allgemeinen Formel V eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50 °C in die Carboxygruppe übergeführt werden.

Bedeutet $R_4$' in einer Verbindung der allgemeinen Formel V beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie Trifluoressigsäure, p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Bedeutet $R_4$' in einer Verbindung der allgemeinen Formel V beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 5 bar abgespalten werden. Bei der

Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

Bedeutet $R_1$ in einer Verbindung der allgemeinen Formel V einen der eingangs erwähnten hydrolysierbaren Reste, so kann dieser während der Umsetzung in eine entsprechende Aminoverbindung übergeführt werden.

d) Zur Herstellung einer Verbindung der allgemeinen Formel I, in der $R_4$ eine 1H-Tetrazolylgruppe darstellt:
Abspaltung eines Schutzrestes von einer Verbindung der allgemeinen Formel

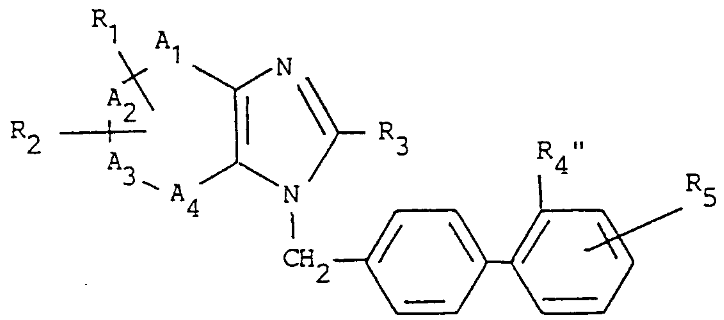

(VI)

in der
$R_1$ bis $R_3$, $R_5$ und $A_1$ bis $A_4$ wie eingangs definiert sind und
$R_4$'' eine in 1-Stellung durch einen Schutzrest geschützte 1H-Tetrazolylgruppe darstellt.

Als Schutzrest kommt beispielsweise die Triphenylmethyl-, Trimethylzinn-, Tributylzinn-, Triphenylzinn-, Propionsäurenitril- oder p-Nitrobenzylgruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100°C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150°C, vorzugsweise bei Temperaturen zwischen 120 und 140°C.

e) Zur Herstellung einer Verbindung der allgemeinen Formel I, in der $R_4$ eine 1H-Tetrazolylgruppe darstellt:
Umsetzung einer Verbindung der allgemeinen Formel

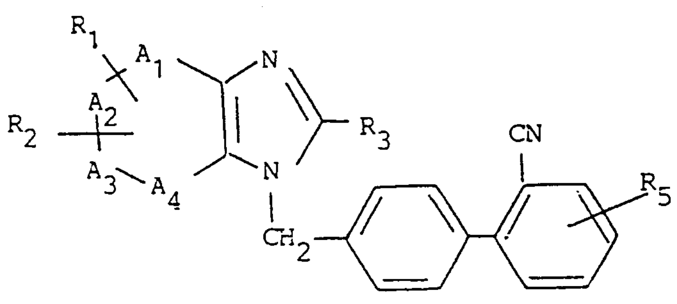

(VII)

in der
$R_1$ bis $R_3$, $R_5$ und $A_1$ bis $A_4$ wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150°C, vorzugsweise bei 125°C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung

von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

f) Zur Herstellung von Verbindungen der allgemeinen Formel I, in der $R_1$ eine gegebenenfalls durch eine Alkylgruppe mit 1 bis 6 Kohlenstoffatomen, durch eine Phenyl-, Cycloalkyl-, Phenylalkyl- oder Cycloalkylalkylgruppe substituierte Aminogruppe darstellt, wobei, soweit nichts anderes erwähnt wurde, die vorstehend erwähnten Alkylteile jeweils 1 bis 3 Kohlenstoffatome und die Cycloalkylteile jeweils 5 bis 7 Kohlenstoffatome enthalten können:
Überführung einer Verbindung der Formel

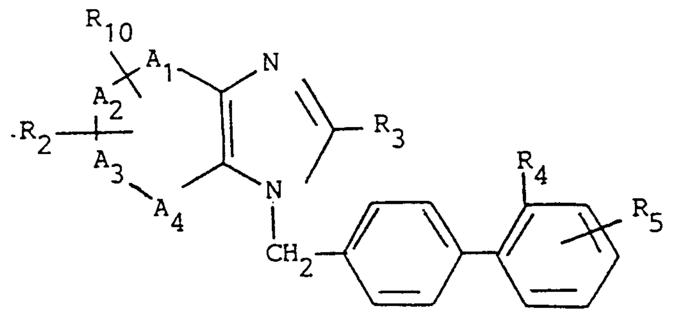

(VIII)

in der
$R_2$ bis $R_5$ und $A_1$ bis $A_4$ wie eingangs definiert sind und
$R_{10}$ eine mittels Hydrolyse, Hydrogenolyse oder Umamidierung in eine gegebenenfalls durch eine Alkylgruppe mit 1 bis 6 Kohlenstoffatomen, durch eine Phenyl-, Cycloalkyl-, Phenylalkyl- oder Cycloalkylalkylgruppe substituierte Aminogruppe überführbare Gruppe darstellt, wobei, soweit nichts anderes erwähnt wurde, die vorstehend erwähnten Alkylteile jeweils 1 bis 3 Kohlenstoffatome und die Cycloalkylteile jeweils 5 bis 7 Kohlenstoffatome enthalten können.

Beispielsweise können Acylaminogruppen, z.B. die Valeroylamino-, Benzoylamino- oder Phthalimidogruppe, mittels Hydrolyse in eine Aminogruppe und
Iminogruppen, z.B. die Phthaliminogruppe, mittels Umamidierung in eine Aminogruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_{10}$ eine Phthalimidogruppe, so kann diese Gruppe in Gegenwart einer primären organischen Base wie Methylamin, Ethylamin oder Propylamin oder mit Hydrazin gegebenenfalls in einem geeigneten Lösungsmittel wie Methanol, Ethanol, Isopropanol, Dimethylformamid, Methanol/Dimethylformamid oder Methanol/Wasser durch Umamidierung bei Temperaturen zwischen 0 und 50°C, vorzugsweise jedoch bei Raumtemperatur, besonders vorteilhaft in eine Aminogruppe übergeführt werden.

g) Zur Herstellung von Verbindungen der allgemeinen Formel I, in der $R_1$ eine Gruppe der Formel

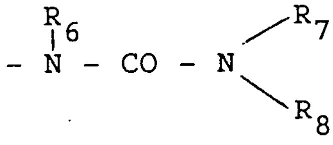

darstellt:
Umsetzung einer Verbindung der Formel

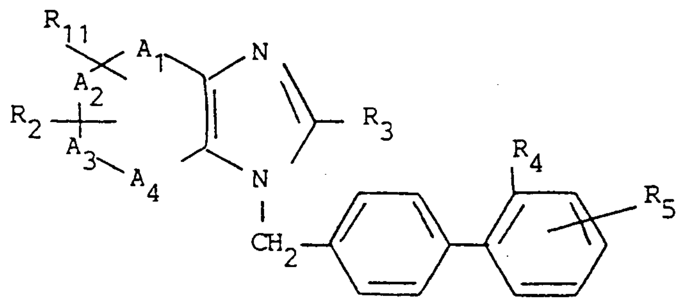

(IX)

in der
$R_2$ bis $R_5$ und $A_1$ bis $A_4$ wie eingangs definiert sind und
$R_{11}$ eine $R_6$NH-Gruppe darstellt, wobei $R_6$ wie eingangs definiert ist, mit einer Verbindung der Formel

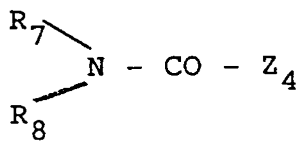

,(X)

in der
$R_7$ und $R_8$ wie eingangs definiert sind,
$Z_4$ eine nukleophile Austrittsgruppe wie ein Chlor- oder Bromatom oder auch
$Z_4$ zusammen mit $R_7$ eine Stickstoff-Kohlenstoff-Bindung bedeuten.

Die Umsetzung wird vorzugsweise in einem Lösungsmittel wie Tetrahydrofuran, Dioxan, Ethylenchlorid oder Benzol gegebenenfalls in Gegewart eines säurebindenden Mittels wie Triethylamin oder Pyridin zweckmäßigerweise bei Temperaturen zwischen 0 und 100°C, vorzugsweise bei Temperaturen zwischen 20 und 80°C, durchgeführt.

h) Zur Herstellung von Verbindungen der allgemeinen Formel I, in der $R_1$ eine gegebenenfalls am Stickstoffatom durch eine Alkylgruppe mit 1 bis 6 Kohlenstoffatomen, durch eine Phenyl-, Cycloalkyl-, Phenylalkyl- oder Cycloalkylalkylgruppe substituierte N-Acylaminogruppe bedeutet:
Acylierung einer Verbindung der allgemeinen Formel

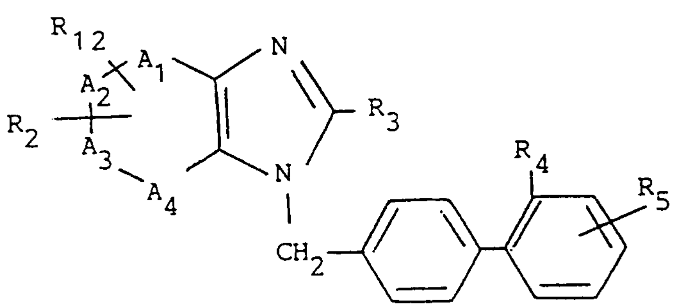

,(XI)

in der
$R_2$ bis $R_5$ und $A_1$ bis $A_4$ wie eingangs definiert sind und
$R_{12}$ eine gegebenenfalls durch eine Alkylgruppe mit 1 bis 6 Kohlenstoffatomen, durch eine Phenyl-, Cycloalkyl-, Phenylalkyl- oder Cycloalkylalkylgruppe substituierte Aminogruppe bedeutet, wobei, soweit nichts anderes erwähnt wurde, die vorstehend erwähnten Alkyteile jeweils 1 bis 3 Kohlenstoffatome und die Cycloalkylteile jeweils 5 bis 7 Kohlenstoffatome enthalten können, mit einer Verbindung der allgemeinen Formel

$R_{13}$ - W - OH ,(XII)

in der
W eine Carbonyl- oder Sulfonylgruppe,

$R_{13}$ eine Alkylgruppe mit 1 bis 6 Kohlenstoffatomen, eine Alkoxygruppe mit 1 bis 3 Kohlenstoffatomen, eine Phenylalkyl-, Cycloalkyl- oder Cycloalkylalkylgruppe mit jeweils 1 bis 3 Kohlenstoffatomen im Alkylteil und 5 bis 7 Kohlenstoffatomen im Cycloalkylteil, eine Phenyl-, Naphthyl-, 2-Carboxy-cyclohexyl- oder 2-Aminocarbonyl-cyclohexylgruppe, wobei die vorstehend erwähnten Phenylkerne durch ein Fluor-, Chlor- oder Bromatom, durch eine Methyl- oder Methoxygruppe mono- oder disubstituiert und die Substituenten gleich oder verschieden sein können, oder auch, wenn W eine Carbonylgruppe darstellt, ein Wasserstoffatom bedeuten, oder mit deren reaktionsfähigen Derivaten wie deren Säurehalogeniden, Säureestern oder Säureanhydriden.

Als reaktionsfähige Derivate einer Verbindung der Formel XII kommen beispielsweise deren Ester wie der Methyl-, Ethyl- oder Benzylester, deren Thioester wie der Methylthio- oder Ethylthioester, deren Halogenide wie das Säurechlorid, deren Anhydride oder Imidazolide in Betracht.

Die Umsetzung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid mit einer entsprechenden Carbonsäure in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid oder Methansulfonylchlorid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

i) Zur Herstellung einer Verbindung der Formel I, in der $R_6$ und $R_7$ zusammen eine Ethylen- oder n-Propylengruppe bedeuten:
Cyclisierung einer Verbindung der Formel

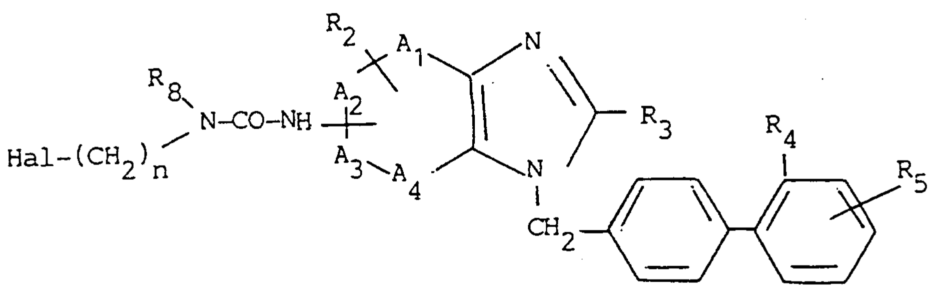

(XIII)

in der
$R_2$ bis $R_5$, $R_8$ und $A_1$ bis $A_4$ wie eingangs definiert sind, Hal ein Chlor-, Brom- oder Jodatom und
n die Zahl 2 oder 3 darstellen, und erforderlichenfalls anschließende Umsetzung mit einer Verbindung der Formel

$R_8$ - Hal ,(XIV)

in der
$R_8$ mit Ausnahme des Wasserstoffatoms wie eingangs definiert ist und
Hal ein Chlor-, Brom- oder Jodatom darstellt.

Die Cyclisierung und erforderlichenfalls die anschließende Alkylierung wird zweckmäßigerweise in einem Lösungsmittel wie Methanol, Ethanol, Benzol oder Dimethylsulfoxid gegebenenfalls in Gegenwart eines Phasentransferkatalysators wie Benzyltriethylammoniumbromid in Gegenwart eines säurebindenden Mittels wie Natriumhydroxid, Natriummethylat, Natriumethylat, Natriumhydrid oder Kalium-tert.butylat bei Temperaturen zwischen 20 und 100°C vorzugsweise bei Temperaturen zwischen 30 und 70°C, durchgeführt.

Ein so erfindungsgemäß erhaltenes Isomerengemisch einer Verbindung der allgemeinen Formel I kann gewünschtenfalls vorzugsweise chromatographisch unter Verwendung eines Trägers wie Kieselgel oder Aluminiumoxid in ihre Enantiomeren aufgetrennt werden.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxygruppe oder eine Tetrazolylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis XIV sind teilweise literaturbekannt oder man erhält diese nach literaturbekannten Verfahren.

So erhält man beispielsweise eine Verbindung der allgemeinen Formel II durch Alkylierung einer entsprechenden o-Amino-nitroverbindung und anschließende Reduktion der Nitrogruppe.

Eine als Ausgangsstoff verwendete Verbindung der allgemeinen Formeln III, V, VI, VII, VIII, IX, XI oder XIII erhält man durch Alkylierung eines entsprechenden o-Diamins oder einer entsprechenden o-Amino-nitroverbindung und anschließender Reduktion der Nitrogruppe und anschließender Cyclisierung einer so erhaltenen o-Diaminoverbindung oder durch NH-Alkylierung einer entsprechenden 1H-Verbindung, wobei das so erhaltene Isomerengemisch anschließend mittels üblicher Methoden, z.B. mittels Chromatographie, aufgetrennt werden kann.

Die neuen Verbindungen der allgemeinen Formel I und deren physiologisch verträgliche Additionssalze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 4'-[(2-n-Butyl-5-methyl-6-phthalimido-3H-imidazo[4,5-b]pyridin-3-yl)methyl]-biphenyl-2-carbonsäure,

B = 4'-[(2-n-Butyl-5-amino-3H-imidazo[4,5-b]pyridin-3-yl)methyl]biphenyl-2-carbonsäure,

C = 4'-[(2-Benzylamino-8-n-butyl-9H-purin-9-yl)-methyl]biphenyl-2-carbonsäure,

D = 4'-[[2-n-Butyl-5-methyl-6-(cis-hexahydrophthalimido)-3H-imidazo[4,5-b]pyridin-3-yl]-methyl]-biphenyl-2-carbonsäure,

E = 4'-[(2-n-Butyl-5-cyclohexylamino-3H-imidazo[4,5-b]pyridin-3-yl)methyl]biphenyl-2-carbonsäure,

F = 4'-[[2-n-Butyl-5-(N-cyclohexylcarbonyl-ethylamino)-3H-imidazo[4,5-b]pyridin-3-yl]-methyl]-biphenyl-2-carbonsäure,

G = 4'-[(2-n-Butyl-5-methoxy-3H-imidazo[4,5-b]pyridin-3-yl)methyl]-2-(1H-tetrazol-5-yl)biphenyl,

H = 4'-[[2-n-Butyl-5-(3-benzyl-3,4,5,6-tetrahydro-2(1H)-pyrimidinon-1-yl)-3H-imidazo[4,5-b]pyridin-3-yl]-methyl]biphenyl-2-carbonsäure,

I = 4'-[(2-n-Propyl-5-cyclohexylcarbonylamino-3H-imidazo[4,5-b]pyridin-3-yl)methyl]-2-(1H-tetrazol-5-yl)biphenyl,

J = 4'-[[2-Ethyl-5-(2,2-dimethyl-propionylamino)-3H-imidazo[4,5-b]pyridin-3-yl]-methyl]-2-(1H-tetrazol-5-yl)biphenyl und

K = 4'-[[2-n-Propyl-5-(2-methylpropylamino)-3H-imidazo[4,5-b]pyridin-3-yl]methyl]-2-(1H-tetrazol-5-yl)biphenyl

auf ihre biologischen Wirkungen wie folgt untersucht:

Ratten (männlich, 180-220 g) werden mit Hexobarbital-Natrium (150 mg/kg i.p.) narkotisiert. Nach Eintreten der Narkose wird eine Trachealkanüle gelegt, die Tiere werden despinalisiert und dann sofort mit einer Atempumpe künstlich beatmet. Der arterielle Blutdruck wird über eine Kanüle in der Arteria Carotis mittels eines Bell & Howell Druckaufnehmers registriert. Substanzen werden über eine Kanüle in die Vena Jugularis appliziert.

Test-Substanzen werden mit drei Dosierungen (10, 20 und 30 mg/kg i.v.) appliziert, wobei pro Tier eine Substanzdosis getestet wird. Drei Minuten nach der intravenösen Applikation der Testsubstanz wird Angiotensin-II in steigender Dosierung intravenös appliziert und so eine kumulative Dosis-Wirkungsbeziehung für Angiotensin-II in Gegenwart der Testsubstanzen erreicht. Der Meßparameter ist die Steigerung des arteriellen Blutdruckes.

Diese Dosis-Wirkungskurven werden mit Standardkurven für Angiotensin-II ohne Testsubstanzen verglichen. Mittels eines Computerprogramms werden die Rechtsverschiebungen der Dosis-Wirkungskurven von Angiotensin-II durch Testsubstanzen ermittelt und entsprechende $pA_2$-Werte für die Testsubstanzen berechnet.

Die gefundenen $pA_2$-Werte für die Substanzen A bis K liegen zwischen 5.1 und 8.5.

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen und deren

physiologisch verträgliche Additionssalze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Additionssalze zur Behandlung pulmonarer Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'schen Krankheit, des Parkinson-Syndroms, der Bulimie, sowie von Störungen kognitiver Funktionen.

Die zur Erzielung einer entsprechenden Wirkung erforderliche Dosierung beträgt zweckmäßigerweise bei intravenöser Gabe 20 bis 100 mg, vorzugsweise 30 bis 70 mg, und bei oraler Gabe 50 bis 200 mg, vorzugsweise 75 bis 150 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

Beispiel A

2-n-Butyl-7-methyl-imidazo[4,5-b]pyridin

3,6 g (29 mMol) 2,3-Diamino-4-methyl-pyridin und 3 ml Valeriansäure werden in 30 ml Phosphoroxytrichlorid 2 Stunden unter Rückfluß gekocht. Das Reaktionsgemisch wird im Vakuum eingedampft und der Rückstand mit 100 ml Eiswasser versetzt. Durch Zugabe von 20%iger Natronlauge wird das Gemisch neutralisiert und anschließend 2 x mit je 100 ml Essigester extrahiert. Nach Trocknung über Magnesiumsulfat und Verdampfen des Solvens erhält man ein Öl.
Ausbeute: 4,8 g (87 % der Theorie),
$R_f$-Wert: 0,40 (Kieselgel; Laufmittel: Ethylmethylketon/Xylol = 1:1)

| $C_{11}H_{15}N_3$ (189,26) | | | |
|---|---|---|---|
| Ber.: | C 69,81 | H 7,99 | N 22,20 |
| Gef.: | 69,60 | 7,91 | 21,96 |

Analog werden folgende Verbindungen erhalten:
8-n-Butyl-2-benzylamino-purin
Öl, $R_f$-Wert: 0,55 (Kieselgel; Laufmittel: Methylenchlorid/Ethanol = 9:1)
8-n-Butyl-2-n-butylamino-purin
Schmelzpunkt: 111-114° C
8-n-Butyl-2-cyclohexylamino-purin
Öl, $R_f$-Wert: 0,60 (Kieselgel; Laufmittel: Methylenchlorid/Ethanol = 9:1)
8-n-Butyl-2-ethoxy-purin
Schmelzpunkt: 181-182° C
8-n-Butyl-2-methoxy-purin
Schmelzpunkt: 166° C
8-n-Butyl-purin
Schmelzpunkt: 178-180° C
2-n-Butyl-5-brom-imidazo[4,5-b]pyridin
Schmelzpunkt: 223-225° C

Beispiel B

2-n-Butyl-5-valeroylamino-imidazo[4,5-b]pyridin

Hergestellt aus 2,6-Bis(n-pentanoylamino)-3-nitro-pyridin analog Beispiel 1.
Ausbeute: 83 % der Theorie,
Schmelzpunkt: 148-150 °C
Analog werden folgende Verbindungen erhalten:
2-n-Butyl-5-dimethylamino-imidazo[4,5-b]pyridin
Schmelzpunkt: 128-129 °C
2-n-Butyl-5-methyl-6-amino-imidazo[4,5-b]pyridin
Öl, $R_f$-Wert: 0,23 (Kieselgel; Laufmittel: Essigester/Ethanol/Ammoniak = 90:10:1)
2-n-Butyl-6-amino-imidazo[4,5-b]pyridin
Öl, $R_f$-Wert: 0,15 (Kieselgel; Laufmittel: Essigester/Ethanol/Ammoniak = 90:10:1)

Beispiel C

2-n-Butyl-5-(cis-hexahydrophthalimido)-imidazo[4,5-b]pyridin

Hergestellt durch Umsetzung von 2-n-Butyl-5-amino-imidazo[4,5-b]pyridin mit cis-Hexahydrophthalsäureanhydrid analog Beispiel 4. Ausbeute: 63 % der Theorie,
Öl, $R_f$-Wert: 0,50 (Kieselgel; Laufmittel: Methylenchlorid/Ethanol = 9:1)

Analog werden folgende Verbindungen erhalten:
2-n-Butyl-5-methyl-6-phthalimido-imidazo[4,5-b]pyridin
Öl, $R_f$-Wert: 0,77 (Kieselgel; Laufmittel: Methylenchlorid/Ethanol = 19:1)
2-n-Butyl-6-phthalimido-imidazo[4,5-b]pyridin
Öl, $R_f$-Wert: 0,68 (Kieselgel; Laufmittel: Essigester/Ethanol/Ammoniak = 90:10:1)
2-n-Propyl-5-(2,2-dimethylpropionylamino)-imidazo[4,5-b]pyridin
Öl, $R_f$-Wert: 0,42 (Kieselgel; Laufmittel: Essigester/Petrolether = 1:1)
2-n-Propyl-5-cyclohexylcarbonylamino-imidazo[4,5-b]pyridin
Öl, $R_f$-Wert: 0,67 (Kieselgel; Laufmittel: Essigester/Petrolether = 1:1)

Beispiel 1

4'-[(2-n-Butyl-5-methylamino-3H-imidazo[4,5-b]pyridin-3-yl)methyl]biphenyl-2-carbonsäure-tert.butylester

1,8 g (3,5 mMol) 2-[N-4-(2-tert.Butoxycarbonyl-phenyl)benzyl-N-pentanoylamino]-3-nitro-6-methylaminopyridin werden in 200 ml Ethanol gelöst, mit 1,0 g 10%igem Palladium auf Aktivkohle versetzt und 2 Stunden bei Raumtemperatur mit 5 bar Wasserstoffdruck hydriert. Nach beendigter Wasserstoffaufnahme wird vom Katalysator abfiltriert und eingedampft. Der Rückstand wird in 20 ml Eisessig gelöst und 30 Minuten auf dem Dampfbad erhitzt. Anschließend wird das Reaktionsgemisch eingedampft, der Rückstand in 100 ml Essigester gelöst und mit gesättigter Natriumhydrogencarbonatlösung und mit gesättigter Natriumchloridlösung gewaschen. Nach Trocknung über Magnesiumsulfat, Eindampfen des Solvens und Säulenchromatographie an Kieselgel (Korngröße: 0,06-0,2 mm, Elutionsmittel: Petrolether/Essigester = 1:1) erhält man ein farbloses Öl.
Ausbeute: 1,4 g (86 % der Theorie),
Öl, $R_f$-Wert: 0,27 (Kieselgel; Laufmittel: Petrolether/Essigester = 1:1)

| $C_{29}H_{34}N_4O_2$ (470,61) | | | |
|---|---|---|---|
| Ber.: | C 74,01 | H 7,28 | N 11,91 |
| Gef.: | 73,86 | 7,35 | 12,13 |

Analog werden folgende Verbindungen erhalten:
4'-[(2-n-Butyl-5-cyclohexylamino-3H-imidazo[4,5-b]pyridin-3-yl)methyl]biphenyl-2-carbonsäure-tert.butylester
Öl, $R_f$-Wert: 0,45 (Kieselgel; Laufmittel: Petrolether/Essigester = 1:1)
4'-[(2-n-Butyl-5-n-butylamino-3H-imidazo[4,5-b]pyridin-3-yl)methyl]biphenyl-2-carbonsäure-tert.butylester
Öl, $R_f$-Wert: 0,43 (Kieselgel; Laufmittel: Petrolether/Essigester = 1:1)
4'-[(2-n-Butyl-5-ethylamino-3H-imidazo[4,5-b]pyridin-3-yl)methyl]biphenyl-2-carbonsäure-tert.butylester

Öl, $R_f$-Wert: 0,36 (Kieselgel; Laufmittel: Petrolether/Essigester = 1:1)
4'-[(2-n-Butyl-5-cyclohexylmethylamino-3H-imidazo[4,5-b]pyridin-3-yl)methyl]biphenyl-2-carbonsäure-tert.butylester
Öl, $R_f$-Wert: 0,48 (Kieselgel; Laufmittel: Petrolether/Essigester = 1:1)
4'-[[2-n-Propyl-5-(2-methylpropylamino)-3H-imidazo[4,5-b]pyridin-3-yl]-methyl]-2-(1-triphenylmethyl-tetrazol-5-yl)biphenyl
Schmelzpunkt: 132-135 °C
4'-[[2-n-Butyl-5-(2,4-dimethoxybenzyl)amino-3H-imidazo[4,5-b]pyridin-3-yl]-methyl]biphenyl-2-carbonsäure-tert.butylester
Öl, $R_f$-Wert: 0,40 (Kieselgel; Laufmittel: Petrolether/Essigester = 1:1)
4'-[(2-n-Butyl-5-benzylamino-3H-imidazo[4,5-b]pyridin-3-yl)methyl]biphenyl-2-carbonsäure-tert.butylester
Öl, $R_f$-Wert: 0,36 (Kieselgel; Laufmittel: Petrolether/Essigester = 1:1)
4'-[(2-n-Butyl-5-methoxy-1H-imidazo[4,5-b]pyridin-1-yl)methyl]biphenyl-2-carbonsäure-tert.butylester
Öl, $R_f$-Wert: 0,44 (Kieselgel; Laufmittel: Ethylmethylketon/Xylol = 1:2)
4'-[(2-n-Butyl-5-(4-methyl-piperidino)-3H-imidazo[4,5-b]pyridin-3-yl]methyl]-2-(1-triphenylmethyl-tetrazol-5-yl)-biphenyl
Schmelzpunkt: 147-149 °C

## Beispiel 2

4'-[(2-n-Butyl-5-valeroylamino-3H-imidazo[4,5-b]pyridin-3-yl)methyl]biphenyl-2-carbonsäure-tert.butylester (I) und
4'-[(2-n-Butyl-5-valeroylamino-1H-imidazo[4,5-b]pyridin-1-yl)methyl]biphenyl-2-carbonsäure-tert.butylester (II)

8,9 g (10,6 mMol) 2-n-Butyl-5-valeroylamino-3H-imidazo[4,5-b]pyridin werden in 400 ml Aceton gelöst, mit 7,3 g (53 mMol) Kaliumcarbonat und mit 5,5 g (15,9 mMol) 4'-Brommethyl-biphenyl-2-carbonsäure-tert.butylester versetzt und unter Rühren 6 Stunden zum Rückfluß erhitzt. Das Reaktionsgemisch wird filtriert und das Filtrat zur Trockene eingedampft. Der Rückstand wird über eine Kieselgelsäule (Korngröße: 0,063-0,2 mm) gereinigt, wobei als Eluationsmittel ein Gemisch von Petrolether und Essigsäureethylester im Verhältnis 3:1 verwendet wird. Die einheitlichen Fraktionen werden zur Trockene eingeengt und mit Diethylether verreiben. Die so kristallisierten Feststoffe werden mit Ether gewaschen und getrocknet.

I: Ausbeute: 4,2 g (73 % der Theorie),
Schmelzpunkt: 102-104 °C

| $C_{33}H_{39}N_4O_3$ (539,70) | | | |
|---|---|---|---|
| Ber.: | C 73,20 | H 7,46 | N 10,36 |
| Gef.: | 73,18 | 7,72 | 10,19 |

II: Ausbeute: 1,1 g (20 % der Theorie),
Schmelzpunkt: 107-108 °C

| $C_{33}H_{39}N_4O_3$ (539,70) | | | |
|---|---|---|---|
| Ber.: | C 73,20 | H 7,46 | N 10,36 |
| Gef.: | 73,14 | 7,44 | 10,38 |

Analog werden folgende Verbindungen erhalten:
4'-[(2-n-Propyl-5-butanoylamino-3H-imidazo[4,5-b]pyridin-3-yl)methyl]biphenyl-2-carbonsäure-tert.butylester
Schmelzpunkt: 157-158 °C
4'-[(2-n-Propyl-5-butanoylamino-1H-imidazo[4,5-b]pyridin-1-yl)methyl]biphenyl-2-carbonsäure-tert.butylester
amorph, $R_f$-Wert: 0,38 (Kieselgel; Laufmittel: Essigester)
4'-[(2-n-Propyl-5-butanoylamino-3H-imidazo[4,5-b]pyridin-3-yl)methyl]biphenyl-4-brom-2-carbonsäure-tert.butylester
Öl, $R_f$-Wert: 0,40 (Kieselgel; Laufmittel: Methylenchlorid/Ethanol = 19:1)

4'-[(2-n-Butyl-5-dimethylamino-3H-imidazo[4,5-b]pyridin-3-yl)methyl]biphenyl-2-carbonsäure-tert.butylester
Öl, $R_f$-Wert: 0,37 (Aluminiumoxid; Laufmittel: Essigester/Petrolether = 1:1)
4'-[(2-n-Butyl-5-brom-3H-imidazo[4,5-b]pyridin-3-yl)methyl]biphenyl-2-carbonsäure-tert.butylester
Öl, $R_f$-Wert: 0,55 (Kieselgel; Laufmittel: Methylethylketon/Xylol = 1:2)
4'-[(2-n-Butyl-5-brom-1H-imidazo[4,5-b]pyridin-1-yl)methyl]biphenyl-2-carbonsäure-tert.butylester
Öl, $R_f$-Wert: 0,20 (Kieselgel; Laufmittel: Methylethylketon/Xylol = 1:2)
4'-[(2-n-Butyl-5-methyl-6-phthalimido-3H-imidazo[4,5-b]pyridin-3-yl)methyl]biphenyl-2-carbonsäure-tert.butylester
Öl, $R_f$-Wert: 0,52 (Kieselgel; Laufmittel: Methylethylketon/Xylol = 1:4)
4'-[(2-n-Butyl-5-methyl-6-phthalimido-1H-imidazo[4,5-b]pyridin-1-yl)methyl]biphenyl-2-carbonsäure-tert.butylester
Öl, $R_f$-Wert: 0,18 (Kieselgel; Laufmittel: Methylethylketon/Xylol = 1:1)
4'-[(2-n-Butyl-6-phthalimido-3H-imidazo[4,5-b]pyridin-3-yl)methyl]biphenyl-2-carbonsäure-tert.butylester
Öl, $R_f$-Wert: 0,62 (Kieselgel; Laufmittel: Methylethylketon/Xylol = 1:1)
4'-[(2-n-Butyl-6-phthalimido-1H-imidazo[4,5-b]pyridin-1-yl)methyl]biphenyl-2-carbonsaure-tert.butylester
Öl, $R_f$-Wert: 0,43 (Kieselgel; Laufmittel: Methylethylketon/Xylol = 1:1)
4'-[(2-n-Butyl-6-hexahydrophthalimido-3H-imidazo[4,5-b]pyridin-3-yl)-methyl]biphenyl-2-carbonsäure-tert.butylester
Öl, $R_f$-Wert: 0,58 (Kieselgel; Laufmittel: Methylethylketon/Xylol = 1:1)
4'-[(2-n-Butyl-6-hexahydrophthalimido-1H-imidazo[4,5-b]pyridin-1-yl)-methyl]biphenyl-2-carbonsäure-tert.butylester
Öl, $R_f$-Wert: 0,31 (Kieselgel; Laufmittel: Methylethylketon/Xylol = 1:1)
4'-[[2-n-Butyl-5-(cis-hexahydrophthalimido)-3H-imidazo[4,5-b]pyridin-3-yl]methyl]-2-(1-triphenylmethyl-tetrazol-5-yl)biphenyl
Öl, $R_f$-Wert: 0,45 (Kieselgel; Laufmittel: Essigester)
4'-[(2-Benzylamino-8-n-butyl-9H-purin-9-yl)methyl]biphenyl-2-carbonsäure-tert.butylester
Öl, $R_f$-Wert: 0,45 (Kieselgel; Laufmittel: Essigester)
4'-[(2-n-Butylamino-8-n-butyl-9H-purin-9-yl)methyl]biphenyl-2-carbonsäure-methylester
Schmelzpunkt: 125-126 °C
4'-[(2-Cyclohexylamino-8-n-butyl-9H-purin-9-yl)methyl]biphenyl-2-carbonsäure-methylester
Öl, $R_f$-Wert: 0,52 (Kieselgel; Laufmittel: Methylenchlorid/Ethanol = 95:5)
4'-[(2-Ethoxy-8-n-butyl-9H-purin-9-yl)methyl]biphenyl-2-carbonsäure-methylester
Öl, $R_f$-Wert: 0,55 (Kieselgel; Laufmittel: Methylenchlorid/Ethanol = 95:5)
4'-[(2-Methoxy-8-n-butyl-9H-purin-9-yl)methyl]biphenyl-2-carbonsäure-methylester
Öl, $R_f$-Wert: 0,50 (Kieselgel; Laufmittel: Methylenchlorid/Ethanol = 95:5)
4'-[[2-n-Propyl-5-(2,2-dimethylpropionylamino)-3H-imidazo[4,5-b]pyridin-3-yl]methyl]-2-(1H-tetrazol-5-yl)-biphenyl
Schmelzpunkt: 177-178 °C
4'-[(2-n-Propyl-5-cyclohexylcarbonylamino-3H-imidazo[4,5-b]pyridin-3-yl)methyl]-2-(1H-tetrazol-5-yl)biphenyl
Schmelzpunkt: 183-184 °C

## Beispiel 3

### 4'-[(2-n-Butyl-5-amino-3H-imidazo[4,5-b]pyridin-3-yl)methyl]biphenyl-2-carbonsäure-tert.butylester

3,4 g (6,3 mMol) 4'-[(2-n-Butyl-5-valeroylamino-3H-imidazo[4,5-b]pyridin-3-yl)methyl]biphenyl-2-carbonsäure-tert.butylester werden in 75 ml Ethanol gelöst, mit 35 ml 2N Natronlauge versetzt und unter Rühren 8 Stunden auf 80 °C erhitzt. Das Reaktionsgemisch wird eingedampft, in Essigester aufgenommen und mit Wasser extrahiert. Die wässrige Phase wird 2 x mit ca. 100 ml Essigester reextrahiert. Die organischen Phasen werden vereinigt, mit gesättigter Kochsalzlösung gewaschen und über Magnesiumsulfat getrocknet. Nach Verdampfen des Solvens wird der Rückstand durch Verreiben mit Petrolether kristallisiert.
Ausbeute: 2,1 g (73 % der Theorie),
Schmelzpunkt: 112-124 °C

| $C_{28}H_{32}N_4O_2$ (456,59) | | | |
|---|---|---|---|
| Ber.: | C 73,66 | H 7,07 | N 12,21 |
| Gef.: | 73,72 | 7,20 | 12,12 |

Analog werden folgende Verbindungen erhalten:
4'-[(2-n-Propyl-5-amino-3H-imidazo[4,5-b]pyridin-3-yl)methyl]biphenyl-2-carbonsäure-tert.butylester
Öl, $R_f$-Wert: 0,50 (Kieselgel; Laufmittel: Essigester)
4'-[(2-n-Butyl-5-methyl-6-amino-3H-imidazo[4,5-b]pyridin-3-yl)methyl]biphenyl-2-carbonsäure-tert.butylester
Öl, $R_f$-Wert: 0,56 (Kieselgel; Laufmittel: Essigester/Ethanol/Ammoniak = 90:10:1)
4'-[(2-n-Butyl-5-methyl-6-amino-1H-imidazo[4,5-b]pyridin-1-yl)methyl]biphenyl-2-carbonsäure-tert.butylester
Öl, $R_f$-Wert: 0,48 (Kieselgel; Laufmittel: Essigester/Ethanol/Ammoniak = 90:10:1)

## Beispiel 4

4'-[[2-n-Butyl-5-(N-acetyl-cyclohexylamino)-3H-imidazo[4,5-b]pyridin-3-yl]methyl]biphenyl-2-carbonsäure

0,3 g (0,62 mMol) 4'-[(2-n-Butyl-5-cyclohexylamino-3H-imidazo[4,5-b]pyridin-3-yl)methyl]biphenyl-2-carbonsäure werden in 3,0 g (38 mMol) Acetylchlorid gelöst und 2 Stunden unter Rückfluß gekocht. Das Reaktionsgemisch wird eingedampft, der Rückstand mit Wasser versetzt und mit wässriger Ammoniaklösung neutralisiert. Der nach Ansäuerung mit Eisessig gebildete Niederschlag wird abgesaugt, mit Wasser gewaschen und getrocknet.
Ausbeute: 0,22 g (68 % der Theorie),
Schmelzpunkt: 121-123 °C

| $C_{32}H_{36}N_4O_3$ (524,67) | | | |
|---|---|---|---|
| Ber.: | C 73,26 | H 6,92 | N 10,68 |
| Gef.: | 72,43 | 6,93 | 10,96 |

Analog werden folgende Verbindungen erhalten:
4'-[(2-n-Propyl-5-benzylcarbonylamino-3H-imidazo[4,5-b]pyridin-3-yl)methyl]biphenyl-2-carbonsäure-tert.butylester
Öl, $R_f$-Wert: 0,36 (Kieselgel; Laufmittel: Petrolether/Essigester = 1:1)
4'-[[2-n-Butyl-5-(cis-hexahydrophthalimido)-3H-imidazo[4,5-b]pyridin-3-yl]methyl]biphenyl-2-carbonsäure-tert.butylester
Öl, $R_f$-Wert: 0,66 (Kieselgel; Laufmittel: Essigester)
4'-[[2-n-Butyl-5-(N-acetyl-n-butylamino)-3H-imidazo[4,5-b]pyridin-3-yl]methyl]biphenyl-2-carbonsäure-tert.butylester
Öl, $R_f$-Wert: 0,21 (Kieselgel; Laufmittel: Petrolether/Essigester = 1:1)
4'-[[2-n-Butyl-5-(N-ethoxycarbonyl-ethylamino)-3H-imidazo[4,5-b]pyridin-3-yl]methyl]biphenyl-2-carbonsäure-tert.butylester
Öl, $R_f$-Wert: 0,45 (Kieselgel; Laufmittel: Petrolether/Essigester = 1:1)
4'-[[2-n-Butyl-5-(N-cyclohexylcarbonyl-ethylamino)-3H-imidazo[4,5-b]pyridin-3-yl]methyl]biphenyl-2-carbonsäure-tert.butylester
Öl, $R_f$-Wert: 0,34 (Kieselgel; Laufmittel: Petrolether/Essigester = 1:1)
4'-[(2-n-Butyl-5-N-[(2-methyl-propionyl)-n-butylamino]-3H-imidazo[4,5-b]pyridin-3-yl]methyl]biphenyl-2-carbonsäure-tert.butylester
Öl, $R_f$-Wert: 0,48 (Kieselgel; Laufmittel: Petrolether/Essigester = 1:1)
4'-[[2-n-Butyl-5-(N-ethoxycarbonyl-n-butylamino)-3H-imidazo[4,5-b]pyridin-3-yl]methyl]biphenyl-2-carbonsäure-tert.butylester
Öl, $R_f$-Wert: 0,53 (Kieselgel; Laufmittel: Petrolether/Essigester = 1:1)
4'-[(2-n-Butyl-5-methyl-6-dimethylaminocarbonylamino-1H-imidazo[4,5-b]pyridin-1-yl)methyl]biphenyl-2-carbonsäure-tert.butylester
amorph, $R_f$-Wert: 0,55 (Kieselgel; Laufmittel: Essigester/Ethanol/Ammoniak = 80:20:2)
4'-[(2-n-Butyl-5-methyl-6-dimethylaminocarbonylamino-3H-imidazo[4,5-b]pyridin-3-yl)methyl]biphenyl-2-carbonsäure-tert.butylester

amorph, $R_f$-Wert: 0,50 (Kieselgel; Laufmittel: Essigester/Ethanol/Ammoniak = 90:10:1)
4'-[[2-n-Butyl-5-methyl-6-(N-ethyl-cyclohexylcarbonylamino)-3H-imidazo[4,5-b]pyridin-3-yl]methyl]biphenyl-2-carbonsäure-tert.butylester
Öl, $R_f$-Wert: 0,46 (Kieselgel; Laufmittel: Methylenchlorid/Ethanol = 19:1)
4'-[[2-n-Butyl-5-methyl-6-(5-chloropentanoylamino)-1H-imidazo[4,5-b]pyridin-1-yl]methyl]biphenyl-2-carbonsäure-tert.butylester
Öl, $R_f$-Wert: 0,63 (Kieselgel; Laufmittel: Methylenchlorid/Ethanol = 9:1)
4'-[[2-n-Butyl-5-methyl-6-(N-acetyl-n-butylamino)-3H-imidazo[4,5-b]pyridin-3-yl]methyl]biphenyl-2-carbonsäure-tert.butylester
Öl, $R_f$-Wert: 0,48 (Kieselgel; Laufmittel: Methylketon/Ethanol = 1:2)
4'-[[2-n-Butyl-5-(4-chlorphenylsulfonylamino)-3H-imidazo[4,5-b]pyridin-3-yl]methyl]biphenyl-2-carbonsäure-tert.butylester
Öl, $R_f$-Wert: 0,39 (Kieselgel; Laufmittel: Methylenchlorid/Ethanol = 9:1)

Beispiel 5

4'-[[2-n-Butyl-5-(N-acetyl-cyclohexylmethylamino)-3H-imidazo[4,5-b]pyridin-3-yl)methyl]biphenyl-2-carbonsäure

Hergestellt analog Beispiel 4 aus 4'-[(2-n-Butyl-5-cyclohexylmethylamino-3H-imidazo[4,5-b]pyridin-3-yl)-methyl]biphenyl-2-carbonsäure und Acetylchlorid.
Ausbeute: 93 % der Theorie,
Schmelzpunkt: 180-185 °C

| $C_{33}H_{38}N_4O_3$ (538,20) | | | |
|---|---|---|---|
| Ber.: | C 73,58 | H 7,11 | N 10,40 |
| Gef.: | 73,56 | 7,37 | 10,46 |

Beispiel 6

4'-[(2-n-Butyl-5-propionylamino-3H-imidazo[4,5-b]pyridin-3-yl)methyl]biphenyl-2-carbonsaure

Hergestellt analog Beispiel 4 aus 4'-[(2-n-Butyl-5-amino-3H-imidazo[4,5-b]pyridin-3-yl)methyl]biphenyl-2-carbonsäure und Propionsäureanhydrid.
Ausbeute: 66 % der Theorie,
Schmelzpunkt: 278-281 °C

| $C_{27}H_{28}N_4O_3$ (456,55) | | | |
|---|---|---|---|
| Ber.: | C 71,03 | H 6,18 | N 12,27 |
| Gef.: | 70,86 | 6,23 | 12,40 |

Beispiel 7

4'-[[2-n-Butyl-5-(2-methylpropionylamino)-3H-imidazo[4,5-b]pyridin-3-yl]methyl]biphenyl-2-carbonsäure

Hergestellt analog Beispiel 4 aus 4'-[(2-n-Butyl-5-amino-3H-imidazo[4,5-b]pyridin-3-yl)methyl]biphenyl-2-carbonsäure und Isobuttersäurechlorid.
Ausbeute: 49 % der Theorie,
Schmelzpunkt: 250 °C

| $C_{28}H_{30}N_4O_3$ (430,58) | | | |
|---|---|---|---|
| Ber.: | C 71,47 | H 6,43 | N 11,91 |
| Gef.: | 71,26 | 6,31 | 11,66 |

Beispiel 8

4'-[[2-n-Butyl-5-(N-(3-chlorpropylaminocarbonyl)-amino)-3H-imidazo[4,5-b]pyridin-3-yl]methyl]biphenyl-2-carbonsäure-tert.butylester

1,3 g (2,9 mMol) 4'-[(2-n-Butyl-5-amino-3H-imidazo[4,5-b]pyridin -3-yl)methyl]biphenyl-2-carbonsäure-tert.butylester werden in 10 ml Dimethylformamid gelöst, mit 1,4 g (12 mMol) 3-Chlorpropylisocyanat versetzt und 40 Stunden bei Raumtemperatur gerührt. Nach Zugabe von 200 ml Eiswasser extrahiert man zweimal mit je 100 ml Essigester. Nach Trocknung über Magnesiumsulfat wird das Solvens verdampft und der Rückstand mit Ether verrieben. Der gebildete Niederschlag wird abgesaugt und getrocknet.
Ausbeute: 1,5 g (90 % der Theorie),
Schmelzpunkt: 207-209 °C

| $C_{32}H_{38}ClN_5O_3$ (646,17) | | | |
|---|---|---|---|
| Ber.: | C 66,71 | H 6,65 | N 12,16 |
| Gef.: | 66,71 | 6,72 | 12,47 |

Analog werden folgende Verbindungen erhalten:
4'-[[2-n-Propyl-5-(N-(3-chlorpropylaminocarbonyl)-amino)-3H-imidazo[4,5-b]pyridin-3-yl]methyl]biphenyl-2-carbonsäure-tert.butylester
Öl, $R_f$-Wert: 0,20 (Kieselgel; Laufmittel: Essigester)
4'-[[2-n-Butyl-5-(N-cyclohexylaminocarbonyl-ethylamino)-3H-imidazo[4,5-b]pyridin-3-yl]methyl]biphenyl-2-carbonsäure-tert.butylester
Öl, $R_f$-Wert: 0,30 (Kieselgel; Laufmittel: Essigester/Petrolether = 1:1)
4'-[[2-n-Butyl-5-(N-cyclohexylaminocarbonyl-n-butylamino)-3H-imidazo[4,5-b]pyridin-3-yl]methyl]biphenyl-2-carbonsäure-tert.butylester
Öl, $R_f$-Wert: 0,55 (Kieselgel; Laufmittel: Essigester/Petrolether = 1:2)
4'-[[2-n-Butyl-5-methyl-6-[N-(3-chlorpropylaminocarbonyl)amino]-3H-imidazo[4,5-b]pyridin-3-yl]methyl]-biphenyl-2-carbonsäure-tert.butylester
Öl, $R_f$-Wert: 0,58 (Kieselgel; Laufmittel: Essigester/Ethanol/Ammoniak = 90:10:1)
4'-[[2-n-Propyl-6-(N-benzylaminocarbonyl-isobutylamino)-3H-imidazo[4,5-b]pyridin-3-yl]methyl]-2-(1-triphenylmethyl-tetrazol-5-yl)biphenyl
Öl, $R_f$-Wert: 0,67 (Kieselgel; Laufmittel: Methylenchlorid/Ethanol = 19:1)
4'-[[2-n-Propyl-5-(N-cyclohexylaminocarbonyl-isobutylamino)-3H-imidazo[4,5-b]pyridin-3-yl]methyl]-2-(1-triphenylmethyl-tetrazol-5-yl)biphenyl
Öl, $R_f$-Wert: 0,62 (Kieselgel; Laufmittel: Methylenchlorid/Ethanol = 19:1)

Beispiel 9

4'-[(2-n-Butyl-5-cyclohexylaminocarbonylamino-3H-imidazo[4,5-b]pyridin-3-yl)methyl]biphenyl-2-carbonsäure

Hergestellt analog Beispiel 8 aus 4'-[(2-n-Butyl-5-amino-3H-imidazo[4,5-b]pyridin-3-yl)methyl]biphenyl-2-carbonsäure und Cyclohexylisocyanat.
Ausbeute: 46 % der Theorie,
Schmelzpunkt: 287-291 °C

| $C_{31}H_{35}N_5O_3$ (525,66) | | | |
|---|---|---|---|
| Ber.: | C 70,83 | H 6,71 | N 13,32 |
| Gef.: | 69,01 | 6,66 | 13,18 |

Beispiel 10

4'-[[2-n-Butyl-5-(3,4,5,6-tetrahydro-2(1H)-pyrimidinon-1-yl)-3H-imidazo[4,5-b]pyridin-3-yl]methyl]biphenyl-2-carbonsäure-tert.butylester

0,26 g (5,3 mMol) Natriumhydrid werden in 20 ml tert.Butanol gelöst. Nach 5 Minuten bei Raumtemperatur werden 0,58 g (1,0 mMol) 4'-[[2-n-Butyl-5-(N-(3-chlorpropylaminocarbonyl)amino)-3H-imidazo[4,5-b]-pyridin-3-yl]methyl]biphenyl-2-carbonsäure-tert.butylester portionsweise zugegeben. Man rührt 10 Stunden bei Raumtemperatur. Durch Zugabe von 2N Salzsäure wird der pH-Wert auf 5 gebracht und das tert.Butanol im Vakuum verdampft. Der Rückstand wird mit je 100 ml Essigester und Wasser verrührt, die organische Phase abgetrennt und die wässrige Phase 2 x mit je 50 ml Essigester extrahiert. Die vereinigten organischen Phasen werden mit gesättigter Kochsalzlösung gewaschen und über Magnesiumsulfat getrocknet. Nach Verdampfen des Solvens wird der Rückstand mit Ether verrieben, der gebildete Niederschlag abgesaugt und getrocknet.
Ausbeute: 0,4 g (74 % der Theorie),
$R_f$-Wert: 0,33 (Kieselgel; Laufmittel: Essigester/Ethanol = 9:1)

| $C_{32}H_{37}N_5O_3$ (539,68) | | | |
|---|---|---|---|
| Ber.: | C 71,22 | H 6,91 | N 12,98 |
| Gef.: | 70,99 | 6,98 | 12,81 |

Analog werden folgende Verbindungen erhalten:
4'-[[2-n-Propyl-5-(3,4,5,6-tetrahydro-2(1H)-pyrimidinon-1-yl)-3H-imidazo[4,5-b]pyridin-3-yl]methyl]biphenyl-2-carbonsäure-tert.butylester
Schmelzpunkt: 175 °C
4'-[[2-n-Butyl-5-methyl-6-(3,4,5,6-tetrahydro-2(1H)-pyrimidinon-1-yl)-3H-imidazo[4,5-b]pyridin-3-yl]methyl]-biphenyl-2-carbonsäure-tert.butylester
Schmelzpunkt: 200-202 °C
4'-[[2-n-Butyl-5-methyl-6-(2-oxo-piperidin-1-yl)-1H-imidazo[4,5-b]pyridin-1-yl]methyl]biphenyl-2-carbonsäure-tert.butylester
$R_f$-Wert: 0,49 (Kieselgel; Laufmittel: Methylenchlorid/Ethanol = 9:1)
4'-[[2-n-Butyl-5-methyl-6-(n-butansultam-1-yl)-3H-imidazo[4,5-b]pyridin-3-yl]methyl]biphenyl-2-carbonsäure-tert.butylester
$R_f$-Wert: 0,50 (Kieselgel; Laufmittel: Methylenchlorid/Ethanol = 19:1)
4'-[[2-n-Butyl-5-methyl-6-(2-oxo-piperidin-1-yl)-3H-imidazo[4,5-b]pyridin-3-yl]methyl]biphenyl-2-carbonsäure-tert.butylester
$R_f$-Wert: 0,60 (Kieselgel; Laufmittel: Methylenchlorid/Ethanol = 19:1)

Beispiel 11

4'-[[2-n-Butyl-5-(3-benzyl-3,4,5,6-tetrahydro-2(1H)-pyrimidinon-1-yl)-3H-imidazo[4,5-b]pyridin-3-yl]methyl]-biphenyl-2-carbonsäure-tert.butylester

0,4 g (0,74 mMol) 4'-[[2-n-Butyl-5-(N-(3-chlorpropylaminocarbonyl)-amino)-3H-imidazo[4,5-b]pyridin-3-yl)methyl]biphe nyl-2-carbonsäure-tert.butylester werden in 20 ml Dimethylformamid suspendiert und mit 0,04 g (0,85 mMol) Natriumhydrid versetzt. Man rührt 10 Minuten bei 60 °C, gibt 0,5 ml (4,25 mMol) Benzylbromid hinzu und rührt 3 Stunden bei Raumtemperatur. Man gießt das Reaktionsgemisch auf Eis und extrahiert 2 x mit je 100 ml Essigester. Die vereinigten organischen Phasen werden mit gesättigter Kochsalzlösung gewaschen und über Magnesiumsulfat getrocknet. Nach Verdampfen des Solvens erhält man ein farbloses Öl.

Ausbeute: 0,35 g (75 % der Theorie),
$R_f$-Wert: 0,41 (Kieselgel; Laufmittel: Methylenchlorid/Essigester = 1:1)
Massenspektrum: $M^+$ = 629

Analog werden folgende Verbindungen erhalten:
4'-[[2-n-Butyl-5-(3-methyl-3,4,5,6-tetrahydro-2(1H)-pyrimidinon-1-yl)-3H-imidazo[4,5-b]pyridin-3-yl]methyl]-biphenyl-2-carbonsäure-tert.butylester
Öl, $R_f$-Wert: 0,41 (Kieselgel; Laufmittel: Essigester/Ethanol = 9:1)
4'-[[2-n-Butyl-5-methyl-6-(3-benzyl-3,4,5,6-tetrahydro-2(1H)-pyrimidinon-1-yl)-3H-imidazo[4,5-b]pyridin-3-yl]-methyl]biphenyl-2-carbonsäure-tert.butylester
Öl, $R_f$-Wert: 0,44 (Kieselgel; Laufmittel: Methylenchlorid/Ethanol = 19:1)

Beispiel 12

4'-[[2-n-Butyl-5-(3-benzyl-3,4,5,6-tetrahydro-2(1H)-pyrimidinon-1-yl)-3H-imidazo[4,5-b]pyridin-3-yl]methyl]-biphenyl-2-carbonsäure

0,35 g (0,55 mMol) 4'-[[2-n-Butyl-5-(3-benzyl-3,4,5,6-tetrahydro-2(1H)-pyrimidinon-1-yl)-3H-imidazo[4,5-b]pyridin-3-yl) methyl]-biphenyl-2-carbonsäure-tert.butylester werden in 10 ml Methylenchlorid gelöst und mit 5 ml Trifluoressigsäure versetzt. Man rührt 65 Stunden bei Raumtemperatur. Das Solvens wird verdampft, der Rückstand in Eiswasser aufgenommen und mit Eisessig angesäuert. Der dabei gebildete Niederschlag wird abfiltriert, mit Wasser gewaschen und bei 60 °C getrocknet.
Ausbeute: 0,26 g (82 % der Theorie),
Schmelzpunkt: 168-170 °C

| $C_{35}H_{35}N_5O_3$ (573,70) | | | |
|---|---|---|---|
| Ber.: | C 73,28 | H 6,15 | N 12,21 |
| Gef.: | 73,37 | 6,51 | 12,12 |

Beispiel 13

4'-[[2-n-Butyl-5-(3-methyl-3,4,5,6-tetrahydro-2(1H)-pyrimidinon-1-yl)-3H-imidazo[4,5-b]pyridin-3-yl]methyl]-biphenyl-2-carbonsäure

Hergestellt analog Beispiel 12 aus 4'-[[2-n-Butyl-5-(3-methyl-3,4,5,6-tetrahydro-2(1H)-pyrimidinon-1-yl)-3H-imidazo[4,5-b]pyridin-3-yl]methyl]biphenyl-2-carbonsäure-tert.butylester und Trifluoressigsäure.
Ausbeute: 64 % der Theorie,
Schmelzpunkt: 249-252 °C

| $C_{29}H_{31}N_5O_3$ (497,60) | | | |
|---|---|---|---|
| Ber.: | C 70,00 | H 6,28 | N 14,00 |
| Gef.: | 69,85 | 6,40 | 13,89 |

Beispiel 14

4'-[[2-n-Butyl-5-(3,4,5,6-tetrahydro-2(1H)-pyrimidinon-1-yl)-3H-imidazo[4,5-b]pyridin-3-yl]methyl]biphenyl-2-carbonsäure

Hergestellt analog Beispiel 12 aus 4'-[[2-n-Butyl-5-(3,4,5,6-tetrahydro-2(1H)-pyrimidinon-1-yl)-3H-imidazo[4,5-b]pyridin-3-yl]-methyl]biphenyl-2-carbonsäure-tert.butylester und Trifluoressigsäure.
Ausbeute: 80 % der Theorie,
Schmelzpunkt: 300-302 °C

| $C_{28}H_{29}N_5O_3$ (483,58) | | | |
|---|---|---|---|
| Ber.: | C 68,27 | H 6,14 | N 14,22 |
| Gef.: | 68,47 | 6,11 | 14,28 |

Beispiel 15

4'-[[2-n-Propyl-5-(3,4,5,6-tetrahydro-2(1H)-pyrimidinon-1-yl)-3H-imidazo[4,5-b]pyridin-3-yl]methyl]biphenyl-2-carbonsäure

Hergestellt analog Beispiel 12 aus 4'-[[2-n-Propyl-5-(3,4,5,6-tetrahydro-2(1H)-pyrimidinon-1-yl)-3H-imidazo[4,5-b]pyridin-3-yl]methyl]biphenyl-2-carbonsäure-tert.butylester und Trifluoressigsäure.
Ausbeute: 92 % der Theorie,
Schmelzpunkt: 265-268° C

| $C_{27}H_{27}N_5O_3$ (469,55) | | | |
|---|---|---|---|
| Ber.: | C 69,07 | H 5,80 | N 14,92 |
| Gef.: | 68,87 | 5,78 | 15,00 |

Beispiel 16

4'-[(2-n-Butyl-5-cyclohexylmethylamino-3H-imidazo[4,5-b]pyridin-3-yl)methyl]biphenyl-2-carbonsäure

Hergestellt analog Beispiel 12 aus 4'-[(2-n-Butyl-5-cyclohexylmethylamino-3H-imidazo[4,5-b]pyridin-3-yl)methyl]biphenyl-2-carbonsäure-tert.butylester und Trifluoressigsäure. Ausbeute: 23,6 % der Theorie,
Schmelzpunkt: 202-205° C

| $C_{31}H_{36}N_4O_2$ (496,66) | | | |
|---|---|---|---|
| Ber.: | C 74,97 | H 7,31 | N 11,28 |
| Gef.: | 74,82 | 7,84 | 10,98 |

Beispiel 17

4'-[(2-n-Butyl-5-ethylamino-3H-imidazo[4,5-b]pyridin-3-yl)methyl]biphenyl-2-carbonsäure

Hergestellt analog Beispiel 12 aus 4'-[(2-n-Butyl-5-ethylamino-3H-imidazo[4,5-b]pyridin-3-yl)methyl]-biphenyl-2-carbonsäure-tert.butylester und Trifluoressigsäure.
Ausbeute: 76 % der Theorie,
Schmelzpunkt: 245-247° C

| $C_{26}H_{28}N_4O_2$ (428,54) | | | |
|---|---|---|---|
| Ber.: | C 72,87 | H 6,59 | N 13,09 |
| Gef.: | 72,72 | 6,65 | 12,84 |

Beispiel 18

4'-[(2-n-Butyl-5-n-butylamino-3H-imidazo[4,5-b]pyridin-3-yl)methyl]biphenyl-2-carbonsäure

Hergestellt analog Beispiel 12 aus 4'-[(2-n-Butyl-5-n-butylamino-3H-imidazo[4,5-b]pyridin-3-yl)methyl]-

biphenyl-2-carbonsäure-tert.butylester und Trifluoressigsäure.
Ausbeute: 67 % der Theorie,
Schmelzpunkt: 217-219 °C

| $C_{28}H_{32}N_4O_2$ (456,59) | | | |
|---|---|---|---|
| Ber.: | C 73,66 | H 7,06 | N 12,27 |
| Gef.: | 73,46 | 7,03 | 12,17 |

Beispiel 19

4'-[(2-n-Butyl-5-cyclohexylamino-3H-imidazo[4,5-b]pyridin-3-yl)methyl]biphenyl-2-carbonsäure

Hergestellt analog Beispiel 12 aus 4'-[(2-n-Butyl-5-cyclohexylamino-3H-imidazo[4,5-b]pyridin-3-yl)-methyl]biphenyl-2-carbonsäure-tert.butylester und Trifluoressigsäure.
Ausbeute: 67 % der Theorie,
Schmelzpunkt: 221-224 °C

| $C_{30}H_{34}N_4O_2$ (482,63) | | | |
|---|---|---|---|
| Ber.: | C 73,29 | H 7,18 | N 11,40 |
| Gef.: | 73,51 | 6,95 | 11,25 |

Beispiel 20

4'-[(2-n-Butyl-5-methylamino-3H-imidazo[4,5-b]pyridin-3-yl)methyl]biphenyl-2-carbonsäure

Hergestellt analog Beispiel 12 aus 4'-[(2-n-Butyl-5-methylamino-3H-imidazo[4,5-b]pyridin-3-yl)methyl]-biphenyl-2-carbonsäure-tert.butylester und Trifluoressigsäure. Ausbeute: 61 % der Theorie,
Schmelzpunkt: 274-277 °C

| $C_{25}H_{26}N_4O_2$ (414,51) | | | |
|---|---|---|---|
| Ber.: | C 72,44 | H 6,32 | N 13,52 |
| Gef.: | 72,26 | 6,26 | 13,30 |

Beispiel 21

4'-[(2-n-Butyl-5-amino-3H-imidazo[4,5-b]pyridin-3-yl)methyl]biphenyl-2-carbonsäure

Hergestellt analog Beispiel 12 aus 4'-[(2-n-Butyl-5-amino-3H-imidazo[4,5-b]pyridin-3-yl)methyl]biphenyl-2-carbonsäure-tert.butylester und Trifluoressigsäure.
Ausbeute: 35 % der Theorie,
Schmelzpunkt: 238-240 °C

| $C_{24}H_{24}N_4O_2$ (400,48) | | | |
|---|---|---|---|
| Ber.: | C 71,98 | H 6,04 | N 13,99 |
| Gef.: | 71,90 | 5,96 | 13,86 |

Beispiel 22

4'-[(2-n-Butyl-5-dimethylamino-3H-imidazo[4,5-b]pyridin-3-yl)methyl]biphenyl-2-carbonsäure

Hergestellt analog Beispiel 12 aus 4'-[(2-n-Butyl-5-dimethylamino-3H-imidazo[4,5-b]pyridin-3-yl)-methyl]biphenyl-2-carbonsäure-tert.butylester und Trifluoressigsäure.
Ausbeute: 81 % der Theorie,
Schmelzpunkt: 213-215 °C

| $C_{26}H_{28}N_4O_2$ (428,54) | | | |
|---|---|---|---|
| Ber.: | C 72,87 | H 6,57 | N 13,08 |
| Gef.: | 72,82 | 6,73 | 12,90 |

Beispiel 23

4'-[(2-n-Butyl-5-benzylamino-3H-imidazo[4,5-b]pyridin-3-yl)methyl]biphenyl-2-carbonsäure

Hergestellt analog Beispiel 12 aus 4'-[(2-n-Butyl-5-benzylamino-3H-imidazo[4,5-b]pyridin-3-yl)methyl]-biphenyl-2-carbonsäure-tert.butylester und Trifluoressigsäure.
Ausbeute: 67 % der Theorie,
Schmelzpunkt: 224-225 °C

| $C_{31}H_{30}N_4O_2$ (490,61) | | | |
|---|---|---|---|
| Ber.: | C 75,89 | H 6,16 | N 11,42 |
| Gef.: | 75,70 | 6,24 | 11,37 |

Beispiel 24

4'-[[2-n-Butyl-5-(2,4-dimethoxybenzylamino)-3H-imidazo[4,5-b]pyridin-3-yl]methyl]biphenyl-2-carbonsäure

Hergestellt analog Beispiel 12 aus 4'-[[2-n-Butyl-5-(2,4-dimethoxybenzylamino)-3H-imidazo[4,5-b]-pyridin-3-yl)methyl]biphenyl-2-carbonsäure-tert.butylester und Trifluoressigsäure.
Ausbeute: 73 % der Theorie,
Schmelzpunkt: 223-226 °C

| $C_{33}H_{34}N_4O_4$ (490,61) | | | |
|---|---|---|---|
| Ber.: | C 71,98 | H 6,22 | N 10,18 |
| Gef.: | 71,70 | 6,21 | 10,16 |

Beispiel 25

4'-[[2-n-Butyl-5-(N-isobutyryl-n-butylamino)-3H-imidazo[4,5-b]pyridin-3-yl]methyl]biphenyl-2-carbonsäure

Hergestellt analog Beispiel 12 aus 4'-[[(2-n-Butyl-5-(N-isobutyryl-n-butylamino)-3H-imidazo[4,5-b]pyridin-3-yl]methyl]biphenyl-2-carbonsäure-tert.butylester und Trifluoressigsäure.
Ausbeute: 33 % der Theorie,
Schmelzpunkt: 186-189 °C

| $C_{32}H_{38}N_4O_3$ (490,61) | | | |
|---|---|---|---|
| Ber.: | C 72,98 | H 7,27 | N 10,64 |
| Gef.: | 73,09 | 7,45 | 10,53 |

Beispiel 26

4'-[[(2-n-Butyl-5-(N-acetyl-n-butylamino)-3H-imidazo[4,5-b]pyridin-3-yl]methyl]biphenyl-2-carbonsäure

Hergestellt analog Beispiel 12 aus 4'-[[2-n-Butyl-5-(N-acetyl-n-butylamino)-3H-imidazo[4,5-b]pyridin-3-yl]methyl]biphenyl-2-carbonsäure-tert.butylester und Trifluoressigsäure.
Ausbeute: 36 % der Theorie,
Schmelzpunkt: 173-175 °C

| $C_{30}H_{34}N_4O_3$ (498,63) | | | |
|---|---|---|---|
| Ber.: | C 72,26 | H 6,87 | N 11,24 |
| Gef.: | 72,39 | 7,00 | 11,07 |

Beispiel 27

4'-[[2-n-Butyl-5-(N-cyclohexylcarbonyl-ethylamino)-3H-imidazo[4,5-b]pyridin-3-yl]methyl]biphenyl-2-carbonsäure

Hergestellt analog Beispiel 12 aus 4'-[(2-n-Butyl-5-(N-cyclohexylcarbonyl-ethylamino)-3H-imidazo[4,5-b]-pyridin-3-yl]methyl]biphenyl-2-carbonsäure-tert.butylester und Trifluoressigsäure.
Ausbeute: 95 % der Theorie,
Schmelzpunkt: 203-205 °C

| $C_{33}H_{38}N_4O_3$ (538,70) | | | |
|---|---|---|---|
| Ber.: | C 73,58 | H 7,11 | N 10,40 |
| Gef.: | 73,66 | 7,19 | 10,35 |

Beispiel 28

4'-[[2-n-Butyl-5-(N-acetyl-ethylamino)-3H-imidazo[4,5-b]pyridin-3-yl]methyl]biphenyl-2-carbonsäure

Hergestellt analog Beispiel 12 aus 4'-[[2-n-Butyl-5-(N-acetyl-ethylamino)-3H-imidazo[4,5-b]pyridin-3-yl]-methyl]biphenyl-2-carbonsäure-tert.butylester und Trifluoressigsäure.
Ausbeute: 80 % der Theorie,
Schmelzpunkt: 89-93 °C

| $C_{33}H_{38}N_4O_3$ (470,58) | | | |
|---|---|---|---|
| Ber.: | C 70,99 | H 6,47 | N 11,79 |
| Gef.: | 70,79 | 6,47 | 11,52 |

Beispiel 29

4'-[(2-n-Butyl-5-valeroylamino-3H-imidazo[4,5-b]pyridin-3-yl)methyl]biphenyl-2-carbonsäure

Hergestellt analog Beispiel 12 aus 4'-[(2-n-Butyl-5-valeroylamino-3H-imidazo[4,5-b]pyridin-3-yl)methyl]-biphenyl-2-car bonsäure-tert.butylester und Trifluoressigsäure.
Ausbeute: 56 % der Theorie,
Schmelzpunkt: 240-242 °C

| $C_{33}H_{38}N_4O_3$ (484,60) | | | |
|---|---|---|---|
| Ber.: | C 71,88 | H 6,66 | N 11,56 |
| Gef.: | 71,61 | 6,72 | 11,47 |

Beispiel 30

4'-[(2-n-Propyl-5-butanoylamino-3H-imidazo[4,5-b]pyridin-3-yl)methyl]biphenyl-2-carbonsäure

Hergestellt analog Beispiel 12 aus 4'-[(2-n-Propyl-5-butanoylamino-3H-imidazo[4,5-b]pyridin-3-yl)-methyl]biphenyl-2-carbonsäure-tert.butylester und Trifluoressigsäure.
Ausbeute: 68 % der Theorie,
Schmelzpunkt: 254-255 °C

| $C_{27}H_{28}N_4O_3$ (456,55) | | | |
|---|---|---|---|
| Ber.: | C 71,03 | H 6,18 | N 12,27 |
| Gef.: | 70,98 | 6,25 | 12,36 |

Beispiel 31

4'-[(2-n-Propyl-5-butanoylamino-3H-imidazo[4,5-b]pyridin-3-yl)methyl]biphenyl-4-brom-2-carbonsäure

Hergestellt analog Beispiel 12 aus 4'-[(2-n-Propyl-5-butanoylamino-3H-imidazo[4,5-b]pyridin-3-yl)-methyl]biphenyl-4-brom-2-carbonsäure-tert.butylester und Trifluoressigsäure.
Ausbeute: 83 % der Theorie,
Schmelzpunkt: 244-245 °C

| $C_{27}H_{27}N_4O_3Br$ (535,54) | | | | |
|---|---|---|---|---|
| Ber.: | C 60,56 | H 5,08 | N 10,46 | Br 14,92 |
| Gef.: | 60,42 | 5,07 | 10,41 | 14,82 |

Beispiel 32

4'-[[2-n-Propyl-5-(2-methyl-valeroylamino)-3H-imidazo[4,5-b]pyridin-3-yl]methyl]biphenyl-2-carbonsäure

Hergestellt analog Beispiel 12 aus 4'-[[2-n-Propyl-5-(2-methyl-valeroylamino)-3H-imidazo[4,5-b]pyridin-3-yl]methyl]biphenyl-2-carbonsäure-tert.butylester und Trifluoressigsäure.
Ausbeute: 97 % der Theorie,
Schmelzpunkt: 244-245 °C

| $C_{29}H_{32}N_4O_3$ (484,61) | | | |
|---|---|---|---|
| Ber.: | C 71,88 | H 6,66 | N 11,56 |
| Gef.: | 71,77 | 6,79 | 11,51 |

Beispiel 33

4'-[(2-n-Propyl-5-benzylcarbonylamino-3H-imidazo[4,5-b]pyridin-3-yl)methyl]biphenyl-2-carbonsäure

Hergestellt analog Beispiel 12 aus 4'-[(2-n-Propyl-5-benzylcarbonylamino-3H-imidazo[4,5-b]pyridin-3-yl)-methyl]biphenyl-2-carbonsäure-tert.butylester und Trifluoressigsäure.
Ausbeute: 93 % der Theorie,
Schmelzpunkt: 252-254 °C

| $C_{31}H_{28}N_4O_3$ (504,60) | | | |
|---|---|---|---|
| Ber.: | C 73,79 | H 5,59 | N 11,10 |
| Gef.: | 73,85 | 5,78 | 10,93 |

Beispiel 34

4'-[(2-n-Propyl-5-butanoylamino-1H-imidazo[4,5-b]pyridin-1-yl)methyl]biphenyl-2-carbonsäure

Hergestellt analog Beispiel 12 aus 4'-[(2-n-Propyl-5-butanoylamino-1H-imidazo[4,5-b]pyridin-1-yl)-methyl]biphenyl-2-carbonsäure-tert.butylester und Trifluoressigsäure.
Ausbeute: 88 % der Theorie,
Schmelzpunkt: 120 °C

| $C_{27}H_{28}N_4O_3$ (456,55) | | | |
|---|---|---|---|
| Ber.: | C 70,34 | H 6,23 | N 12,15 |
| Gef.: | 70,14 | 6,24 | 12,34 |

Beispiel 35

4'-[(2-n-Butyl-5-(N-ethoxycarbonyl-ethylamino)-3H-imidazo[4,5-b]pyridin-3-yl]methyl]biphenyl-2-carbonsäure

Hergestellt analog Beispiel 12 aus 4'-[[2-n-Butyl-5-(N-ethoxycarbonyl-ethylamino)-3H-imidazo[4,5-b]-pyridin-3-yl]methyl]biphenyl-2-carbonsäure-tert.butylester und Trifluoressigsäure.
Ausbeute: 95 % der Theorie,
Schmelzpunkt: 182-185 °C

| $C_{29}H_{32}N_4O_4$ (500,61) | | | |
|---|---|---|---|
| Ber.: | C 69,58 | H 6,44 | N 11,19 |
| Gef.: | 69,72 | 6,57 | 11,13 |

Beispiel 36

4'-[[2-n-Butyl-5-(N-cyclohexylaminocarbonyl-ethylamino)-3H-imidazo[4,5-b]pyridin-3-yl]methyl]biphenyl-2-carbonsäure

Hergestellt analog Beispiel 12 aus 4'-[(2-n-Butyl-5-(N-cyclohexylaminocarbonyl-ethylamino)-3H-imidazo-[4,5-b]pyridin-3-yl)methyl]biphenyl-2-carbonsäure-tert.butylester und Trifluoressigsäure.
Ausbeute: 95 % der Theorie,
Schmelzpunkt: 182-185 °C

| $C_{33}H_{39}N_5O_3$ (500,61) | | | |
|---|---|---|---|
| Ber.: | C 71,58 | H 7,10 | N 12,65 |
| Gef.: | 71,77 | 7,22 | 12,59 |

Beispiel 37

4'-[[2-n-Butyl-5-(N-ethoxycarbonyl-n-butylamino)-3H-imidazo[4,5-b]pyridin-3-yl]methyl]biphenyl-2-carbonsäure

Hergestellt analog Beispiel 12 aus 4'-[[2-n-Butyl-5-(N-ethoxycarbonyl-n-butylamino)-3H-imidazo[4,5-b]-pyridin-3-yl]methyl]biphenyl-2-carbonsäure-tert.butylester und Trifluoressigsäure.
Ausbeute: 37 % der Theorie,
Schmelzpunkt: 154-156 °C

| $C_{31}H_{36}N_4O_4$ (528,66) | | | |
|---|---|---|---|
| Ber.: | C 70,43 | H 6,86 | N 10,60 |
| Gef.: | 70,68 | 7,10 | 10,50 |

Beispiel 38

4'-[[2-n-Butyl-5-(N-cyclohexylaminocarbonyl-n-butylamino)-3H-imidazo[4,5-b]pyridin-3-yl]methyl]biphenyl-2-carbonsäure

Hergestellt analog Beispiel 12 aus 4'-[[2-n-Butyl-5-(N-cyclohexylaminocarbonyl-n-butylamino)-3H-imidazo[4,5-b]pyridin-3-yl]methyl]biphenyl-2-carbonsäure-tert.butylester und Trifluoressigsäure.
Ausbeute: 89 % der Theorie,
Schmelzpunkt: 195-198 °C

| $C_{35}H_{43}N_5O_3$ (581,47) | | | |
|---|---|---|---|
| Ber.: | C 72,26 | H 7,45 | N 12,04 |
| Gef.: | 72,29 | 7,66 | 11,81 |

Beispiel 39

4'-[[2-n-Butyl-5-(n-butylaminocarbonylamino)-1H-imidazo[4,5-b]pyridin-1-yl]methyl]biphenyl-2-carbonsäure

Hergestellt analog Beispiel 12 aus 4'-[[2-n-Butyl-5-(n-butylaminocarbonylamino)-1H-imidazo[4,5-b]-pyridin-1-yl]methyl] biphenyl-2-carbonsäure-tert.butylester und Trifluoressigsäure.
Ausbeute: 83 % der Theorie,
Schmelzpunkt: 290-295 °C

| $C_{29}H_{33}N_5O_3$ (499,62) | | | |
|---|---|---|---|
| Ber.: | C 69,72 | H 6,66 | N 14,02 |
| Gef.: | 69,62 | 6,76 | 13,98 |

Beispiel 40

4'-[[2-n-Butyl-5-(cis-hexahydrophthalimido)-3H-imidazo[4,5-b]pyridin-3-yl]methyl]biphenyl-2-carbonsäure

Hergestellt analog Beispiel 12 aus 4'-[[2-n-Butyl-5-(cis-hexahydrophthalimido)-3H-imidazo[4,5-b]pyridin-3-yl]methyl]biphenyl-2-carbonsäure-tert.butylester und Trifluoressigsäure.
Ausbeute: 84 % der Theorie,
Schmelzpunkt: 113-115 ° C

| $C_{32}H_{32}N_4O_4$ (536,64) | | | |
|---|---|---|---|
| Ber.: | C 62,76 | H 5,11 | N 8,61 |
| Gef.: | 62,79 | 5,21 | 8,48 |

Beispiel 41

4'-[(2-n-Butyl-5-methoxy-1H-imidazo[4,5-b]pyridin-1-yl)methyl]biphenyl-2-carbonsäure-trifluoracetat-hydrat

Hergestellt analog Beispiel 12 aus 4'-[(2-n-Butyl-5-methoxy-1H-imidazo[4,5-b]pyridin-1-yl)methyl]-biphenyl-2-carbonsäure-tert.butylester und Trifluoressigsäure.
Ausbeute: 80 % der Theorie,
Schmelzpunkt: 126-128 ° C

| $C_{25}H_{25}N_3O_3$ x $CF_3COOH$ x $H_2O$ (547,54) | | | |
|---|---|---|---|
| Ber.: | C 59,23 | H 5,15 | N 7,68 |
| Gef.: | 59,46 | 4,99 | 7,63 |

Beispiel 42

4'-[(2-n-Butyl-6-brom-1H-imidazo[4,5-b]pyridin-1-yl)methyl]biphenyl-2-carbonsäure

Hergestellt analog Beispiel 12 aus 4'-[(2-n-Butyl-6-brom-1H-imidazo[4,5-b]pyridin-1-yl)methyl]biphenyl-2-carbonsäure-tert.butylester und Trifluoressigsäure.
Ausbeute: 79 % der Theorie,
Schmelzpunkt: 239-240 ° C

| $C_{24}H_{22}BrN_3O_2$ (464,36) | | | | |
|---|---|---|---|---|
| Ber.: | C 62,08 | H 4,77 | N 9,05 | Br 17,21 |
| Gef.: | 61,83 | 4,71 | 8,92 | 17,43 |

Beispiel 43

4'-[(2-n-Butyl-6-brom-3H-imidazo[4,5-b]pyridin-3-yl)methyl]biphenyl-2-carbonsäure

Hergestellt analog Beispiel 12 aus 4'-[(2-n-Butyl-6-brom-3H-imidazo[4,5-b]pyridin-3-yl)methyl]biphenyl-2-carbonsäure-tert.butylester und Trifluoressigsäure.
Ausbeute: 85 % der Theorie,
Schmelzpunkt: 221-223 ° C

| $C_{24}H_{22}BrN_3O_2$ (464,36) | | | | |
|---|---|---|---|---|
| Ber.: | C 62,08 | H 4,77 | N 9,05 | Br 17,21 |
| Gef.: | 61,95 | 4,84 | 8,96 | 17,38 |

Beispiel 44

4'-[(2-n-Butyl-5-methyl-6-phthalimido-3H-imidazo[4,5-b]pyridin-3-yl)methyl]biphenyl-2-carbonsäure

Hergestellt analog Beispiel 12 aus 4'-[(2-n-Butyl-5-methyl-6-phthalimido-3H-imidazo[4,5-b]pyridin-3-yl)-methyl]biphenyl-2-carbonsäure-tert.butylester und Trifluoressigsäure.
Ausbeute: 75 % der Theorie,
Schmelzpunkt: 336-340 °C

| $C_{33}H_{28}N_4O_4$ (544,62) | | | |
|---|---|---|---|
| Ber.: | C 72,78 | H 5,18 | N 10,29 |
| Gef.: | 72,59 | 5,18 | 10,26 |

Beispiel 45

4'-[(2-n-Butyl-5-methyl-6-phthalimido-1H-imidazo[4,5-b]pyridin-1-yl)methyl]-biphenyl-2-carbonsäure

Hergestellt analog Beispiel 12 aus 4'-[(2-n-Butyl-5-methyl-6-phthalimido-1H-imidazo[4,5-b]pyridin-1-yl)-methyl]biphenyl-2-carbonsäure-tert.butylester und Trifluoressigsäure.
Ausbeute: 63 % der Theorie,
Schmelzpunkt: 301-303 °C

| $C_{33}H_{28}N_4O_4$ (544,62) | | | |
|---|---|---|---|
| Ber.: | C 72,78 | H 5,18 | N 10,29 |
| Gef.: | 72,71 | 5,25 | 10,18 |

Beispiel 46

4'-[(8-n-Butyl-2-methoxy-9H-purin-9-yl)-methyl]biphenyl-2-carbonsäure

Hergestellt analog Beispiel 12 aus 4'-[(8-n-Butyl-2-methoxy-9H-purin-9-yl)-methyl]biphenyl-2-carbonsäure-tert.butylester und Trifluoressigsäure.
Ausbeute: 73 % der Theorie,
Schmelzpunkt: 146-148 °C

| $C_{24}H_{24}N_4O_3$ (416,48) | | | |
|---|---|---|---|
| Ber.: | C 69,21 | H 5,81 | N 13,45 |
| Gef.: | 68,97 | 5,84 | 13,17 |

Beispiel 47

4'-[(8-n-Butyl-2-methoxy-7H-purin-7-yl)-methyl]biphenyl-2-carbonsäure

Hergestellt analog Beispiel 12 aus 4'-[(8-n-Butyl-2-methoxy-7H-purin-7-yl)-methyl]biphenyl-2-carbonsäure-tert.butylester und Trifluoressigsäure.
Ausbeute: 73 % der Theorie,
Schmelzpunkt: 146-148 °C

| $C_{24}H_{24}N_4O_3$ (416,48) | | | |
|---|---|---|---|
| Ber.: | C 69,21 | H 5,81 | N 13,45 |
| Gef.: | 69,07 | 5,94 | 13,27 |

Beispiel 48

4'-[(2-Benzylamino-8-n-butyl-9H-purin-9-yl)-methyl]biphenyl-2-carbonsäure

Hergestellt analog Beispiel 12 aus 4'-[(2-Benzylamino-8-n-butyl-9H-purin-9-yl)-methyl]biphenyl-2-carbonsäure-tert.butylester und Trifluoressigsäure.
Ausbeute: 13 % der Theorie,
Schmelzpunkt: 232-234 ° C

| $C_{30}H_{29}N_5O_2$ (491,60) | | | |
|---|---|---|---|
| Ber.: | C 73,20 | H 5,95 | N 14,25 |
| Gef.: | 73,16 | 6,05 | 14,44 |

Beispiel 49

4'-[(2-n-Butyl-5-methyl-6-(N-acetyl-n-butylamino)-3H-imidazo[4,5-b]pyridin-3-yl)-methyl]biphenyl-2-carbonsäure

Hergestellt analog Beispiel 12 aus 4'-[(2-n-Butyl-5-methyl-6-(N-acetyl-n-butylamino)-3H-imidazo[4,5-b]-pyridin-3-yl)-methyl]biphenyl-2-carbonsäure-tert.butylester und Trifluoressigsäure.
Ausbeute: 56 % der Theorie,
Schmelzpunkt: 144-146 ° C

| $C_{31}H_{36}N_4O_3$ (512,65) | | | |
|---|---|---|---|
| Ber.: | C 72,63 | H 7,08 | N 10,93 |
| Gef.. | 72,39 | 7,15 | 10,79 |

Beispiel 50

4'-[(2-n-Butyl-5-methyl-6-amino-3H-imidazo[4,5-b]pyridin-3-yl)-methyl]biphenyl-2-carbonsäure

Hergestellt analog Beispiel 12 aus 4'-[(2-n-Butyl-5-methyl-6-amino-3H-imidazo[4,5-b]pyridin-3-yl)-methyl]biphenyl-2-carbonsäure-tert.butylester und Trifluoressigsäure.
Ausbeute: 80 % der Theorie,
Schmelzpunkt: 168-170 ° C

| $C_{26}H_{26}N_4O_2$ (414,57) | | | |
|---|---|---|---|
| Ber.: | C 72,44 | H 6,32 | N 13,52 |
| Gef.. | 72,40 | 6,50 | 13,32 |

Beispiel 51

4'-[(2-n-Butyl-6-hexahydrophthalimido-3H-imidazo[4,5-b]pyridin-3-yl)-methyl]biphenyl-2-carbonsäure

Hergestellt analog Beispiel 12 aus 4'-[(2-n-Butyl-6-hexahydrophthalimido-3H-imidazo[4,5-b]pyridin-3-yl)-methyl]-bi phenyl-2-carbonsäure-tert.butylester und Trifluoressigsäure.
Ausbeute: 90 % der Theorie,
Schmelzpunkt: 179-181 °C

| $C_{32}H_{32}N_4O_4$ (536,64) | | | |
|---|---|---|---|
| Ber.: | C 71,62 | H 6,01 | N 10,44 |
| Gef.: | 71,87 | 6,00 | 10,36 |

Beispiel 52

4'-[(2-n-Butyl-5-methyl-6-butyrylamino-3H-imidazo[4,5-b]pyridin-3-yl)-methyl]biphenyl-2-carbonsäure

Hergestellt analog Beispiel 12 aus 4'-[(2-n-Butyl-5-methyl-6-butyrylamino-3H-imidazo[4,5-b]pyridin-3-yl)-methyl]biphenyl-2-carbonsäure-tert.butylester und Trifluoressigsäure.

Beispiel 53

4'-[[2-n-Butyl-5-methyl-6-(2-oxo-piperidin-1-yl)-1H-imidazo[4,5-b]pyridin-1-yl)methyl]biphenyl-2-carbonsäure-trifluoracetat

Hergestellt analog Beispiel 12 aus 4'-[[2-n-Butyl-5-methyl-6-(2-oxo-piperidin-1-yl)-1H-imidazo[4,5-b]-pyridin-1-yl)methyl]biphenyl-2-carbonsäure-tert.butylester und Trifluoressigsäure.
Ausbeute: 91 % der Theorie,
Schmelzpunkt: 171-173 °C

| $C_{30}H_{32}N_4O_3$ x $CF_3COOH$ (610,65) | | | |
|---|---|---|---|
| Ber.: | C 62,94 | H 5,45 | N 9,18 |
| Gef.: | 62,74 | 5,49 | 8,98 |

Beispiel 54

4'-[[2-n-Butyl-5-methyl-6-(2-methyl-propionylamino)-3H-imidazo[4,5-b]pyridin-3-yl]methyl]biphenyl-2-carbonsäure

Hergestellt analog Beispiel 12 aus 4'-[[2-n-Butyl-5-methyl-6-(2-methyl-propionylamino)-3H-imidazo[4,5-b]pyridin-3-yl]methyl]biphenyl-2-carbonsäure-tert.butylester und Trifluoressigsäure.

Beispiel 55

4'-[[2-n-Butyl-5-methyl-6-(N_cyclohexylcarbonyl-ethylamino)-3H-imidazo[4,5-b]pyridin-3-yl)methyl]biphenyl-2-carbonsäure

Hergestellt analog Beispiel 12 aus 4'-[[2-n-Butyl-5-methyl-6-(N-cyclohexylcarbonyl-ethylamino)-3H-imidazo[4,5-b]pyridin-3-yl)-methyl]biphenyl-2-carbonsäure-tert.butylester und Trifluoressigsäure.
Ausbeute: 78 % der Theorie,
Schmelzpunkt: 164-166 °C

| $C_{34}H_{40}N_4O_3$ (552,72) | | | |
|---|---|---|---|
| Ber.: | C 73,88 | H 7,29 | N 10,14 |
| Gef.: | 73,58 | 7,29 | 10,04 |

Beispiel 56

4'-[[2-n-Butyl-5-methyl-6-(cis-hexahydrophthalimido)-3H-imidazo[4,5-b]pyridin-3-yl]methyl]biphenyl-2-carbonsäure

Hergestellt analog Beispiel 12 aus 4'-[[2-n-Butyl-5-methyl-6-(cis-hexahydrophthalimido)-3H-imidazo[4,5-b]pyridin-3-yl]methyl]biphenyl-2-carbonsäure-tert.butylester und Trifluoressigsäure.
Ausbeute: 81 % der Theorie,
Schmelzpunkt: 261-263 ° C

| $C_{33}H_{34}N_4O_4$ (550,67) | | | |
|---|---|---|---|
| Ber.:<br>Gef.: | C 71,98<br>71,78 | H 6,22<br>6, 25 | N 10,18<br>9,95 |

Beispiel 57

4'-[(2-n-Butyl-6-(cis-hexahydrophthalimido)-1H-imidazo[4,5-b]pyridin-1-yl)methyl]biphenyl-2-carbonsäure-dihydrat

Hergestellt analog Beispiel 12 aus 4'-[(2-n-Butyl-6-(cis-hexahydrophthalimido)-1H-imidazo[4,5-b]pyridin-1-yl)methyl]biphenyl-2-carbonsäure-tert.butylester und Trifluoressigsäure.
Ausbeute: 70 % der Theorie,
Schmelzpunkt: 203-205 ° C

| $C_{32}H_{32}N_4O_4$ x 2 $H_2O$ (572,68) | | | |
|---|---|---|---|
| Ber.:<br>Gef.: | C 67,11<br>67,25 | H 6,33<br>6,30 | N 9,78<br>9,78 |

Beispiel 58

4'-[[2-n-Butyl-5-methyl-6-(3-benzyl-3,4,5,6-tetrahydro-2(1H)-pyrimidinon-1-yl)-3H-imidazo[4,5-b]pyridin-3-yl]-methyl]biphenyl-2-carbonsäure-trifluoracetat

Hergestellt analog Beispiel 12 aus 4'-[[2-n-Butyl-5-methyl-6-(3-benzyl-3,4,5,6-tetrahydro-2(1H)-pyrimidinon-1-yl)-3H-imidazo[4,5-b]pyridin-3-yl]methyl]biphenyl-2-carbonsäure-tert.butylester und Trifluoressigsäure.
Ausbeute: 77 % der Theorie,
Schmelzpunkt: 168-170 ° C

| $C_{36}H_{37}N_5O_3$ x $CF_3COOH$ (701,76) | | | |
|---|---|---|---|
| Ber.:<br>Gef.: | C 65,04<br>64,98 | H 5,46<br>5,67 | N 9,98<br>9,91 |

Beispiel 59

4'-[[2-n-Butyl-5-[3-(4-methoxy)benzyl-3,4,5,6-tetrahydro-2(1H)-pyrimidinon-1-yl]-3H-imidazo[4,5-b]pyridin-3-yl]methyl]biphenyl-2-carbonsäure

Hergestellt analog Beispiel 12 aus 4'-[[2-n-Butyl-5-[3-(4-methoxy)benzyl-3,4,5,6-tetrahydro-2(1H)-pyrimidinon-1-yl]-3H-imidazo[4,5-b]pyridin-3-yl]methyl]biphenyl-2-carbonsäure-tert.butylester und Trifluoressigsäure.

Beispiel 60

4'-[[2-n-Butyl-5-[3-(4-hydroxy)benzyl-3,4,5,6-tetrahydro-2(1H)-pyrimidinon-1-yl]-3H-imidazo[4,5-b]pyridin-3-yl]methyl]biphenyl-2-carbonsäure

Hergestellt analog Beispiel 12 aus 4'-[[2-n-Butyl-5-[3-(4-hydroxy)-benzyl-3,4,5,6-tetrahydro-2(1H)-pyrimidinon-1-yl]-3H-imidazo[4,5-b]pyridin-3-yl]methyl]biphenyl-2-carbonsäure-tert.butylester und Trifluoressigsäure.

Beispiel 61

4'-[[(2-n-Butyl-5-(3-cyclohexylmethyl-3,4,5,6-tetrahydro-2(1H)-pyrimidinon-1-yl)-3H-imidazo[4,5-b]pyridin-3-yl]methyl]biphenyl-2-carbonsäure

Hergestellt analog Beispiel 12 aus 4'-[[2-n-Butyl-5-(3-cyclohexylmethyl-3,4,5,6-tetrahydro-2(1H)-pyrimidinon-1-yl)-3H-imidazo[4,5-b]pyridin-3-yl]methyl]biphenyl-2-carbonsäure-tert.butylester und Trifluoressigsäure.

Beispiel 62

4'-[[2-n-Propyl-5-[(2-carboxymethyl)pyrrolidino]-3H-imidazo[4,5-b]pyridin-3-yl]methyl]biphenyl-2-carbonsäure

Hergestellt analog Beispiel 12 aus 4'-[[2-n-Propyl-5-[(2-carboxymethyl)pyrrolidino]-3H-imidazo[4,5-b]-pyridin-3-yl]methyl]biphenyl-2-carbonsäure-tert.butylester und Trifluoressigsäure.
Ausbeute: 37 % der Theorie,
Schmelzpunkt: 137-139 °C

| $C_{29}H_{30}N_4O_4$ (498,62) | | | |
|---|---|---|---|
| Ber.:<br>Gef.: | C 69,86<br>69,59 | H 6,06<br>6,20 | N 11,24<br>11,04 |

Beispiel 63

4'-[(2-n-Butyl-6-phthalimido-1H-imidazo[4,5-b]pyridin-1-yl)methyl]biphenyl-2-carbonsäure-trifluoracetat

Hergestellt analog Beispiel 12 aus 4'-[(2-n-Butyl-6-phthalimido-1H-imidazo[4,5-b]pyridin-1-yl)methyl]-biphenyl-2-carbonsäure-tert.butylester und Trifluoressigsäure.
Ausbeute: 74 % der Theorie,
Schmelzpunkt: 168-170 °C

| $C_{32}H_{26}N_4O_4$ x $CF_3COOH$ (644,61) | | | |
|---|---|---|---|
| Ber.:<br>Gef.: | C 63,35<br>63,50 | H 4,22<br>4,53 | N 8,96<br>9,06 |

Beispiel 64

4'-[[8-n-Butyl-2-(n-butylamino)-9H-purin-9-yl]methyl]biphenyl-2-carbonsäure

0,45 g (0,95 mMol) 4'-[[8-n-Butyl-2-(n-butylamino)-9H-purin-9-yl]methyl]biphenyl-2-carbonsäuremethylester werden in 20 ml Methanol und 10 ml Wasser gelöst, mit 0,4 g gepulvertem Kaliumhydroxid versetzt und 3 Stunden zum Rückfluß erhitzt. Anschließend wird das Reaktionsgemisch eingedampft und der Rückstand in 30 ml Wasser gelöst. Man filtriert über Kohle und säuert mit Eisessig an. Der gebildete Niederschlag wird abgesaugt, mit Wasser gewaschen und getrocknet.

Ausbeute: 0,4 g (92 % der Theorie),
Schmelzpunkt: 213-215 °C

| $C_{27}H_{31}N_5O_2$ (457,58) | | | |
|---|---|---|---|
| Ber.: | C 70,87 | H 6,83 | N 15,31 |
| Gef.: | 70,70 | 6,89 | 15,19 |

Beispiel 65

4'-[(8-n-Butyl-2-cyclohexylamino-9H-purin-9-yl)methyl]biphenyl-2-carbonsäure

Hergestellt analog Beispiel 64 aus 4'-[(8-n-Butyl-2-cyclohexylamino-9H-purin-9-yl)methyl]biphenyl-2-carbonsäuremethylester und methanolischer Kalilauge.
Ausbeute: 55 % der Theorie,
Schmelzpunkt: 213-215 °C

| $C_{29}H_{33}N_5O_2$ (483,62) | | | |
|---|---|---|---|
| Ber.: | C 72,02 | H 6,88 | N 14,48 |
| Gef.: | 71,84 | 6,98 | 14,62 |

Beispiel 66

4'-[(8-n-Butyl-2-ethoxy-9H-purin-9-yl)methyl]biphenyl-2-carbonsäure

Hergestellt analog Beispiel 64 aus 4'-[(8-n-Butyl-2-ethoxy-9H-purin-9-yl)methyl]biphenyl-2-carbonsäuremethylester und methanolischer Kalilauge.
Ausbeute: 48 % der Theorie,
Schmelzpunkt: 190-192 °C

| $C_{25}H_{26}N_4O_3$ (430,51) | | | |
|---|---|---|---|
| Ber.: | C 69,75 | H 6,09 | N 13,01 |
| Gef.: | 69,75 | 6,13 | 12,83 |

Beispiel 67

4'-[(8-n-Butyl-2-ethoxy-7H-purin-7-yl)methyl]biphenyl-2-carbonsäure

Hergestellt analog Beispiel 64 aus 4'-[(8-n-Butyl-2-ethoxy-7H-purin-7-yl)methyl]biphenyl-2-carbonsäuremethylester und methanolischer Kalilauge.
Ausbeute: 10 % der Theorie,
Schmelzpunkt: 155-158 °C

| $C_{25}H_{26}N_4O_3$ (430,51) | | | |
|---|---|---|---|
| Ber.: | C 69,75 | H 6,09 | N 13,01 |
| Gef.: | 69,95 | 6,10 | 11,83 |

Beispiel 68

4'-[[2-n-Propyl-5-(2-methylpropylamino)-3H-imidazo[4,5-b]pyridin-3-yl]methyl]-2-(1H-tetrazol-5-yl)biphenyl

0,32 g (0,42 mMol) 4'-[[2-n-Propyl-5-(2-methylpropylamino)-3H-imidazo[4,5-b]pyridin-3-yl)methyl]-2-(1-triphenylmethyl-tetrazol-5-yl)-biphenyl werden in 20 ml methanolischer Salzsäure gelöst und 2 Stunden bei Raumtemperatur gerührt. Das Solvens wird eingedampft, der Rückstand mit 20 ml Wasser verreiben, abgesaugt und getrocknet. Nach Säulenchromatographie an Kieselgel (Korngröße: 0,06-0,2 mm, Elutionsmittel: Methylenchlorid/Ethanol 0-10 %) erhält man einen weißen Feststoff.
Ausbeute: 0,1 g (51 % der Theorie),
Schmelzpunkt: 128-130 °C

| $C_{27}H_{30}N_8$ (466,60) | | | |
|---|---|---|---|
| Ber.: | C 69,50 | H 6,48 | N 24,02 |
| Gef.: | 69,44 | 6,73 | 24,04 |

Beispiel 69

4'-[[2-n-Butyl-5-(2-aminocarbonyl-cyclohexylcarbonylamino)-3H-imidazo[4,5-b]pyridin-3-yl]methyl]-2-(1H-tetrazol-5-yl)biphenyl

Hergestellt analog Beispiel 68 aus 4'-[[2-n-Butyl-5-(2-aminocarbonyl-cyclohexylcarbonylamino)-3H-imidazo[4,5-b]pyridin-3-yl)methyl]-2-(1-triphenylmethyl-tetrazol-5-yl)biphenyl und methanolischer Salzsäure.
Ausbeute: 37 % der Theorie,
Schmelzpunkt: 165-175 °C

| $C_{32}H_{35}N_9O_2$ (577,70) | | | |
|---|---|---|---|
| Ber.: | C 66,53 | H 6,11 | N 21,82 |
| Gef.: | 65,73 | 6,13 | 21,84 |

Beispiel 70

4'-[[2-n-Butyl-5-(cis-hexahydrophthalimido)-3H-imidazo[4,5-b]pyridin-3-yl]methyl]-2-(1H-tetrazol-5-yl)-biphenyl

Hergestellt analog Beispiel 68 aus 4'-[[2-n-Butyl-5-(cis-hexahydrophthalimido)-3H-imidazo[4,5-b]pyridin-3-yl]methyl]-2-(1-triphenylmethyl-tetrazol-5-yl)biphenyl und Eisessig. Ausbeute: 30 % der Theorie,
Schmelzpunkt: 127 °C
$C_{32}H_{32}N_8O_2$ (560,66)
Massenspektrum: $M^+$ = 560

Beispiel 71

4'-[[2-n-Butyl-5-(4-methyl-piperidino)-3H-imidazo[4,5-b]pyridin-3-yl]methyl]-2-(1H-tetrazol-5-yl)-biphenyl

Hergestellt analog Beispiel 68 aus 4'-[[2-n-Butyl-5-(4-methyl-piperidino)-3H-imidazo[4,5-b]pyridin-3-yl]-methyl]-2-(1-triphenylmethyl-tetrazol-5-yl)-biphenyl und methanolischer Salzsäure.
Ausbeute: 39 % der Theorie,
Schmelzpunkt: 187-189 °C
$C_{30}H_{34}N_8$ (506,70)
Massenspektrum: $M^+$ = 506

Beispiel 72

4'-[[2-n-Propyl-5-[N-(3-phenylpropionyl)isobutylamino]-3H-imidazo[4,5-b]pyridin-3-yl]methyl]-2-(1H-tetrazol-

5-yl)-biphenyl

Hergestellt analog Beispiel 68 aus 4'-[[2-n-Propyl-5-[N-(3-phenylpropionyl)-isobutylamino]-3H-imidazo-[4,5-b]pyridin-3-yl]methyl]-2-(1-triphenylmethyl-tetrazol-5-yl)-biphenyl und methanolischer Salzsäure.

Beispiel 73

4'-[[2-n-Propyl-5-(N-benzylaminocarbonyl-isobutylamino)-3H-imidazo[4,5-b]pyridin-3-yl]methyl]-2-(1H-tetrazol-5-yl)-biphenyl

Hergestellt analog Beispiel 68 aus 4'-[[2-n-Propyl-5-(N-benzylaminocarbonyl-isobutylamino)-3H-imidazo-[4,5-b]pyridin-3-yl]methyl]-2-(1-triphenylmethyl-tetrazol-5-yl)-biphenyl und methanolischer Salzsäure.
Ausbeute: 37 % der Theorie,
Schmelzpunkt: 195-196 ° C

| $C_{35}H_{37}N_9O$ (599,75) | | | |
|---|---|---|---|
| Ber.: | C 70,09 | H 6,22 | N 21,02 |
| Gef.: | 69,95 | 6,32 | 20,86 |

Beispiel 74

4'-[[2-n-Propyl-5-(N-cyclohexylaminocarbonyl-isobutylamino)-3H-imidazo[4,5-b]pyridin-3-yl]methyl]-2-(1H-tetrazol-5-yl)-biphenyl

Hergestellt analog Beispiel 68 aus 4'-[[2-n-Propyl-5-(N-cyclohexylaminocarbonyl-isobutylamino)-3H-imidazo[4,5-b]pyridin-3-yl]methyl]-2-(1-triphenylmethyl-tetrazol-5-yl)-biphenyl und methanolischer Salzsäure.
Ausbeute: 38 % der Theorie,
Schmelzpunkt: 112-113 ° C

| $C_{34}H_{41}N_9O$ (591,77) | | | |
|---|---|---|---|
| Ber.: | C 69,01 | H 6,98 | N 21,30 |
| Gef.: | 68,86 | 6,88 | 21,18 |

Beispiel 75

4'-[[2-n-Butyl-5-(3-benzyl-3,4,5,6-tetrahydro-2(1H)-pyrimidinon-1-yl)-3H-imidazo[4,5-b]pyridin-3-yl]methyl]-2-(1H-tetrazol-5-yl)-biphenyl

Hergestellt analog Beispiel 68 aus 4'-[[2-n-Butyl-5-(3-benzyl-3,4,5,6-tetrahydro-2(1H)-pyrimidinon-1-yl)-3H-imidazo[4,5-b]pyridin-3-yl]methyl]-2-(1-triphenylmethyl-tetrazol-5-yl)-biphenyl und methanolischer Salzsäure.

Beispiel 76

4'-[(2-n-Butyl-5-methyl-3H-imidazo[4,5-b]pyridin-3-yl)methyl]biphenyl-2-carbonsäure

Hergestellt analog Beispiel 12 aus 4'-[(2-n-Butyl-5-methyl-3H-imidazo[4,5-b]pyridin-3-yl)methyl]biphenyl-2-carbonsäure-tert.butylester und Trifluoressigsäure.
Ausbeute: 65 % der Theorie,
Schmelzpunkt: 233-235 ° C

| $C_{25}H_{25}N_3O_2$ (399,50) | | | |
|---|---|---|---|
| Ber.: | C 75,16 | H 6,31 | N 10,52 |
| Gef.: | 75,06 | 6,35 | 10,46 |

Beispiel 77

4'-[[2-n-Butyl-5-(2-oxo-pyrrolidino)-3H-imidazo[4,5-b]pyridin-3-yl]methyl]-2-(1H-tetrazol-5-yl)-biphenyl

Hergestellt analog Beispiel 68 aus 4'-[[2-n-Butyl-5-(2-oxo-pyrrolidino)-3H-imidazo[4,5-b]pyridin-3-yl]-methyl]-2-(1-triphenylmethyl-tetrazol-5-yl)-biphenyl und methanolischer Salzsäure.

Beispiel 78

4'-[[2-n-Propyl-5-(2-oxo-pyrrolidino)-3H-imidazo[4,5-b]pyridin-3-yl]methyl]-2-(1H-tetrazol-5-yl)-biphenyl

Hergestellt analog Beispiel 68 aus 4'-[[2-n-Propyl-5-(2-oxo-pyrrolidino)-3H-imidazo[4,5-b]pyridin-3-yl]-methyl]-2-(1-triphenylmethyl-tetrazol-5-yl)-biphenyl und methanolischer Salzsäure.

Beispiel 79

4'-[[2-n-Butyl-5-(2-oxo-piperidino)-3H-imidazo[4,5-b]pyridin-3-yl]methyl]-2-(1H-tetrazol-5-yl)-biphenyl

Hergestellt analog Beispiel 68 aus 4'-[[2-n-Butyl-5-(2-oxo-piperidino)-3H-imidazo[4,5-b]pyridin-3-yl]-methyl]-2-(1-triphenyl-methyl-tetrazol-5-yl)-biphenyl und methanolischer Salzsäure.

Beispiel 80

4'-[[2-n-Propyl-5-(2-oxo-piperidino)-3H-imidazo[4,5-b]pyridin-3-yl]methyl]-2-(1H-tetrazol-5-yl)-biphenyl

Hergestellt analog Beispiel 68 aus 4'-[[2-n-Propyl-5-(2-oxo-piperidino)-3H-imidazo[4,5-b]pyridin-3-yl]-methyl]-2-(1-triphenyl-methyl-tetrazol-5-yl)-biphenyl und methanolischer Salzsäure.

Beispiel 81

4'-[[2-n-Butyl-5-methyl-6-(cis-hexahydrophthalimido)-1H-imidazo[4,5-b]pyridin-1-yl]methyl]biphenyl-2-carbonsäure-trifluoracetat

Hergestellt analog Beispiel 12 aus 4'-[[2-n-Butyl-5-methyl-6-(cis-hexahydrophthalimido)-1H-imidazo[4,5-b]pyridin-1-yl]methyl]biphenyl-2-carbonsäure-tert.butylester und Trifluoressigsäure.
Ausbeute: 79 % der Theorie,
Schmelzpunkt: 188-190 °C

| $C_{33}H_{34}N_4O_4$ x $CF_3COOH$ (664,68) | | | |
|---|---|---|---|
| Ber.: | C 63,24 | H 5,31 | N 8,42 |
| Gef.: | 63,52 | 5,65 | 8,69 |

Beispiel 82

4'-[(2-n-Butyl-5-methyl-6-dimethylaminocarbonylamino-1H-imidazo[4,5-b]pyridin-1-yl)methyl]biphenyl-2-carbonsäure-trifluoracetat

Hergestellt analog Beispiel 12 aus 4'-[[2-n-Butyl-5-methyl-6-dimethylaminocarbonylamino-1H-imidazo-[4,5-b]pyridin-1-yl]methyl]biphenyl-2-carbonsäure-tert.butylester und Trifluoressigsäure.

Ausbeute: 83 % der Theorie,
Schmelzpunkt: 147-149° C

| $C_{28}H_{31}N_5O_3$ x $CF_3COOH$ (599,61) | | | |
|---|---|---|---|
| Ber.: | C 60,09 | H 5,37 | N 11,68 |
| Gef.: | 60,31 | 5,39 | 11,51 |

Beispiel 83

4'-[(2-n-Butyl-5-methyl-1H-imidazo[4,5-b]pyridin-1-yl)methyl]biphenyl-2-carbonsäure

Hergestellt analog Beispiel 12 aus 4'-[(2-n-Butyl-5-methyl-1H-imidazo[4,5-b]pyridin-1-yl)methyl]biphenyl-2-carbonsäure-tert.butylester und Trifluoressigsäure.
Ausbeute: 77 % der Theorie,
Schmelzpunkt: 230-232° C

| $C_{25}H_{25}N_3O_2$ (399,50) | | | |
|---|---|---|---|
| Ber.: | C 75,16 | H 6,31 | N 10,52 |
| Gef.: | 74,86 | 6,39 | 10,46 |

Beispiel 84

4'-[(2-n-Butyl-6-phthalimido-3H-imidazo[4,5-b]pyridin-3-yl)methyl]biphenyl-2-carbonsäure

Hergestellt analog Beispiel 12 aus 4'-[(2-n-Butyl-6-phthalimido-3H-imidazo[4,5-b]pyridin-3-yl)methyl]-biphenyl-2-carbon säure-tert.butylester und Trifluoressigsäure.
Ausbeute: 78 % der Theorie,
Schmelzpunkt: 271-272° C

| $C_{32}H_{26}N_4O_4$ (530,59) | | | |
|---|---|---|---|
| Ber.: | C 72,44 | H 4,94 | N 10,56 |
| Gef.: | 72,37 | 4,99 | 10,48 |

Beispiel 85

4'-[[2-n-Butyl-5-(4-chlorphenyl)sulfonylamino-3H-imidazo[4,5-b]pyridin-3-yl]-methyl]biphenyl-2-carbonsäure-hydrat

Hergestellt analog Beispiel 12 aus 4'-[[2-n-Butyl-5-(4-chlorphenyl)sulfonylamino-3H-imidazo[4,5-b]-pyridin-3-yl]methyl]biphenyl-2-carbonsäure-tert.butylester und Trifluoressigsäure.
Ausbeute: 63 % der Theorie,
Schmelzpunkt: 158-160° C

| $C_{30}H_{27}N_4O_4ClS$ x $H_2O$ (593,10) | | | |
|---|---|---|---|
| Ber.: | C 60,75 | H 4,93 | N 9,45 |
| Gef.: | 60,62 | 4,76 | 9,27 |

Beispiel 86

4'-[(2-n-Butyl-5-n-butylsulfonylamino-3H-imidazo[4,5-b]pyridin-3-yl)methyl]biphenyl-2-carbonsäure

Hergestellt analog Beispiel 4 aus 4'-[(2-n-Butyl-5-amino-3H-imidazo[4,5-b]pyridin-3-yl)methyl]biphenyl-2-carbonsäure und Butansulfonylchlorid in Pyridin.
Ausbeute: 16 % der Theorie,
Schmelzpunkt: > 250 ° C

| $C_{28}H_{32}N_4O_4S$ (520,70) | | | |
|---|---|---|---|
| Ber.: | C 65,59 | H 6,19 | N 10,76 |
| Gef.: | 65,41 | 6,28 | 10,58 |

Analog werden folgende Verbindungen erhalten:
4'-[(2-n-Butyl-5-n-propylsulfonylamino-3H-imidazo[4,5-b]pyridin-3-yl)methyl]biphenyl-2-carbonsäure
4'-[(2-n-Butyl-5-isopropylsulfonylamino-3H-imidazo[4,5-b]pyridin-3-yl)methyl]biphenyl-2-carbonsäure
4'-[[2-n-Butyl-5-(3-chlorpropylsulfonylamino)-3H-imidazo[4,5-b]pyridin-3-yl)methyl]biphenyl-2-carbonsäure
4'-[(2-n-Butyl-5-n-hexylsulfonylamino-3H-imidazo[4,5-b]pyridin-3-yl)methyl]biphenyl-2-carbonsäure
4'-[(2-n-Butyl-5-benzylsulfonylamino-3H-imidazo[4,5-b]pyridin-3-yl)methyl]biphenyl-2-carbonsäure

## Beispiel 87

4'-[[2-n-Butyl-5-(n-butansultam-1-yl)-3H-imidazo[4,5-b]pyridin-3-yl]-methyl]-2-(1H-tetrazol-5-yl)biphenyl

Hergestellt analog Beispiel 68 aus 4'-[[2-n-Butyl-5-(n-butansultam-1-yl)-3H-imidazo[4,5-b]pyridin-3-yl]-methyl]-2-(1-triphenylmethyl-tetrazol-5-yl)biphenyl und methanolischer Salzsäure.
Analog werden folgende Verbindungen erhalten:
4'-[[2-n-Propyl-5-(n-butansultam-1-yl)-3H-imidazo[4,5-b]pyridin-3-yl]-methyl]-2-(1H-tetrazol-5-yl)biphenyl
4'-[[2-n-Butyl-5-(n-propansultam-1-yl)-3H-imidazo[4,5-b]pyridin-3-yl]-methyl]-2-(1H-tetrazol-5-yl)biphenyl
4'-[[2-n-Propyl-5-(n-propansultam-1-yl)-3H-imidazo[4,5-b]pyridin-3-yl]-methyl]-2-(1H-tetrazol-5-yl)biphenyl
4'-[[2-n-Butyl-5-(n-butansultam-1-yl)-3H-imidazo[4,5-b]pyridin-3-yl]-methyl]biphenyl-2-carbonsäure
4'-[[2-n-Propyl-5-(n-butansultam-1-yl)-3H-imidazo[4,5-b]pyridin-3-yl]-methyl]biphenyl-2-carbonsäure
4'-[[2-n-Butyl-5-(n-propansultam-1-yl)-3H-imidazo[4,5-b]pyridin-3-yl]-methyl]biphenyl-2-carbonsäure
4'-[[2-n-Propyl-5-(n-propansultam-1-yl)-3H-imidazo[4,5-b]pyridin-3-yl]-methyl]biphenyl-2-carbonsäure

## Beispiel 88

4'-[(2-n-Butyl-5-methoxy-3H-imidazo[4,5-b]pyridin-3-yl)methyl]-2-(1H-tetrazol-5-yl)biphenyl

Hergestellt analog Beispiel 68 aus 4'-[(2-n-Butyl-5-methoxy-3H-imidazo[4,5-b]pyridin-3-yl)methyl]-2-(1-triphenylmethyl-tetrazol-5-yl)biphenyl und methanolischer Salzsäure.
Ausbeute: 43 % der Theorie,
Schmelzpunkt: 206-207 ° C

| $C_{25}H_{25}N_7O$ (439,52) | | | |
|---|---|---|---|
| Ber.: | C 68,32 | H 5,73 | N 22,31 |
| Gef.: | 68,11 | 5,88 | 22,19 |

## Beispiel 89

4'-[(2-n-Butyl-3H-imidazo[4,5-b]pyridin-5-on-3-yl)methyl]-2-(1H-tetrazol-5-yl)biphenyl

Hergestellt analog Beispiel 68 aus 4'-[(2-n-Butyl-3H-imidazo[4,5-b]pyridin-5-on-3-yl)methyl]-2-(1-triphenylmethyl-tetrazol-5-yl)biphenyl und methanolischer Salzsäure.
Ausbeute: 18 % der Theorie,
Schmelzpunkt: amorph

| $C_{24}H_{23}N_7O$ (425,50) | | | |
|---|---|---|---|
| Ber.: | C 67,75 | H 5,45 | N 23,04 |
| Gef.: | 67,54 | 5,42 | 22,91 |

Beispiel 90

4'-[[2-n-Propyl-5-(2,2-dimethylpropionylamino)-3H-imidazo[4,5-b]pyridin-3-yl]methyl]-2-(1H-tetrazol-5-yl)-biphenyl

Hergestellt analog Beispiel 68 aus 4'-[[2-n-Propyl-5-(2,2-dimethylpropionylamino)-3H-imidazo[4,5-b]-pyridin-3-yl]methyl]-2-(1-triphenylmethyl-tetrazol-5-yl)biphenyl und methanolischer Salzsäure.
Ausbeute: 81 % der Theorie,
Schmelzpunkt: 217-220 °C

| $C_{28}H_{30}N_8O$ (494,60) | | | |
|---|---|---|---|
| Ber.: | C 67,99 | H 6,11 | N 22,66 |
| Gef.: | 67,82 | 6,22 | 22,46 |

Beispiel 91

4'-[(2-n-Propyl-5-cyclohexylcarbonylamino-3H-imidazo[4,5-b]pyridin-3-yl)methyl]-2-(1H-tetrazol-5-yl)biphenyl

Hergestellt analog Beispiel 68 aus 4'-[(2-n-Propyl-5-cyclohexylcarbonylamino-3H-imidazo[4,5-b]pyridin-3-yl)methyl]-2-(1-triphenylmethyl-tetrazol-5-yl)biphenyl und methanolischer Salzsäure.
Ausbeute: 89 % der Theorie,
Schmelzpunkt: 229-231 °C

| $C_{30}H_{32}N_8O$ (520,64) | | | |
|---|---|---|---|
| Ber.: | C 69,21 | H 6,20 | N 21,52 |
| Gef.: | 69,14 | 6,20 | 21,32 |

Beispiel 92

4'-[(2-n-Butyl-5-methyl-6-dimethylaminocarbonylamino-3H-imidazo[4,5-b]pyridin-3-yl)methyl]biphenyl-2-carbonsäure

Hergestellt analog Beispiel 12 aus 4'-[(2-n-Butyl-5-methyl-6-dimethylaminocarbonylamino-3H-imidazo-[4,5-b]pyridin-3-yl)methyl]biphenyl-2-carbonsäure-tert.butylester und Trifluoressigsäure.
Ausbeute: 74 % der Theorie,
Schmelzpunkt: 220-221 °C

| $C_{28}H_{31}N_5O_3$ (485,58) | | | |
|---|---|---|---|
| Ber.: | C 69,26 | H 6,44 | N 14,42 |
| Gef.: | 69,08 | 6,47 | 14,25 |

Beispiel 93

4'-[(2-n-Butyl-4-chlor-3H-imidazo[4,5-c]pyridin-3-yl)methyl]biphenyl-2-carbonsäure

Hergestellt analog Beispiel 12 aus 4'-[(2-n-Butyl-4-chlor-3H-imidazo[4,5-c]pyridin-3-yl)methyl]biphenyl-2-carbonsäure-tert.butylester und Trifluoressigsäure.
Ausbeute: 85 % der Theorie,
Schmelzpunkt: 221-222 °C

| $C_{24}H_{22}ClN_3O_2$ (419,92) | | | |
|---|---|---|---|
| Ber.: | C 68,65 | H 5,28 | N 10,00 |
| Gef.: | 68,66 | 5,15 | 10,19 |

Beispiel 94

4'-[(2-n-Butyl-4-chlor-1H-imidazo[4,5-c]pyridin-1-yl)methyl]biphenyl-2-carbonsäure

Hergestellt analog Beispiel 12 aus 4'-[(2-n-Butyl-4-chlor-1H-imidazo[4,5-c]pyridin-1-yl)methyl]biphenyl-2-carbonsäure-tert.butylester und Trifluoressigsäure.
Ausbeute: 85 % der Theorie,
Schmelzpunkt: 221-222 °C

| $C_{24}H_{22}ClN_3O_2$ (419,92) | | | |
|---|---|---|---|
| Ber.: | C 68,65 | H 5,28 | N 10,00 |
| Gef.: | 68,56 | 5,18 | 10,09 |

Beispiel 95

4'-[(2-Ethyl-5-propionylamino-3H-imidazo[4,5-b]pyridin-3-yl)methyl]-biphenyl-2-carbonsäure

Hergestellt analog Beispiel 12 aus 4'-[(2-Ethyl-5-propionylamino-3H-imidazo[4,5-b]pyridin-3-yl)methyl]-biphenyl-2-carbonsäure-tert.butylester und Trifluoressigsäure.
Ausbeute: 99 % der Theorie,
Schmelzpunkt: 283-293 °C

| $C_{25}H_{24}N_4O_3$ (428,50) | | | |
|---|---|---|---|
| Ber.: | C 70,08 | H 5,65 | N 13,08 |
| Gef.: | 69,87 | 5,68 | 13,05 |

Beispiel 96

4'-[[2-n-propyl-5-(5-hydroxy-valeroylamino)-3H-imidazo[4,5-b]pyridin-3-yl]-methyl]-biphenyl-2-carbonsäure

Hergestellt analog Beispiel 12 aus 4'-[[2-n-Propyl-5-(5-hydroxy-valeroylamino)-3H-imidazo[4,5-b]pyridin-3-yl]-methyl]-biphenyl-2-carbonsäure-tert.butylester und Trifluoressigsäure.
Ausbeute: 80 % der Theorie,
Schmelzpunkt: 220 °C

| $C_{28}H_{30}N_4O_4$ (486,60) | | | |
|---|---|---|---|
| Ber.: | C 69,12 | H 6,22 | N 11,52 |
| Gef.: | 68,97 | 6,44 | 11,39 |

Beispiel 97

4'-[[2-Ethyl-5-(3-methyl-3,4,5,6-tetrahydro-2(1H)-pyrimidinon-1-yl)-3H-imidazo[4,5-b]pyridin-3-yl]-methyl]-biphenyl-2-carbonsäure-semihydrat

Hergestellt analog Beispiel 12 aus 4'-[[2-Ethyl-5-(3-methyl-3,4,5,6-tetrahydro-2(1H)-pyrimidinon-1-yl)-3H-imidazo[4,5-b]pyridin-3-yl]-methyl]-biphenyl-2-carbonsäure-tert.butylester und Trifluoressigsäure.
Ausbeute: 62 % der Theorie,
Schmelzpunkt: ab 250 °C (Zers.)

| $C_{27}H_{27}N_5O_3$ x 1/2 $H_2O$ (478,60) | | | |
|---|---|---|---|
| Ber.: | C 67,77 | H 5,90 | N 14,64 |
| Gef.: | 67,98 | 5,84 | 14,66 |

Beispiel 98

4'-[[2-n-Propyl-5-(3-methyl-3,4,5,6-tetrahydro-2(1H)-pyrimidinon-1-yl)-3H-imidazo[4,5-b]pyridin-3-yl]-methyl]-biphenyl-2-carbonsäure

Hergestellt analog Beispiel 12 aus 4'-[[2-n-Propyl-5-(3-methyl-3,4,5,6-tetrahydro-2(1H)-pyrimidinon-1-yl)-3H-imidazo[4,5-b]pyridin-3-yl]-methyl]-biphenyl-2-carbonsäure-tert.butylester und Trifluoressigsäure.
Ausbeute: 74 % der Theorie,
Schmelzpunkt: 202-205 °C

| $C_{28}H_{29}N_5O_3$ (483,57) | | | |
|---|---|---|---|
| Ber.: | C 69,55 | H 6,04 | N 14,48 |
| Gef.: | 69,30 | 5,98 | 14,62 |

Beispiel 99

4'-[[2-Ethyl-5-(3-benzyl-3,4,5,6-tetrahydro-2(1H)-pyrimidinon-1-yl)-3H-imidazo[4,5-b]pyridin-3-yl]-methyl]-biphenyl-2-carbonsäure-semihydrat

Hergestellt analog Beispiel 12 aus 4'-[[2-Ethyl-5-(3-benzyl-3,4,5,6-tetrahydro-2(1H)-pyrimidinon-1-yl)-3H-imidazo[4,5-b]pyridin-3-yl]-methyl]-biphenyl-2-carbonsäure-tert.butylester und Trifluoressigsäure.
Ausbeute: 66 % der Theorie,
Schmelzpunkt: 137-138 °C

| $C_{33}H_{31}N_5O_3$ x 0,5 $H_2O$ (545,70) | | | |
|---|---|---|---|
| Ber.: | C 72,64 | H 5,73 | N 12,84 |
| Gef.: | 72,40 | 5,76 | 12,88 |

Beispiel 100

4'-[[2-n-Propyl-5-(3-benzyl-3,4,5,6-tetrahydro-2(1H)-pyrimidinon-1-yl)-3H-imidazo[4,5-b]pyridin-3-yl]-methyl]-biphenyl-2-carbonsäure

Hergestellt analog Beispiel 12 aus 4'-[[2-n-Propyl-5-(3-benzyl-3,4,5,6-tetrahydro-2(1H)-pyrimidinon-1-yl)-3H-imidazo[4,5-b]pyridin-3-yl]-methyl]-biphenyl-2-carbonsäure-tert.butylester und Trifluoressigsäure.
Ausbeute: 77 % der Theorie,
Schmelzpunkt: 149-152 °C

| $C_{34}H_{33}N_5O_3$ (559,67) | | | |
|---|---|---|---|
| Ber.: | C 72,97 | H 5,94 | N 12,51 |
| Gef.: | 73,11 | 5,91 | 12,39 |

Beispiel 101

4'-[[2-n-Propyl-5-(N-cyclohexylaminocarbonyl-ethylamino)-3H-imidazo[4,5-b]pyridin-3-yl]-methyl]-biphenyl-2-carbonsäure

Hergestellt analog Beispiel 12 aus 4'-[[2-n-Propyl-5-(N-cyclohexylaminocarbonyl-ethylamino)-3H-imidazo[4,5-b]pyridin-3-yl]-methyl]-biphenyl-2-carbonsäure-tert.butylester und Trifluoressigsäure.
Ausbeute: 10 % der Theorie,
Schmelzpunkt: 166-167 °C

| $C_{34}H_{33}N_5O_3$ (539,69) | | | |
|---|---|---|---|
| Ber.: | C 71,22 | H 6,91 | N 12,98 |
| Gef.: | 71,37 | 6,70 | 12,80 |

Beispiel 102

4'-[[2-Ethyl-5-(N-cyclohexylaminocarbonyl-ethylamino)-3H-imidazo[4,5-b]pyridin-3-yl]-methyl]-biphenyl-2-carbonsäure

Hergestellt analog Beispiel 12 aus 4'-[[2-Ethyl-5-(N-cyclohexylaminocarbonyl-ethylamino)-3H-imidazo-[4,5-b]pyridin-3-yl]-methyl]-biphenyl-2-carbonsäure-tert.butylester und Trifluoressigsäure.
Ausbeute: 25 % der Theorie,
Schmelzpunkt: 95-100 °C (Zers.)

| $C_{31}H_{35}N_5O_3$ (525,66) | | | |
|---|---|---|---|
| Ber.: | C 70,83 | H 6,71 | N 13,32 |
| Gef.: | 70,69 | 6,65 | 13,30 |

Beispiel 103

4'-[[2-n-Propyl-5-(N-cyclohexylcarbonyl-ethylamino)-3H-imidazo[4,5-b]pyridin-3-yl]-methyl]-biphenyl-2-carbonsäure

Hergestellt analog Beispiel 12 aus 4'-[[2-n-Propyl-5-(N-cyclohexylcarbonyl-ethylamino)-3H-imidazo[4,5-b]pyridin-3-yl]-methyl]-biphenyl-2-carbonsäure-tert.butylester und Trifluoressigsäure.
Ausbeute: 83 % der Theorie,
Schmelzpunkt: 112-115 °C

| $C_{32}H_{36}N_4O_3$ (524,67) | | | |
|---|---|---|---|
| Ber.: | C 73,26 | H 6,92 | N 10,68 |
| Gef.: | 73,18 | 7,19 | 10,67 |

Beispiel 104

4'-[[2-Ethyl-5-(N-cyclohexylcarbonyl-ethylamino)-3H-imidazo[4,5-b]pyridin-3-yl]-methyl]-biphenyl-2-carbonsäure

Hergestellt analog Beispiel 12 aus 4'-[[2-Ethyl-5-(N-cyclohexylcarbonyl-ethylamino)-3H-imidazo[4,5-b]-pyridin-3-yl]-methyl]-biphenyl-2-carbonsäure-tert.butylester und Trifluoressigsäure.
Ausbeute: 91 % der Theorie,
Schmelzpunkt: 223-224 ° C

| $C_{31}H_{34}N_4O_3$ (510,65) | | | |
|---|---|---|---|
| Ber.: | C 72,92 | H 6,71 | N 10,97 |
| Gef.: | 72,97 | 6,65 | 10,93 |

Beispiel 105

4'-[[2-(N-Isobutyryl-n-butylamino)-8-n-butyl-9H-purin-9-yl]-methyl]-biphenyl-2-carbonsäure

Hergestellt analog Beispiel 4 aus 4'-[[2-(n-Butylamino)-8-n-butyl-9H-purin-9-yl]-methyl]-biphenyl-2-carbonsäure und Isobuttersäurechlorid.
Ausbeute: 38 % der Theorie,
Schmelzpunkt: 80 ° C

| $C_{31}H_{37}N_5O_3$ (527,68) | | | |
|---|---|---|---|
| Ber.: | C 70,56 | H 7,07 | N 13,27 |
| Gef.: | 70,45 | 7,22 | 13,06 |

Beispiel 106

4'-[[2-n-Butyl-5-methyl-6-(2-oxo-piperidin-1-yl)-3H-imidazo[4,5-b]pyridin-3-yl]-methyl]-biphenyl-2-carbonsäure

Hergestellt analog Beispiel 12 aus 4'-[[2-n-Butyl-5-methyl-6-(2-oxo-piperidin-1-yl)-3H-imidazo[4,5-b]-pyridin-3-yl]-methyl]-biphenyl-2-carbonsäure-tert.butylester und Trifluoressigsäure.
Ausbeute: 77 % der Theorie,
Schmelzpunkt: 266-268 ° C

| $C_{30}H_{32}N_4O_3$ (496,62) | | | |
|---|---|---|---|
| Ber.: | C 72,56 | H 6,50 | N 11,23 |
| Gef.: | 72,46 | 6,73 | 11,10 |

Beispiel 107

4'-[[2-n-Butyl-5-methyl-6-(n-butansultam-1-yl)-3H-imidazo[4,5-b]pyridin-3-yl]-methyl]-biphenyl-2-carbonsäure

Hergestellt analog Beispiel 12 aus 4'-[[2-n-Butyl-5-methyl-6-(n-butansultam-1-yl)-3H-imidazo[4,5-b]-pyridin-3-yl]-methyl]-biphenyl-2-carbonsäure-tert.butylester und Trifluoressigsäure.
Ausbeute: 78 % der Theorie,
Schmelzpunkt: 287-288 ° C

| $C_{29}H_{32}N_4O_4S$ (532,67) | | | |
|---|---|---|---|
| Ber.: | C 65,39 | H 6,06 | N 10,52 |
| Gef.: | 65,26 | 6,25 | 10,37 |

Beispiel 108

4'-[[2-n-Butyl-5-methyl-6-(N-cyclohexylaminocarbonyl-ethylamino)-1H-imidazo[4,5-b]pyridin-1-yl]-methyl]-biphenyl-2-carbonsäure

Hergestellt analog Beispiel 12 aus 4'-[[2-n-Butyl-5-methyl-6-(N-cyclohexylaminocarbonyl-ethylamino)-1H-imidazo[4,5-b]pyridin-1-yl]-methyl]-biphenyl-2-carbonsäure-tert.butylester und Trifluoressigsäure.
Ausbeute: 88 % der Theorie,
Schmelzpunkt: 177-179 °C

| $C_{34}H_{31}N_5O_3$ (567,74) | | | |
|---|---|---|---|
| Ber.: | C 71,93 | H 7,28 | N 12,34 |
| Gef.: | 71,76 | 7,20 | 12,13 |

Beispiel 109

4'-[[2,5-Dimethyl-6-(3-benzyl-3,4,5,6-tetrahydro-2(1H)-pyrimidinon-1-yl)-3H-imidazo[4,5-b]pyridin-3-yl]-methyl]-biphenyl-2-carbonsäure

Hergestellt analog Beispiel 12 aus 4'-[[2,5-Dimethyl-6-(3-benzyl-3,4,5,6-tetrahydro-2(1H)-pyrimidinon-1-yl)-3H-imidazo[4,5-b]pyridin-3-yl]-methyl]-biphenyl-2-carbonsäure-tert.butylester und Trifluoressigsäure.
Ausbeute: 74 % der Theorie,
Schmelzpunkt: 277-280 °C

| $C_{33}H_{31}N_5O_3$ (545,65) | | | |
|---|---|---|---|
| Ber.: | C 72,64 | H 5,73 | N 12,84 |
| Gef.: | 72,87 | 5,57 | 12,66 |

Beispiel 110

4'-[[2,5-Dimethyl-6-(3-benzyl-3,4,5,6-tetrahydro-2(1H)-pyrimidinon-1-yl)-1H-imidazo[4,5-b]pyridin-1-yl]-methyl]-biphenyl-2-carbonsäure

Hergestellt analog Beispiel 12 aus 4'-[[2,5-Dimethyl-6-(3-benzyl-3,4,5,6-tetrahydro-2(1H)-pyrimidinon-1-yl)-1H-imidazo[4,5-b]pyridin-1-yl]-methyl]-biphenyl-2-carbonsäure-tert.butylester und Trifluoressigsäure.
Ausbeute: 77 % der Theorie,
Schmelzpunkt: 142-144 °C

| $C_{33}H_{31}N_5O_3$ (545,65) | | | |
|---|---|---|---|
| Ber.: | C 72,64 | H 5,73 | N 12,84 |
| Gef.: | 72,59 | 5,51 | 12,60 |

Beispiel 111

4'-[[2-Ethyl-5-methyl-6-(3-benzyl-3,4,5,6-tetrahydro-2(1H)-pyrimidinon-1-yl)-3H-imidazo[4,5-b]pyridin-3-yl]-methyl]-biphenyl-2-carbonsaure

Hergestellt analog Beispiel 12 aus 4'-[[2-Ethyl-5-methyl-6-(3-benzyl-3,4,5,6-tetrahydro-2(1H)-pyrimidinon-1-yl)-3H-imidazo[4,5-b]pyridin-3-yl]-methyl]-biphenyl-2-carbonsäure-tert.butylester und Trifluoressigsäure.
Ausbeute: 73 % der Theorie,
Schmelzpunkt: 272-274° C

| $C_{34}H_{33}N_5O_3$ (559,68) | | | |
|---|---|---|---|
| Ber.: | C 72,97 | H 5,94 | N 12,51 |
| Gef.: | 72,87 | 5,97 | 12,37 |

Beispiel 112

4'-[(2-n-Butyl-5-ethyl-6-dimethylaminocarbonylamino-3H-imidazo[4,5-b]pyridin-3-yl)methyl]-biphenyl-2-carbonsäure-trifluoracetat

Hergestellt analog Beispiel 12 aus 4'-[(2-n-Butyl-5-ethyl-6-dimethylaminocarbonylamino-3H-imidazo[4,5-b]pyridin-3-yl)methyl]-biphenyl-2-carbonsäure-tert.butylester und Trifluoressigsäure.
Ausbeute: 72 % der Theorie,
Schmelzpunkt: 180-182° C

| $C_{29}H_{33}N_5O_3$ x $CF_3COOH$ (613,65) | | | |
|---|---|---|---|
| Ber.: | C 60,67 | H 5,58 | N 11,41 |
| Gef.: | 60,81 | 5,81 | 11,65 |

Beispiel 113

4'-[(2-n-Propyl-5-methyl-6-dimethylaminocarbonylamino-3H-imidazo[4,5-b]pyridin-3-yl)methyl]-biphenyl-2-carbonsäure-trifluoracetat

Hergestellt analog Beispiel 12 aus 4'-[(2-n-Propyl-5-methyl-6-dimethylaminocarbonylamino-3H-imidazo-[4,5-b]pyridin-3-yl)methyl]-biphenyl-2-carbonsäure-tert.butylester und Trifluoressigsäure.
Ausbeute: 81 % der Theorie,
Schmelzpunkt: 243-245° C

| $C_{27}H_{29}N_5O_3$ x $CF_3COOH$ (585,60) | | | |
|---|---|---|---|
| Ber.: | C 59,48 | H 5,16 | N 11,96 |
| Gef.: | 59,68 | 5,26 | 11,99 |

Beispiel 114

4'-[(2-n-Propyl-5-methyl-6-dimethylaminocarbonylamino-1H-imidazo[4,5-b]pyridin-1-yl)methyl]-biphenyl-2-carbonsäure-trifluoracetat

Hergestellt analog Beispiel 12 aus 4'-[(2-n-Propyl-5-methyl-6-dimethylaminocarbonylamino-1H-imidazo-[4,5-b]pyridin-1-yl)methyl]-biphenyl-2-carbonsäure-tert.butylester und Trifluoressigsäure.
Ausbeute: 90 % der Theorie,
Schmelzpunkt: 213-216° C

| $C_{27}H_{29}N_5O_3$ x $CF_3COOH$ (585,60) | | | |
|---|---|---|---|
| Ber.: | C 59,48 | H 5,16 | N 11,96 |
| Gef.: | 59,61 | 5,13 | 11,77 |

Beispiel 115

4'-[(2-n-Butyl-5-methyl-6-diethylaminocarbonylamino-3H-imidazo[4,5-b]pyridin-3-yl)methyl]-biphenyl-2-carbonsäure-trifluoracetat

Hergestellt analog Beispiel 12 aus 4'-[(2-n-Butyl-5-methyl-6-diethylaminocarbonylamino-3H-imidazo[4,5-b]pyridin-3-yl)methyl]-biphenyl-2-carbonsäure-tert.butylester und Trifluoressigsäure.
Ausbeute: 90 % der Theorie,
Schmelzpunkt: 137-140 ° C

| $C_{30}H_{35}N_5O_3$ x $CF_3COOH$ (627,67) | | | |
|---|---|---|---|
| Ber.: | C 61,23 | H 5,78 | N 11,16 |
| Gef.: | 60,97 | 5,93 | 10,98 |

Beispiel 116

4'-[[2-n-Butyl-5-ethyl-6-(3-benzyl-3,4,5,6-tetrahydro-2(1H)-pyrimidinon-1-yl)-3H-imidazo[4,5-b]pyridin-3-yl)-methyl]-biphenyl-2-carbonsäure

Hergestellt analog Beispiel 12 aus 4'-[[2-n-Butyl-5-ethyl-6-(3-benzyl-3,4,5,6-tetrahydro-2(1H)-pyrimidinon-1-yl)-3H-imidazo[4,5-b]pyridin-3-yl]-methyl]-biphenyl-2-carbonsäure-tert.butylester und Trifluoressigsäure.
Ausbeute: 85 % der Theorie,
Schmelzpunkt: 173-175 ° C

| $C_{37}H_{39}N_5O_3$ (601,76) | | | |
|---|---|---|---|
| Ber.: | C 73,85 | H 6,53 | N 11,64 |
| Gef.: | 73,63 | 6,40 | 11,41 |

Beispiel 117

4'-[[2-n-Propyl-5-methyl-6-(3-benzyl-3,4,5,6-tetrahydro-2(1H)-pyrimidinon-1-yl)-3H-imidazo[4,5-b]pyridin-3-yl]-methyl]-biphenyl-2-carbonsäure

Hergestellt analog Beispiel 12 aus 4'-[[2-n-Propyl-5-methyl-6-(3-benzyl-3,4,5,6-tetrahydro-2(1H)-pyrimidinon-1-yl)-3H-imidazo[4,5-b]pyridin-3-yl]-methyl]-biphenyl-2-carbonsäure-tert.butylester und Trifluoressigsäure.
Ausbeute: 64 % der Theorie,
Schmelzpunkt: 199-201 ° C

| $C_{35}H_{35}N_5O_3$ (573,69) | | | |
|---|---|---|---|
| Ber.: | C 73,27 | H 6,05 | N 12,21 |
| Gef.: | 73,20 | 6,19 | 12,08 |

Beispiel 118

4'-[[2-n-Butyl-5-methyl-6-(3-dimethylaminocarbonyl-3,4,5,6-tetrahydro-2(1H)-pyrimidinon-1-yl)-3H-imidazo-[4,5-b]pyridin-3-yl]-methyl]-biphenyl-2-carbonsäure

Hergestellt analog Beispiel 12 aus 4'-[[2-n-Butyl-5-methyl-6-(3-dimethylaminocarbonyl-3,4,5,6-tetrahydro-2(1H)-pyrimidinon-1-yl)-3H-imidazo[4,5-b]pyridin-3-yl]-methyl]-biphenyl-2-carbonsäure-tert.butylester und Trifluoressigsäure.

Beispiel 119

4'-[[2-n-Butyl-5-methyl-6-(3-benzyl-3,4,5-trihydro-2(1H)-imidazolinon-1-yl)-3H-imidazo[4,5-b]pyridin-3-yl]-methyl]-biphenyl-2-carbonsäure

Hergestellt analog Beispiel 12 aus 4'-[[2-n-Butyl-5-methyl-6-(3-benzyl-3,4,5-trihydro-2(1H)-imidazolinon-1-yl)-3H-imidazo[4,5-b]pyridin-3-yl]-methyl]-biphenyl-2-carbonsäure-tert.butylester und Trifluoressigsäure.
Ausbeute: 92 % der Theorie,
Schmelzpunkt: 149-151 °C

| $C_{35}H_{35}N_5O_3$ (573,70) | | | |
|---|---|---|---|
| Ber.: | C 73,28 | H 6,15 | N 12,21 |
| Gef.: | 73,16 | 5,98 | 12,07 |

Beispiel 120

4'-[(2-n-Propyl-5-methyl-6-(cis-hexahydrophthalimido)-3H-imidazo[4,5-b]pyridin-3-yl)methyl]-biphenyl-2-carbonsäure

Hergestellt analog Beispiel 12 aus 4'-[(2-n-Propyl-5-methyl-6-(cis-hexahydrophthalimido)-3H-imidazo-[4,5-b]pyridin-3-yl)methyl]-biphenyl-2-carbonsäure-tert.butylester und Trifluoressigsäure.
Ausbeute: 77 % der Theorie,
Schmelzpunkt: 269-270 °C

| $C_{32}H_{32}N_4O_4$ (536,64) | | | |
|---|---|---|---|
| Ber.: | C 71,62 | H 6,01 | N 10,44 |
| Gef.: | 71,48 | 6,24 | 10,31 |

Beispiel 121

4'-[(2-n-Propyl-5-methyl-6-(cis-hexahydrophthalimido)-1H-imidazo[4,5-b]pyridin-1-yl)methyl]-biphenyl-2-carbonsäure

Hergestellt analog Beispiel 12 aus 4'-[(2-n-Propyl-5-methyl-6-(cis-hexahydrophthalimido)-1H-imidazo-[4,5-b]pyridin-1-yl)methyl]-biphenyl-2-carbonsäure-tert.butylester und Trifluoressigsäure.
Ausbeute: 79 % der Theorie,
Schmelzpunkt: 219-221 °C

| $C_{32}H_{32}N_4O_4$ (536,64) | | | |
|---|---|---|---|
| Ber.: | C 71,62 | H 6,01 | N 10,44 |
| Gef.: | 71,45 | 5,86 | 10,21 |

Beispiel 122

4'-[[2-n-Butyl-5-(N-phenylsulfonyl-methylamino)-3H-imidazo[4,5-b]pyridin-3-yl]-methyl]-biphenyl-2-carbonsäure Hergestellt analog Beispiel 12 aus 4'-[[2-n-Butyl-5-(N-phenylsulfonyl-methylamino)-3H-imidazo-[4,5-b]pyridin-3-yl]-methyl]-biphenyl-2-carbonsäure-tert.butylester und Trifluoressigsäure.
Ausbeute: 83 % der Theorie,
Schmelzpunkt: 226-227 ° C

| $C_{31}H_{30}N_4O_4S$ (554,60) | | | | |
|---|---|---|---|---|
| Ber.:<br>Gef.: | C 67,14<br>67,00 | H 5,45<br>5,51 | N 10,10<br>10,25 | S 5,79<br>5,78 |

Beispiel 123

4'-[[2-n-Propyl-5-(3-benzyl-3,4,5,6-tetrahydro-2(1H)-pyrimidinon-1-yl)-3H-imidazo[4,5-b]pyridin-3-yl]-methyl]-2-(1H-tetrazol-5-yl)-biphenyl

Hergestellt analog Beispiel 68 aus 4'-[[2-n-Propyl-5-(3-benzyl-3,4,5,6-tetrahydro-2(1H)-pyrimidinon-1-yl)-3H-imidazo[4,5-b]pyridin-3-yl]-methyl]-2-(1-triphenylmethyl-tetrazol-5-yl)-biphenyl und methanolischer Salzsäure.
Ausbeute: 72 % der Theorie,
Schmelzpunkt: amorph

| $C_{34}H_{33}N_9O$ (583,70) | | | |
|---|---|---|---|
| Ber.:<br>Gef.: | C 69,96<br>70,11 | H 5,70<br>5,57 | N 21,60<br>21,49 |

Beispiel 124

4'-[[2-n-Propyl-5-(2-oxo-indolin-1-yl)-3H-imidazo[4,5-b]pyridin-3-yl]-methyl]-2-(1H-tetrazol-5-yl)-biphenyl

Hergestellt analog Beispiel 68 aus 4'-[[2-n-Propyl-5-(2-oxo-indolin-1-yl)-3H-imidazo[4,5-b]pyridin-3-yl]-methyl]-2-(1-triphenylmethyl-tetrazol-5-yl)-biphenyl und methanolischer Salzsäure.
Ausbeute: 11 % der Theorie,
Schmelzpunkt: sintert ab 190 ° C
$C_{31}H_{26}N_8O$ (526,61)
Massenspektrum: $(M+H)^+$ = 527

Beispiel 125

4'-[(2-n-Butyl-5-methyl-6-dimethylaminocarbonylamino-3H-imidazo[4,5-b]pyridin-3-yl)methyl]-2-(1H-tetrazol-5-yl)-biphenyl

Hergestellt analog Beispiel 68 aus 4'-[(2-n-Butyl-5-methyl-6-dimethylaminocarbonylamino-3H-imidazo-[4,5-b]pyridin-3-yl) methyl]-2-(1-triphenylmethyl-tetrazol-5-yl)-biphenyl und methanolischer Salzsäure.

Beispiel 126

4'-[[2-Ethyl-5-(2,2-dimethyl-propionylamino)-3H-imidazo[4,5-b]pyridin-3-yl]-methyl]-2-(1H-tetrazol-5-yl)-biphenylhydrochlorid

Hergestellt analog Beispiel 68 aus 4'-[[2-Ethyl-5-(2,2-dimethyl-propionylamino)-3H-imidazo[4,5-b]pyridin-3-yl]-methyl]-2-(1-triphenylmethyl-tetrazol-5-yl)-biphenyl und methanolischer Salzsäure.
Ausbeute: 52 % der Theorie,
Schmelzpunkt: ab 188 °C sintern

| $C_{27}H_{28}N_8O$ x HCl (517,04) | | | | |
|---|---|---|---|---|
| Ber.:<br>Gef.: | C 62,95<br>62,73 | H 5,65<br>5,47 | N 21,90<br>21,75 | Cl 6,85<br>6,67 |

Beispiel 127

4'-[(2-n-Butyl-5-ethyl-6-phthalimido-1H-imidazo[4,5-b]pyridin-1-yl)-methyl]-biphenyl-2-carbonsäure

Hergestellt analog Beispiel 12 aus 4'-[(2-n-Butyl-5-ethyl-6-phthalimido-1H-imidazo[4,5-b]pyridin-1-yl)-methyl]-biphenyl-2-carbonsäure-tert.butylester und Trifluoressigsäure.
Ausbeute: 56 % der Theorie,
Schmelzpunkt: 277-279 °C

| $C_{34}H_{30}N_4O_4$ (558,64) | | | |
|---|---|---|---|
| Ber.:<br>Gef.: | C 73,10<br>72,97 | H 5,41<br>5,52 | N 10,03<br>10,16 |

Beispiel 128

4'-[(2-n-Butyl-5-methoxy-1H-imidazo[4,5-b]pyridin-1-yl)-methyl]-2-(1H-tetrazol-5-yl)-biphenyl

Hergestellt analog Beispiel 68 aus 4'-[(2-n-Butyl-5-methoxy-1H-imidazo[4,5-b]pyridin-1-yl)-methyl]-2-(1-triphenylmethyl-tetrazol-5-yl)-biphenyl und methanolischer Salzsäure. Ausbeute: 60 % der Theorie,
Schmelzpunkt: 170-171 °C

| $C_{25}H_{25}N_7O$ (439,53) | | | |
|---|---|---|---|
| Ber.:<br>Gef.: | C 68,32<br>68,19 | H 5,73<br>5,53 | N 22,31<br>22,06 |

Bei den nachfolgenden pharmazeutischen Anwendungsbeispielen kann als Wirksubstanz jede geeignete Verbindung der Formel I eingesetzt werden, insbesondere die Verbindungen A bis K des pharmakologischen Versuchsberichtes:

Beispiel I

| Ampullen, enthaltend 50 mg Wirkstoff pro 5 ml | |
|---|---|
| Wirkstoff | 50 mg |
| $KH_2PO_4$ | 2 mg |
| $Na_2HPO_4$ x $2H_2O$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

Ampullen, enthaltend 100 mg Wirkstoff pro 5 ml

| | |
|---|---|
| Wirkstoff | 100 mg |
| Methylglucamin | 35 mg |
| Glykofurol | 1000 mg |
| Polyethylenglykol-Polypropylen-glykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel III

Tabletten, enthaltend 50 mg Wirkstoff

| | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpresst.

Beispiel IV

Dragees, enthaltend 50 mg Wirkstoff

| | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

Dragees, enthaltend 100 mg Wirkstoff

| | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45 °C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden. Beispiel VI

Kapseln, enthaltend 250 mg Wirkstoff

| | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70° C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

Suppositorien, enthaltend 100 mg Wirkstoff

| | |
|---|---|
| Wirkstoff | 100,0 mg |
| Adeps solidus | 1600,0 mg |
| | 1700,0 mg |

Herstellung:

Das Hartfett wird geschmolzen. Bei 40° C wird die gemahlene Wirksubstanz in der Schmelze homogen dispergiert. Es wird auf 38° C abgekühlt und in schwach vorgekühlte Suppositorienformen ausgegossen.

## Patentansprüche

1. Heterocyclische Imidazole der allgemeinen Formel

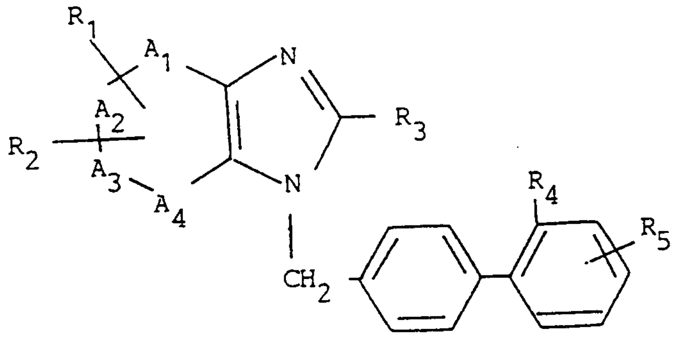

(I)

in der

einer oder zwei der Reste $A_1$, $A_2$, $A_3$ oder $A_4$ ein Stickstoffatom und die verbleibenden Reste $A_1$, $A_2$, $A_3$ oder $A_4$ jeweils eine Methingruppe,

$R_1$ ein Fluor-, Chlor- oder Bromatom, eine Hydroxy-, Alkyl- oder Alkoxygruppe, eine gegebenenfalls am Stickstoffatom durch eine Alkylgruppe mit 1 bis 6 Kohlenstoffatomen, durch eine Phenyl-, Cycloalkyl-, Phenylalkyl- oder Cycloalkylalkylgruppe mono- oder disubstituierte Aminogruppe, wobei die Substituenten gleich oder verschieden sein können, oder monosubstituierte N-Acylaminogruppe, in welcher der Acylrest eine Alkanoylgruppe mit 1 bis 7 Kohlenstoffatomen, welche in 3-, 4-, 5-, 6- oder 7-Stellung durch ein Halogenatom oder eine Hydroxygruppe substituiert sein kann, eine Alkoxycarbonylgruppe mit insgesamt 2 bis 4 Kohlenstoffatomen, eine Alkylsulfonylgruppe mit 1 bis 6 Kohlenstoffatomen, eine Benzoyl-, Benzolsulfonyl-, Phenylalkansulfonyl-, Naphthalensulfonyl-, 2-Carboxy-cyclohexylcarbonyl-, 2-Aminocarbonyl-cyclohexylcarbonyl-, Cycloalkylcarbonyl-, Phenylalkanoyl- oder Cycloalkylalkanoylgruppe darstellt, wobei die vorstehend erwähnten Phenylkerne durch ein Fluor-, Chlor- oder Bromatom, durch eine Methyl- oder Methoxygruppe mono- oder disubstituiert und die Substituenten gleich oder verschieden sein können, eine Alkylsultamgruppe mit 3 bis 5 Kohlenstoffatomen im Alkylteil, eine gegebenenfalls durch eine Alkyl- oder Hydroxycarbonylalkylgruppe substituierte Pyrrolidino-, Piperidino- oder Hexamethyleniminogruppe, eine Pyrrolidinon-, Piperidinon- oder Hexamethyleniminongruppe, eine gegebenenfalls ganz oder teilweise hydrierte Phthalimido- oder 2-Oxo-isoindolin-1-ylgruppe oder eine Gruppe der Formel

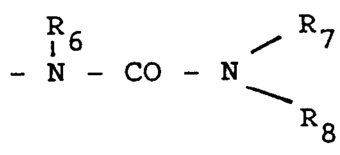

,

in welcher

$R_6$, $R_7$ und $R_8$, die gleich oder verschieden sein können, Wasserstoffatome, Alkylgruppen mit 1 bis 8 Kohlenstoffatomen, Cycloalkyl-, Phenyl-, Cycloalkylalkyl- oder Phenylalkylgruppen oder

$R_7$ auch eine 2-Chlor-ethyl-, 2-Brom-ethyl-, 3-Chlor-propyl- oder 3-Brom-propylgruppe oder auch, wenn $R_6$ und $R_7$ zusammen eine Ethylen- oder Propylengruppe darstellen, eine Dialkylaminocarbonylgruppe, oder

$R_6$ und $R_7$ zusammen eine Ethylen- oder Propylengruppe darstellen, wobei, der Phenylkern der vorstehend erwähnten Phenyl- und Phenylalkylgruppen jeweils durch Hydroxy- oder Alkoxygruppen mono- oder disubstituiert und die Substituenten gleich oder verschieden sein können, und, soweit

nichts anderes erwähnt wurde, die vorstehend erwähnten Alkyl- und Alkoxyteile jeweils 1 bis 3 Kohlenstoffatome, die Alkanoylteile 1 bis 4 Kohlenstoffatome und die Cycloalkylteile jeweils 5 bis 7 Kohlenstoffatome enthalten können, oder

$R_1$ auch ein Wasserstoffatom, wenn nicht gleichzeitig $R_2$ ein Wasserstoffatom, $R_3$ eine n-Butylgruppe, $R_4$ eine Carboxygruppe und $R_5$ ein Wasserstoffatom sowie
(i) einer der Reste $A_1$ bis $A_4$ ein Stickstoffatom und die verbleibenden Reste $A_1$ bis $A_4$ jeweils eine Methingruppe oder
(ii) $A_2$ und $A_4$ oder $A_1$ und $A_3$ jeweils ein Stickstoffatom und die verbleibenden Reste $A_1$ und $A_3$ oder $A_2$ und $A_4$ jeweils eine Methingruppe oder
(iii) $A_4$ ein Stickstoffatom und $A_3$ eine durch eine Hydroxy- oder Methoxygruppe substituierte Methingruppe und die verbleibenden Reste $A_1$ und $A_2$ jeweils eine Methingruppe oder
(iv) $A_4$ ein Stickstoffatom, $A_1$ durch eine Methylgruppe substituierte Methingruppe und die verbleibenden Reste $A_2$ und $A_3$ jeweils eine Methingruppe darstellen,
$R_2$ ein Wasserstoffatom oder eine Alkylgruppe mit 1 bis 3 Kohlenstoffatomen,

$R_3$ eine Alkylgruppe mit 1 bis 6 Kohlenstoffatomen,

$R_4$ eine Carboxy-, Cyano-, 1H-Tetrazolyl- oder 1-Triphenylmethyl-tetrazolylgruppe oder eine Alkoxycarbonylgruppe mit insgesamt 2 bis 5 Kohlenstoffatomen und

$R_5$ ein Wasserstoff-, Fluor-, Chlor- oder Bromatom bedeuten,

deren 1-, 3-Isomerengemische, deren Tautomere, deren Enantiomere sowie deren Additionssalze mit anorganischen oder organischen Säuren oder Basen.

**2.** Heterocyclische Imidazole der allgemeinen Formel I gemaß Anspruch 1, in der

einer oder zwei der Reste $A_1$, $A_2$, $A_3$ oder $A_4$ ein Stickstoffatom und die verbleibenden Reste $A_1$, $A_2$, $A_3$ oder $A_4$ jeweils eine Methingruppe,

$R_1$ ein Fluoratom, eine am Stickstoffatom durch eine Phenyl-, Cycloalkyl-, Phenylalkyl- oder Cycloalkylalkylgruppe substituierte Amino- oder Alkylaminogruppe mit 1 bis 6 Kohlenstoffatomen, eine durch eine Phenyl-, Cycloalkyl-, Phenylalkyl- oder Cycloalkylalkylgruppe disubstituierte Aminogruppe, wobei die Substituenten gleich oder verschieden sein können, oder eine gegebenenfalls am Stickstoffatom durch eine Alkylgruppe mit 1 bis 6 Kohlenstoffatomen oder durch eine Phenyl-, Cycloalkyl-, Phenylalkyl- oder Cycloalkylalkylgruppe substituierte Acylaminogruppe, in welcher der Acylrest eine Alkanoylgruppe mit 2 bis 7 Kohlenstoffatomen, welche endständig durch ein Chloratom oder eine Hydroxygruppe substituiert sein kann, eine Alkoxycarbonylgruppe mit insgesamt 2 bis 4 Kohlenstoffatomen, eine Alkylsulfonylgruppe mit 1 bis 6 Kohlenstoffatomen, eine Formyl-, Benzoyl-, Benzolsulfonyl-, Phenylalkansulfonyl-, Naphthalensulfonyl-, 2-Carboxy-cyclohexylcarbonyl-, 2-Aminocarbonylcyclohexylcarbonyl-, Cycloalkylcarbonyl-, Phenylalkanoyl- oder Cycloalkylalkanoylgruppe darstellt, wobei die vorstehend erwähnten Phenylkerne durch ein Fluor-, Chlor- oder Bromatom, durch eine Methyl- oder Methoxygruppe mono- oder disubstituiert und die Substituenten gleich oder verschieden sein können, eine Alkylsultamgruppe mit 3 bis 5 Kohlenstoffatomen im Alkylteil, eine gegebenenfalls durch eine Alkyl- oder Hydroxycarbonylalkylgruppe substituierte Pyrrolidino-, Piperidino- oder Hexamethyleniminogruppe, eine Pyrrolidinon-, Piperidinon- oder Hexamethyleniminongruppe, eine gegebenenfalls ganz oder teilweise hydrierte Phthalimidogruppe oder eine Gruppe der Formel

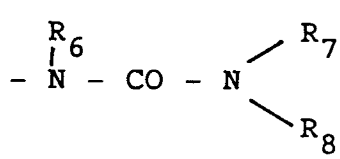
,

in welcher

$R_6$, $R_7$ und $R_8$, die gleich oder verschieden sein können, Wasserstoffatome, Alkylgruppen mit 1 bis 8 Kohlenstoffatomen, Cycloalkyl-, Phenyl-, Cycloalkylalkyl- oder Phenylalkylgruppen oder

$R_7$ auch eine 2-Chlor-ethyl-, 2-Brom-ethyl-, 3-Chlor-propyl- oder 3-Brom-propylgruppe oder auch, wenn $R_6$ und $R_7$ zusammen eine Ethylen- oder Propylengruppe darstellen, eine Dialkylaminocarbonylgruppe, oder

$R_6$ und $R_7$ zusammen eine Ethylen- oder Propylengruppe darstellen, wobei, der Phenylkern der vorstehend erwähnten Phenyl- und Phenylalkylgruppen jeweils durch Hydroxy- oder Alkoxygruppen mono- oder disubstituiert und die Substituenten gleich oder verschieden sein können, und, soweit nichts anderes erwähnt wurde, die vorstehend erwähnten Alkyl- und Alkoxyteile jeweils 1 bis 3 Kohlenstoffatome, die Alkanoylteile 1 bis 4 Kohlenstoffatome und die Cycloalkylteile jeweils 5 bis 7 Kohlenstoffatome enthalten können,

$R_2$ ein Wasserstoffatom oder eine Alkylgruppe mit 1 bis 3 Kohlenstoffatomen,

$R_3$ eine Alkylgruppe mit 1 bis 6 Kohlenstoffatomen,

$R_4$ eine Carboxy-, Cyano-, 1H-Tetrazolyl- oder 1-Triphenylmethyl-tetrazolylgruppe oder eine Alkoxycarbonylgruppe mit insgesamt 2 bis 5 Kohlenstoffatomen und

$R_5$ ein Wasserstoff-, Fluor-, Chlor- oder Bromatom bedeuten,

deren 1-, 3-Isomerengemische, deren Tautomeren, deren Enantiomeren und deren Additionssalze mit organischen oder anorganischen Säuren oder Basen.

**3.** Heterocyclische Imidazole der allgemeinen Formel I gemäß Anspruch 1, in der

einer oder zwei der Reste $A_1$, $A_2$, $A_3$ oder $A_4$ ein Stickstoffatom und die verbleibenden Reste $A_1$, $A_2$, $A_3$ oder $A_4$ jeweils eine Methingruppe,

$R_1$ eine durch eine Cyclohexyl-, Cyclohexylmethyl-, Benzyl- oder Dimethoxybenzylgruppe substituierte Amino-, Methylamino- oder Ethylaminogruppe, eine durch eine Alkanoylgruppe mit 2 bis 6 Kohlenstoffatomen, durch eine Alkansulfonylgruppe mit 1 bis 4 Kohlenstoffatomen, durch eine Alkoxycarbonylgruppe mit insgesamt 2 bis 4 Kohlenstoffatomen, durch eine Cyclohexylcarbonyl-, Benzylcarbonyl-, 2-Aminocarbonyl-cyclohexylcarbonyl-, Benzolsulfonyl- oder Chlorbenzolsulfonylgruppe substituierte Amino-, Cyclohexylamino-, Cyclohexylmethyl- oder Alkylaminogruppe mit 1 bis 4 Kohlenstoffatomen, eine Alkylsultamgruppe mit 3 oder 4 Kohlenstoffatomen im Alkylteil, eine gegebenenfalls durch eine Methyl- oder Hydroxycarbonylmethylgruppe substituierte Pyrrolidino- oder Piperidinogruppe, eine Pyrrolidin-2-on-1-yl- oder Piperidin-2-on-1-yl-gruppe, eine gegebenenfalls ganz hydrierte Phthalimido- oder 2-Oxo-isoindolin-1-yl-gruppe oder eine Gruppe der Formel

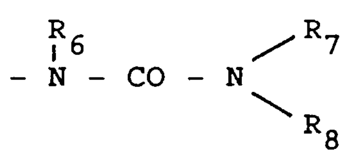

,

in welcher

$R_6$, $R_7$ und $R_8$, die gleich oder verschieden sein können, Wasserstoffatome, Alkylgruppen mit 1 bis 4 Kohlenstoffatomen, Cyclohexyl-, Phenyl-, Cyclohexylmethyl-, Benzyl-, Methoxybenzyl- oder Hydroxybenzylgruppen oder

$R_7$ auch eine 2-Chlor-ethyl-, 2-Brom-ethyl-, 3-Chlor-propyl- oder 3-Brom-propylgruppe oder auch, wenn $R_6$ und $R_7$ zusammen eine Ethylen- oder Propylengruppe darstellen, eine Dimethylaminocarbonylgruppe, oder

$R_6$ und $R_7$ zusammen eine Ethylen- oder Propylengruppe darstellen,

$R_2$ ein Wasserstoffatom, eine Methyl- oder Ethylgruppe,

$R_3$ eine Alkylgruppe mit 1 bis 5 Kohlenstoffatomen,

$R_4$ eine Carboxy- oder 1H-Tetrazolylgruppe und

$R_5$ ein Wasserstoffatom bedeuten,

deren 1-, 3-Isomerengemische, deren Tautomeren, deren Enantiomeren und deren Additionssalze mit organischen oder anorganischen Säuren oder Basen.

**4.** Heterocyclische Imidazole der allgemeinen Formel I gemäß Anspruch 1, in der

einer oder zwei der Reste $A_1$, $A_2$, $A_3$ oder $A_4$ ein Stickstoffatom und die verbleibenden Reste $A_1$, $A_2$, $A_3$ oder $A_4$ jeweils eine Methingruppe,

$R_1$ eine durch eine Cyclohexyl- oder Cyclohexylmethylgruppe substituierte Amino-, Methylamino- oder Ethylaminogruppe, eine durch eine Alkanoylgruppe mit 2 bis 6 Kohlenstoffatomen, durch eine Alkansulfonylgruppe mit 1 bis 4 Kohlenstoffatomen, durch eine Alkoxycarbonylgruppe mit insgesamt 2 bis 4 Kohlenstoffatomen, durch eine Benzylcarbonyl-, 2-Aminocarbonyl-cyclohexylcarbonyl-, Benzolsulfonyl- oder Chlorbenzolsulfonylgruppe substituierte Cyclohexylamino- oder Cyclohexylmethylaminogruppe, eine Cyclohexylcarbonylaminogruppe, eine durch eine Alkansulfonylgruppe mit 1 bis 4 Kohlenstoffatomen, durch eine Cyclohexylcarbonyl-, 2-Aminocarbonyl-cyclohexylcarbonyl-, Benzolsulfonyl- oder Chlorbenzolsulfonylgruppe substituierte Cyclohexylamino-, Cyclohexylmethylamino- oder Alkylaminogruppe mit 1 bis 4 Kohlenstoffatomen, eine Alkylsultamgruppe mit 3 oder 4 Kohlenstoffatomen im Alkylteil, eine durch eine Methyl- oder Hydroxycarbonylmethylgruppe substituierte Pyrrolidino- oder Piperidinogruppe, eine Pyrrolidin-2-on-1-yl-oder Piperidin-2-on-1-yl-gruppe, eine gegebenenfalls ganz hydrierte Phthalimido- oder 2-Oxo-isoindolin-1-yl-gruppe oder eine Gruppe der Formel

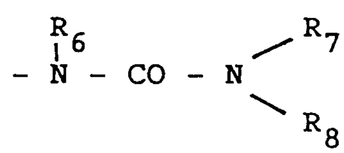 ,

in welcher

einer der Reste $R_6$, $R_7$ oder $R_8$ eine Cyclopentyl-, Cyclohexyl- oder Cyclohexylmethylgruppe und

die verbleibenden Reste der Reste $R_6$, $R_7$ oder $R_8$, die gleich oder verschieden sein können, Wasserstoffatome, Alkylgruppen mit 1 bis 4 Kohlenstoffatomen, Cyclohexyl-, Phenyl-, Cyclohexylmethyl-, Benzyl-, Methoxybenzyl- oder Hydroxybenzylgruppen oder

$R_7$ eine 2-Chlor-ethyl-, 2-Brom-ethyl-, 3-Chlor-propyl- oder 3-Brom-propylgruppe und

$R_6$ und $R_8$, die gleich oder verschieden sein können, Wasserstoffatome, Alkylgruppen mit 1 bis 4 Kohlenstoffatomen, Cyclohexyl-, Phenyl-, Cyclohexylmethyl-, Benzyl-, Methoxybenzyl- oder Hydroxybenzylgruppen oder

$R_6$ und $R_7$ zusammen eine Ethylen- oder Propylengruppe und $R_8$ ein Wasserstoffatom, eine Alkylgruppen mit 1 bis 4 Kohlenstoffatomen, eine Cyclohexyl-, Phenyl-, Cyclohexylmethyl-, Benzyl-, Methoxybenzyl-, Hydroxybenzyl-, 2-Chlor-ethyl-, 2-Brom-ethyl-, 3-Chlor-propyl-, 3-Brom-propyl- oder Dimethylaminocarbonylgruppe darstellen,

$R_2$ ein Wasserstoffatom, eine Methyl- oder Ethylgruppe,

$R_3$ eine Alkylgruppe mit 1 bis 5 Kohlenstoffatomen,

$R_4$ eine Carboxy- oder 1H-Tetrazolylgruppe und

$R_5$ ein Wasserstoffatom bedeuten,

deren 1-, 3-Isomerengemische, deren Tautomeren, deren Enantiomeren und deren Additionssalze mit organischen oder anorganischen Säuren oder Basen.

**5.** Heterocyclische Imidazole der allgemeinen Formel I gemäß Anspruch 1:

4'-[(2-n-Butyl-3H-imidazo[4,5-b]pyridin-3-yl)methyl]biphenyl-2-carbonsäure,

4'-[(2-n-Butyl-5-methyl-6-phthalimido-3H-imidazo[4,5-b]pyridin-3-yl)methyl]-biphenyl-2-carbonsäure,

4'-[(2-n-Butyl-1H-imidazo[4,5-c]pyridin-1-yl)methyl]biphenyl-2-carbonsäure,

4'-[(2-n-Butyl-5-amino-3H-imidazo[4,5-b]pyridin-3-yl)methyl]biphenyl-2-carbonsäure,

4'-[(2-Benzylamino-8-n-butyl-9H-purin-9-yl)-methyl]biphenyl-2-carbonsäure,

4'-[(2-n-Butyl-5-methyl-6-propionylamino-3H-imidazo[4,5-b]pyridin-3-yl)-methyl]biphenyl-2-carbonsäure,

4'-[(2-n-Butyl-5-cyclohexylamino-3H-imidazo[4,5-b]pyridin-3-yl)methyl]biphenyl-2-carbonsäure,

4'-[(2-n-Butyl-5-N-cyclohexylcarbonyl-ethylamino-3H-imidazo[4,5-b]pyridin-3-yl)methyl]biphenyl-2-carbonsäure,

4'-[(2-n-Butyl-5-methyl-6-valeroylamino-3H-imidazo[4,5-b]pyridin-3-yl)methyl]biphenyl-2-carbonsäure,

4'-[[2-n-Butyl-5-(3-benzyl-3,4,5,6-tetrahydro-2(1H)pyrimidinon-1-yl)-3H-imidazo[4,5-b]pyridin-3-yl]-methyl]biphenyl-2-carbonsäure,

4'-[(2-n-Propyl-5-cyclohexylcarbonylamino-3H-imidazo[4,5-b]pyridin-3-yl)methyl]-2-(1H-tetrazol-5-yl)-biphenyl,

4'-[[2-Ethyl-5-(2,2-dimethyl-propionylamino)-3H-imidazo[4,5-b]pyridin-3-yl]-methyl]-2-(1H-tetrazol-5-yl)-biphenyl und

4'-[(2-n-Propyl-5-(2-methyl-propionylamino)-3H-imidazo[4,5-b]pyridin-3-yl)-methyl]-2-(1H-tetrazol-5-yl)-biphenyl

und deren Additionssalze mit anorganischen oder organischen Säuren oder Basen.

**6.** Physiologisch verträgliche Additionssalze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Additionssalz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels mit Angiotensin-antagonistischen Wirkungen.

**9.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder

mehrere inerte Trägerstoffe und/oder Verdünnungsmittel eingearbeitet wird.

**10.** Verfahren zur Herstellung der heterocyclischen Imidazole gemäß den Ansprüchen 1 bis 6, dadurch gekennzeichnet, daß

a) eine Verbindung der allgemeinen Formel

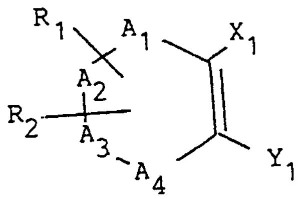

(II)

in der
$R_1$, $R_2$ und $A_1$ bis $A_4$ wie in den Ansprüchen 1 bis 5 definiert sind,
einer der Reste $X_1$ oder $Y_1$ eine Gruppe der allgemeinen Formel

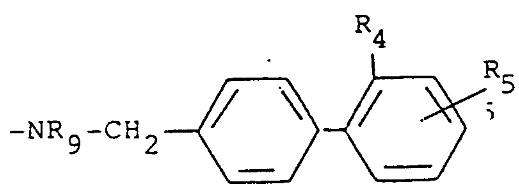

und der andere der Reste $X_1$ oder $Y_1$ eine Gruppe der allgemeinen Formel

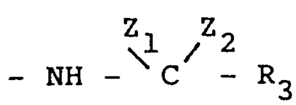

darstellen, wobei
$R_3$ und $R_5$ wie in den Ansprüchen 1 bis 5 definiert sind, $R_9$ ein Wasserstoffatom oder eine $R_3CO$-Gruppe, wobei $R_3$ wie vorstehend erwähnt definiert ist, $Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder
$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, wobei jedoch einer der Reste $X_1$ oder $Y_1$ eine Gruppe der allgemeinen Formel

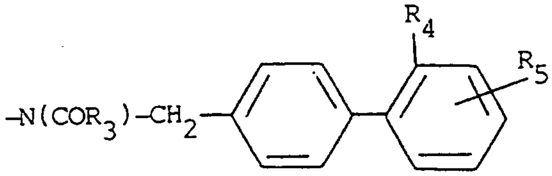

oder

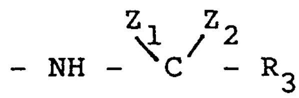

darstellen muß,
cyclisiert und ein gegebenenfalls so erhaltenes N-Oxid reduziert wird oder

b) eine Verbindung der allgemeinen Formel

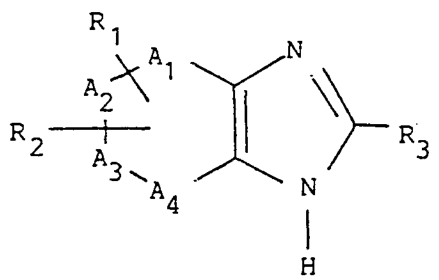

(III)

in der
$R_1$ bis $R_3$ und $A_1$ bis $A_4$ wie in den Ansprüchen 1 bis 5 definiert sind, mit einer Biphenylverbindung der allgemeinen Formel

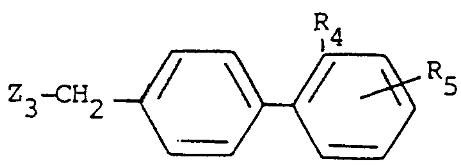

(IV)

in der
$R_4$ und $R_5$ wie in den Ansprüchen 1 bis 5 definiert sind und
$Z_3$ eine nukleophile Austrittsgruppe wie ein Halogenatom oder eine substituierte Sulfonyloxygruppe darstellt, umgesetzt wird oder

c) zur Herstellung einer Verbindung der allgemeinen Formel I, in der $R_4$ eine Carboxygruppe darstellt, eine Verbindung der allgemeinen Formel

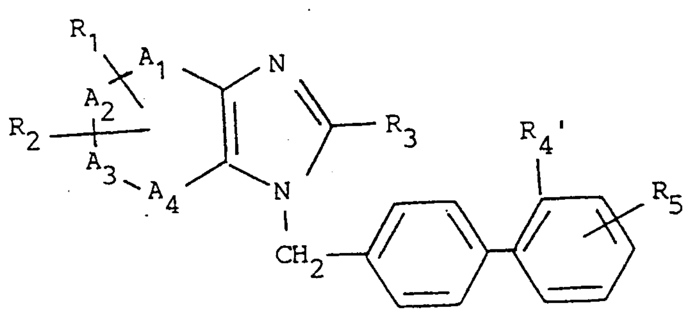

(V)

in der
$R_1$ bis $R_3$, $R_5$ und $A_1$ bis $A_4$ wie in den Ansprüchen 1 bis 5 definiert sind und
$R_4$' eine mittels Hydrolyse, Thermolyse oder Hydrogenolyse in eine Carboxygruppe überführbare Gruppe darstellt, in eine entsprechende Carboxyverbindung übergeführt wird oder

d) zur Herstellung einer Verbindung der allgemeinen Formel I, in der $R_4$ eine 1H-Tetrazolylgruppe

darstellt, ein Schutzrest von einer Verbindung der allgemeinen Formel

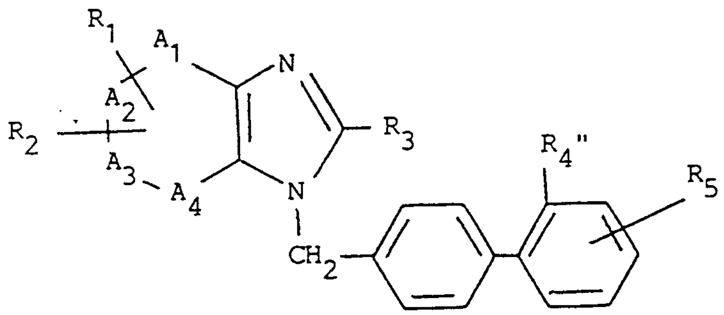

(VI)

in der
$R_1$ bis $R_3$, $R_5$ und $A_1$ bis $A_4$ wie in den Ansprüchen 1 bis 5 definiert sind und
$R_4''$ eine in 1-Stellung durch einen Schutzrest geschützte 1H-Tetrazolylgruppe darstellt, abgespalten wird oder

e) zur Herstellung einer Verbindung der allgemeinen Formel I, in der $R_4$ eine 1H-Tetrazolylgruppe darstellt, eine Verbindung der allgemeinen Formel

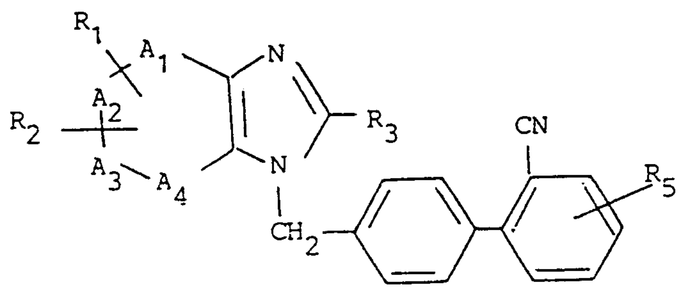

(VII)

in der
$R_1$ bis $R_3$, $R_5$ und $A_1$ bis $A_4$ wie in den Ansprüchen 1 bis 5 definiert sind, mit Stickstoffwasserstoffsäure oder mit deren

Salzen umgesetzt wird oder

f) zur Herstellung von Verbindungen der allgemeinen Formel I, in der $R_1$ eine gegebenenfalls durch eine Alkylgruppe mit 1 bis 6 Kohlenstoffatomen, durch eine Phenyl-, Cycloalkyl-, Phenylalkyl- oder Cycloalkylalkylgruppe substituierte Aminogruppe darstellt, wobei, soweit nichts anderes erwähnt wurde, die vorstehend erwähnten Alkylteile jeweils 1 bis 3 Kohlenstoffatome und die Cycloalkylteile jeweils 5 bis 7 Kohlenstoffatome enthalten können, eine Verbindung der Formel

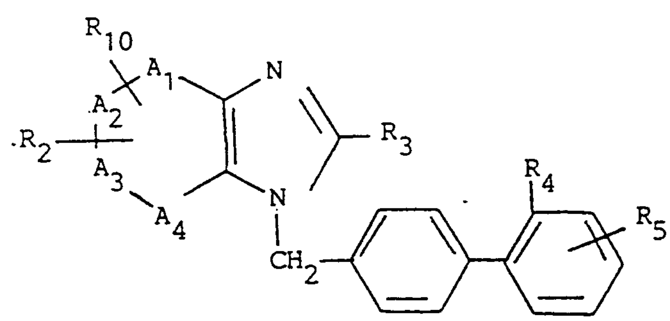

(VIII)

in der

$R_2$ bis $R_5$ und $A_1$ bis $A_4$ wie in den Ansprüchen 1 bis 5 definiert sind und
$R_{10}$ eine mittels Hydrolyse, Hydrogenolyse oder Umamidierung in eine gegebenenfalls durch eine Alkylgruppe mit 1 bis 6 Kohlenstoffatomen, durch eine Phenyl-, Cycloalkyl-, Phenylalkyl- oder Cycloalkylalkylgruppe substituierte Aminogruppe überführbare Gruppe darstellt, wobei, soweit nichts anderes erwähnt wurde, die vorstehend erwähnten Alkylteile jeweils 1 bis 3 Kohlenstoffatome und die Cycloalkylteile jeweils 5 bis 7 Kohlenstoffatome enthalten können, in eine entsprechende Verbindung übergeführt wird oder

g) zur Herstellung von Verbindungen der allgemeinen Formel I, in der $R_1$ eine Gruppe der Formel

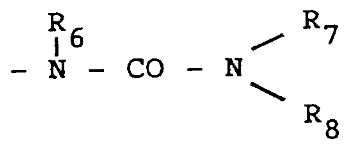

darstellt, eine Verbindung der Formel

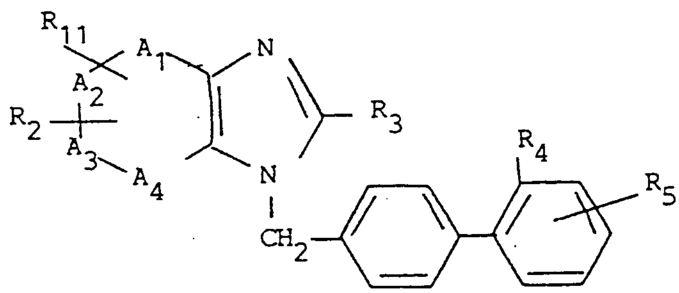

(IX)

in der
$R_2$ bis $R_5$ und $A_1$ bis $A_4$ wie in den Ansprüchen 1 bis 5 definiert sind und
$R_{11}$ eine $R_6$NH-Gruppe darstellt, wobei $R_6$ wie in den Ansprüchen 1 bis 5 definiert ist, mit einer Verbindung der Formel

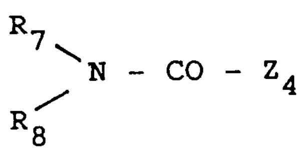

,(X)

in der
$R_7$ und $R_8$ wie in den Ansprüchen 1 bis 5 definiert sind,
$Z_4$ eine nukleophile Austrittsgruppe oder auch
$Z_4$ zusammen mit $R_7$ eine Stickstoff-Kohlenstoff-Bindung bedeuten, umgesetzt wird oder

h) zur Herstellung von Verbindungen der allgemeinen Formel I, in der $R_1$ eine gegebenenfalls am Stickstoffatom durch eine Alkylgruppe mit 1 bis 6 Kohlenstoffatomen, durch eine Phenyl-, Cycloalkyl-, Phenylalkyl- oder Cycloalkylalkylgruppe substituierte N-Acylaminogruppe bedeutet, eine Verbindung der allgemeinen Formel

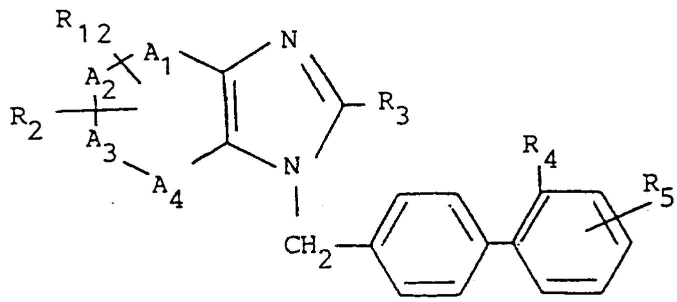

,(XI)

in der
$R_2$ bis $R_5$ und $A_1$ bis $A_4$ wie in den Ansprüchen 1 bis 5 definiert sind und
$R_{12}$ eine gegebenenfalls durch eine Alkylgruppe mit 1 bis 6 Kohlenstoffatomen, durch eine Phenyl-, Cycloalkyl-, Phenylalkyl- oder Cycloalkylalkylgruppe substituierte Aminogruppe bedeutet, wobei, soweit nichts anderes erwähnt wurde, die vorstehend erwähnten Alkyteile jeweils 1 bis 3 Kohlenstoffatome und die Cycloalkylteile jeweils 5 bis 7 Kohlenstoffatome enthalten können, mit einer Verbindung der allgemeinen Formel

$R_{13}$ - W - OH ,(XII)

in der
W eine Carbonyl- oder Sulfonylgruppe,
$R_{13}$ eine Alkylgruppe mit 1 bis 6 Kohlenstoffatomen, eine Alkoxygruppe mit 1 bis 3 Kohlenstoffatomen, eine Phenylalkyl-, Cycloalkyl oder Cycloalkylalkylgruppe mit jeweils 1 bis 3 Kohlenstoffatomen im Alkylteil und 5 bis 7 Kohlenstoffatomen im Cycloalkylteil, eine Phenyl-, Naphthyl-, 2-Carboxy-cyclohexyl- oder 2-Aminocarbonyl-cyclohexylgruppe, wobei die vorstehend erwähnten Phenylkerne durch ein Fluor-, Chlor- oder Bromatom, durch eine Methyl- oder Methoxygruppe mono- oder disubstituiert und die Substituenten gleich oder verschieden sein können, oder auch, wenn W eine Carbonylgruppe darstellt, ein Wasserstoffatom bedeuten, oder mit deren reaktionsfähigen Derivaten wie deren Säurehalogeniden, Säureestern oder Säureanhydriden acyliert wird oder

i) zur Herstellung einer Verbindung der Formel I, in der $R_6$ und $R_7$ zusammen eine Ethylen- oder n-Propylengruppe bedeuten, eine Verbindung der Formel

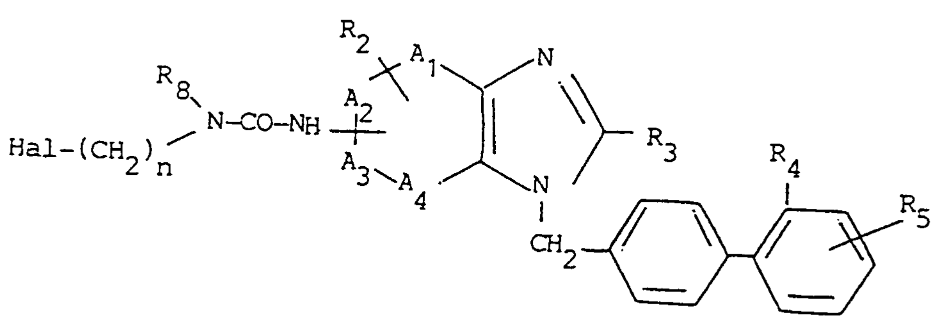

(XIII)

in der
$R_2$ bis $R_5$, $R_8$ und $A_1$ bis $A_4$ wie in den Ansprüchen 1 bis 5 definiert sind,
Hal ein Chlor-, Brom- oder Jodatom und
n die Zahl 2 oder 3 darstellen, cyclisiert und erforderlichenfalls anschließend mit einer Verbindung der Formel

$R_8$ - Hal ,(XIV)

in der
$R_8$ mit Ausnahme des Wasserstoffatoms wie in den Ansprüchen 1 bis 5 definiert ist und

Hal ein Chlor-, Brom- oder Jodatom darstellt, umgesetzt wird und

gewünschtenfalls anschließend ein so erhaltenes 1-, 3-Isomerengemisch einer Verbindung der allgemeinen Formel I mittels Isomerentrennung in ihr 1- und 3-Isomer aufgetrennt wird oder

ein so erhaltenes Racemat mittels Enantiomerentrennung in ihre Enantiomeren aufgetrennt wird oder

eine so erhaltene Verbindung der allgemeinen Formel I in ihr Additionssalz, insbesondere für die pharmazeutische Anwendung in ihr physiologisch verträgliches Salz mit einer anorganischen oder organischen Säure oder Base, übergeführt wird.

Europäisches Patentamt

# EUROPÄISCHER RECHERCHENBERICHT

Nummer der Anmeldung

EP 91113121.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| P,X | EP - A1 - 0 426 021 (FUJISAWA) * Zusammenfassung; Seite 3, Zeilen 6-8; Ansprüche 15,16 * -- | 1-4,6-10 | C 07 D 471/04<br>C 07 D 487/04<br>C 07 D 473/00<br>C 07 D 473/40<br>A 61 K 31/435<br>A 61 K 31/52<br>A 61 K 31/505<br>A 61 K 31/495 |
| P,X | EP - A1 - 0 399 731 (IMPERIAL CHEMICAL INDUSTRIES) * Zusammenfassung; Ansprüche 1,9-11 * -- | 1-4,6-10 | |
| P,A | EP - A2 - 415 886 (CIBA-GEIGY) * Zusammenfassung; Seite 6, Zeilen 34-36 * -- | 1-10 | |
| P,A | EP - A2 - 0 400 974 (MERCK) * Zusammenfassung ; Seite 3, Zeilen 45-50 * -- | 1-10 | |
| A | EP - A2 - 0 260 613 (SEARLE) * Seite 2, Zeilen 14-37; Seite 3, Zeilen 30-45 * ---- | 1-10 | RECHERCHIERTE SACHGEBIETE (Int. Cl.⁵)<br>C 07 D 471/00<br>C 07 D 487/00<br>C 07 D 473/00 |

Der vorliegende Recherchenbericht wurde für alle Patentansprüche erstellt.

| Recherchenort | Abschlußdatum der Recherche | Prüfer |
|---|---|---|
| WIEN | 11-09-1991 | ONDER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument